# EUROPEAN PATENT SPECIFICATION

(11) **EP 3 131 588 B1**
(45) Date of publication and mention of the grant of the patent: **05.11.2025**
(21) Application number: 15780467.5
(22) Date of filing: 14.04.2015
(51) Int. Cl.: A61K 47/50

(54) **IMIDE-BASED MODULATORS OF PROTEOLYSIS AND ASSOCIATED METHODS OF USE**
IMID-BASIERTE MODULATOREN VON PROTEOLYSE UND ASSOZIIERTE VERFAHREN ZUR VERWENDUNG
MODULATEURS DE LA PROTÉOLYSE, À BASE D'IMIDE, ET PROCÉDÉS D'UTILISATION ASSOCIÉS

(30) Priority: 14.04.2014 US 201461979351 P
(43) Date of publication of application: 22.02.2017
(60) Divisional application: 25186756.0
(73) Proprietor: Arvinas Operations, Inc., New Haven, CT 06511 (US)
(72) Inventor: CREWS, Craig, New Haven, CT 06511 (US); CREW, Andrew, P., Guilford, CT 06437 (US); DONG, Hanqing, Madison, CT 06443 (US); WANG, Jing, Milford, CT 06460 (US); QIAN, Yimin, Plainsboro, NJ 08536 (US); JIN, Meizhong, East Northport, NY 11731 (US)
(74) Representative: Potter Clarkson
(86) International application number: PCT/US2015/025813
(87) International publication number: WO 2015/160845

(56) References cited:
- WO-A2-2013/106643
- WO-A2-2013/106643
- US-A1- 2008 051 432
- US-A1- 2008 153 837
- MULLER G W ET AL: "Amino-substituted thalidomide analogs: potent inhibitors of TNF-alpha production", BIOORGANIC & MEDICINAL CHEMISTRY LETTERS, PERGAMON, AMSTERDAM, NL, vol. 9, no. 11, 7 June 1999 (1999-06-07), pages 1625 - 1630, XP004169632, ISSN: 0960-894X, DOI: 10.1016/S0960-894X(99)00250-4
- CAPITOSTI SCOTT M ET AL: "Thalidomide analogues demonstrate dual inhibition of both angiogenesis and prostate cancer", BIOORGANIC & MEDICINAL CHEMISTRY, PERGAMON, GB, vol. 12, no. 2, 5 December 2003 (2003-12-05), pages 327 - 336, XP085038069, ISSN: 0968-0896, DOI: 10.1016/J.BMC.2003.11.007
- KAI LIU ET AL: "Design and biological characterization of hybrid compounds of curcumin and thalidomide for multiple myeloma", ORGANIC & BIOMOLECULAR CHEMISTRY, vol. 11, no. 29, 1 January 2013 (2013-01-01), GB, pages 4757, XP055429153, ISSN: 1477-0520, DOI: 10.1039/c3ob40595h
- RUCHELMAN ALEXANDER L ET AL: "Isosteric analogs of lenalidomide and pomalidomide: Synthesis and biological activity", BIOORGANIC & MEDICINAL CHEMISTRY LETTERS, vol. 23, no. 1, 27 October 2012 (2012-10-27), pages 360 - 365, XP028961553, ISSN: 0960-894X, DOI: 10.1016/J.BMCL.2012.10.071
- VAHID F ET AL: "The role dietary of bioactive compounds on the regulation of histone acetylases and deacetylases: A review", GENE, ELSEVIER, AMSTERDAM, NL, vol. 562, no. 1, 19 February 2015 (2015-02-19), pages 8 - 15, XP029146923, ISSN: 0378-1119, DOI: 10.1016/J.GENE.2015.02.045
- VALLEE ET AL.: "Tricyclic Series of Heat Shock Protein 90 (Hsp90) Inhibitors Part I: Discovery of Tricyclic Imidazo[4,5-c]pyridines as Potent Inhibitors of the Hsp90 Molecular Chaperone.", JOURNAL OF MEDICINAL CHEMISTRY, vol. 54, no. 20, 2011, pages 7206 - 7219, XP055240728, ISSN: 0022-2623, Retrieved from the Internet <URL:http://pubs.acs.org/doi/abs/10.1021/jm200784m> [retrieved on 20150809]

## Description

### FIELD OF THE INVENTION

Described herein are imide-based compounds, including bifunctional compounds comprising the same, and associated methods of use. The bifunctional compounds are useful as modulators of targeted ubiquitination, especially with respect to a variety of polypeptides and other proteins, which are degraded and/or otherwise inhibited by bifunctional compounds according to the present invention.

### BACKGROUND

Most small molecule drugs bind enzymes or receptors in tight and well-defined pockets. On the other hand, protein-protein interactions are notoriously difficult to target using small molecules due to their large contact surfaces and the shallow grooves or flat interfaces involved. E3 ubiquitin ligases (of which hundreds are known in humans) confer substrate specificity for ubiquitination, and therefore, are more attractive therapeutic targets than general proteasome inhibitors due to their specificity for certain protein substrates. The development of ligands of E3 ligases has proven challenging, in part due to the fact that they must disrupt protein-protein interactions. However, recent developments have provided specific ligands which bind to these ligases. For example, since the discovery of nutlins, the first small molecule E3 ligase inhibitors, additional compounds have been reported that target E3 ligases but the field remains underdeveloped.

One E3 ligase with therapeutic potential is the von Hippel-Lindau (VHL) tumor suppressor. VHL comprises the substrate recognition subunit/E3 ligase complex VCB, which includes elongins B and C, and a complex including Cullin-2 and Rbx1. The primary substrate of VHL is Hypoxia Inducible Factor 1α (HIF-1α), a transcription factor that upregulates genes such as the pro-angiogenic growth factor VEGF and the red blood cell inducing cytokine erythropoietin in response to low oxygen levels. We generated the first small molecule ligands of VHL to the substrate recognition subunit of the E3 ligase, VCB, an important target in cancer, chronic anemia and ischemia, and obtained crystal structures confirming that the compound mimics the binding mode of the transcription factor HIF-1α, the major substrate of VHL.

Cereblon is a protein that in humans is encoded by the CRBN gene. CRBN orthologs are highly conserved from plants to humans, which underscores its physiological importance. Cereblon forms an E3 ubiquitin ligase complex with damaged DNA binding protein 1 (DDB1), Cullin-4A (CUL4A), and regulator of cullins 1 (ROC1). This complex ubiquitinates a number of other proteins. Through a mechanism which has not been completely elucidated, cereblon ubquitination of target proteins results in increased levels of fibroblast growth factor 8 (FGF8) and fibroblast growth factor 10 (FGF10). FGF8 in turn regulates a number of developmental processes, such as limb and auditory vesicle formation. The net result is that this ubiquitin ligase complex is important for limb outgrowth in embryos. In the absence of cereblon, DDB1 forms a complex with DDB2 that functions as a DNA damage-binding protein.

Thalidomide, which has been approved for the treatment of a number of immunological indications, has also been approved for the treatment of certain neoplastic diseases, including multiple myeloma. For example, Liu et al. Org. Biomol. Chem., 2013, 11, 4757 discusses the design, synthesis and biological chacracterization of hybrid compounds of thalidomide for treatment of multiple myeloma. In addition to multiple myeloma, thalidomide and several of its analogs are also currently under investigation for use in treating a variety of other types of cancer. While the precise mechanism of thalidomide's anti-tumor activity is still emerging, it is known to inhibit angiogenesis. Recent literature discussing the biology of the imides includes Lu et al Science 343, 305 (2014) and Krönke et al Science 343, 301 (2014).

Significantly, thalidomide and its analogs (e.g. pomolinamiode and lenalinomide) are known to bind cereblon. These agents bind to cereblon, altering the specificity of the complex to induce the ubiquitination and degradation of Ikaros (IKZF1) and Aiolos (IKZF3), transcription factors essential for multiple myeloma growth. Indeed, higher expression of cereblon has been linked to an increase in efficacy of imide drugs in the treatment of multiple myeloma.

An ongoing need exists in the art for effective treatments for disease, especially hyperplasias and cancers, such as multiple myeloma. However, non-specific effects, and the inability to target and modulate certain classes of proteins altogether, such as transcription factors, remain as obstacles to the development of effective anti-cancer agents. As such, small molecule therapeutic agents that leverage or potentiate cereblon's substrate specificity and, at the same time, are "tunable" such that a wide range of protein classes can be targetted and modulated with specificity would be very useful as a therapeutic.

### BRIEF SUMMARY OF THE INVENTION

In a first aspect, the present invention defines a compound having the chemical structure comprising: PTM-L-CLM, or a pharmaceutically acceptable salt, enantiomer or stereoisomer thereof, as defined in appended claim 1. The present invention also defines a compound, or a pharmaceutically acceptable salt, enantiomer or stereoisomer thereof, as defined in appended claim 3.

In a second aspect, the present invention defines a pharmaceutical composition comprising the compound of any one of appended claims 1-3, or a pharmaceutically acceptable salt, enantiomer or stereoisomer thereof, and a pharmaceutically acceptable carrier, additive and/or excipient.

In a third aspect, the present invention defines a compound of any one of appended claims 1-3, or a pharmaceutically acceptable salt, enantiomer or stereoisomer thereof, for use in a method of treating a disease state or condition that is modulated through protein degradation in a patient, the method comprising administering an effective amount of the compound or pharmaceutically acceptable salt, enantiomer or stereoisomer thereof.

In a fourth aspect, the present invention defines a compound of any one of appended claims 1-3, or a pharmaceutically acceptable salt, enantiomer or stereoisomer thereof, for use in a method of treating cancer, the method comprising administering an effective amount of the compound to a patient in need thereof, wherein the cancer is large B-cell lymphoma, prostate cancer, ovarian cancer, breast cancer, multiple myeloma, uterine cancer, colon cancer, or leukemia.

Preferred features are defined in the dependent claims.

The present disclosure describes bifunctional compounds which function to recruit endogenous proteins to an E3 Ubiquitin Ligase for degradation, and methods of using the same. In particular, the present disclosure describes bifunctional or proteolysis targeting chimeric (PROTAC) compounds, which find utility as modulators of targeted ubiquitination of a variety of polypeptides and other proteins, which are then degraded and/or otherwise inhibited by the bifunctional compounds as described herein. An advantage of the compounds described herein is that a broad range of pharmacological activities is possible, consistent with the degradation/inhibition of targeted polypeptides from virtually any protein class or family. In addition, described are methods of using an effective amount of the compounds as described herein for the treatment or amelioration of a disease condition, such as cancer (e.g., multiple myeloma).

As such, in one aspect the disclosure describes novel imide-based compounds as described herein.

For example, described herein are bifunctional or PROTAC compounds, which comprise an E3 Ubiquitin Ligase binding moiety (i.e., a ligand for an E3 Ubquitin Ligase or "ULM" group), and a moiety that binds a target protein (i.e., a protein/polypeptide targeting ligand or "PTM" group) such that the target protein/polypeptide is placed in proximity to the ubiquitin ligase to effect degradation (and inhibition) of that protein. In a preferred embodiment, the ULM is a cereblon E3 Ubiquitin Ligase binding moiety (i.e., a "CLM"). For example, the structure of the bifunctional compound can be depicted as:

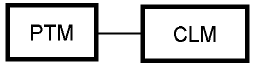

, The bifunctional compound of the present invention further comprises a chemical linker ("L"). In this example, the structure of the bifunctional compound can be depicted as: 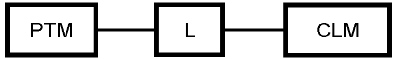 where PTM is a protein/polypeptide targeting moiety, L is a linker, and CLM is a cereblon E3 ubiquitin ligase binding moiety.

In the present invention, the E3 Ubiquitin Ligase is cereblon. As such, in certain embodiments, the CLM of the bifunctional compound comprises chemistries such as imide, amide, thioamide, thioimide derived moieties. For example, the CLM comprises a phthalimido group or an analog or derivative thereof. For example, the CLM comprises a phthalimido-glutarimide group or an analog or derivative thereof. For example, the CLM comprises a member of the group consisting of thalidomide, lenalidomide, pomalidomide, and analogs or derivatives thereof.

For example and in general, compounds as described herein may comprise multiple CLMs, multiple PTMs, multiple chemical linkers or a combination thereof.

Also described are therapeutic compositions comprising an effective amount of a compound as described herein or salt form thereof, and a pharmaceutically acceptable carrier. The therapeutic compositions modulate protein degradation in a patient or subject, for example, an animal such as a human, and can be used for treating or ameliorating disease states or conditions which are modulated through the degraded protein. For example, the therapeutic compositions as described herein may be used to effectuate the degradation of proteins of interest for the treatment or amelioration of a disease, e.g., cancer. For example, described herein is a method of ubiquitinating/ degrading a target protein in a cell. For example, the method comprises administering a bifunctional compound as described herein comprising an CLM and a PTM, preferably linked through a linker moiety, as otherwise described herein, wherein the CLM is coupled to the PTM and wherein the CLM recognizes a ubiquitin pathway protein (e.g., an ubiquitin ligase, preferably an E3 ubiquitin ligase such as, e.g., cereblon) and the PTM recognizes the target protein such that degradation of the target protein will occur when the target protein is placed in proximity to the ubiquitin ligase, thus resulting in degradation/inhibition of the effects of the target protein and the control of protein levels. The control of protein levels afforded by the present method provides treatment of a disease state or condition, which is modulated through the target protein by lowering the level of that protein in the cells of a patient.

Also described is a method for assessing (i.e., determining and/or measuring) a CLM's binding affinity. For example, the method comprises providing a test agent or compound of interest, for example, an agent or compound having an imide moiety (e.g., a phthalimido group, phthalimido-glutarimide group, derivatized thalidomide, derivatized lenalidomide or derivatized pomalidomide), and comparing the cereblon binding affinity and/or inhibitory activity of the test agent or compound as compared to an agent or compound known to bind and/or inhibit the activity of cereblon.

Also described are methods for treating or emeliorating a disease, disorder or symptom thereof in a subject or a patient (e.g., an animal such as a human), comprising administering to a subject in need thereof a composition comprising an effective amount (e.g., a therapeutically effective amount) of a compound as described herein or salt form thereof, and a pharmaceutically acceptable carrier, wherein the composition is effective for treating or ameliorating the disease or disorder or symptom thereof in the subject.

Also described are methods for identifying the effects of the degradation of proteins of interest in a biological system using compounds according to the present invention.

### BRIEF DESCRIPTION OF THE DRAWINGS

The accompanying drawings, which are incorporated into and form a part of the specification;together with the description, serve to explain the principles of the invention. The drawings are not to be construed as limiting the invention.

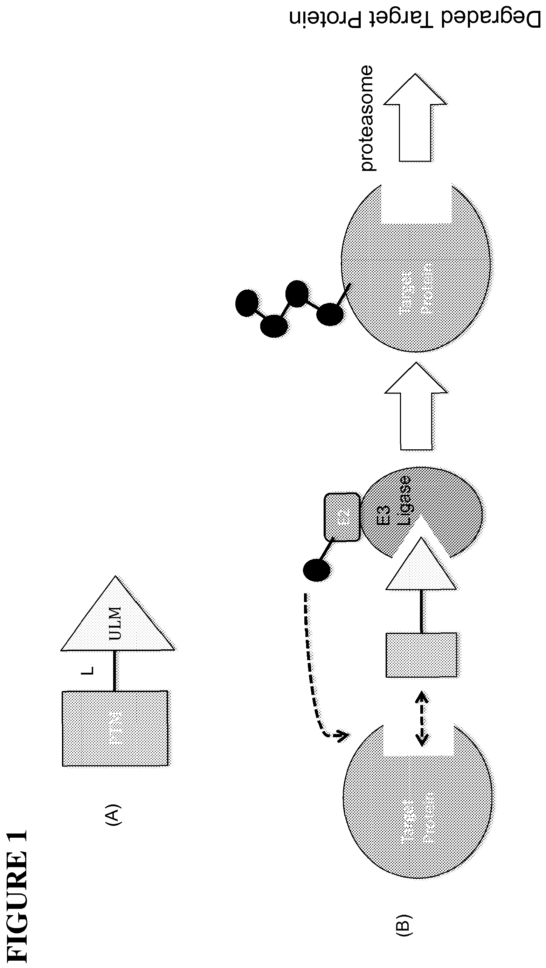

**Figure 1****.** Illustration of general principle for PROTAC function. (**A**) Exemplary PROTACs comprise a protein targeting moiety (PTM; *darkly shaded rectangle*), a ubiquitin ligase binding moiety (ULM; *lightly shaded triangle*), and optionally a linker moiety (L; *black line*) coupling or tethering the PTM to the ULM. (**B**) Illustrates the functional use of the PROTACs as described herein. Briefly, the ULM recognizes and binds to a specific E3 Ubiquitin Ligase, and the PTM binds and recruits a target protein bringing it into close proximity to the E3 Ubiquitin Ligase. Typically, the E3 Ubiquitin Ligase is complexed with an E2 ubiquitin-conjugating protein, and either alone or via the E2 protein catalyzes attachment of ubiquitin (*dark circles*) to a lysine on the target protein via an isopeptide bond. The poly-ubiquitinated protein (*far right*) is then targeted for degration by the proteosomal machinery of the cell.

### DETAILED DESCRIPTION

Presently described are compositions and methods that relate to the surprising and unexpected discovery that an E3 Ubiquitin Ligase protein, e.g., cereblon, ubiquitinates a target protein once it and the target protein are placed in proximity by a bifunctional or chimeric construct that binds the E3 Ubiquitin Ligase protein and the target protein. Accordingly, further described herein are such compounds and compositions comprising an E3 Ubiquintin Ligase binding moiety ("ULM") coupled to a protein target binding moiety ("PTM"), which result in the ubiquitination of a chosen target protein, which leads to degradation of the target protein by the proteasome (*see* **Figure 1**).

Unless otherwise defined, all technical and scientific terms used herein have the same meaning as commonly understood by one of ordinary skill in the art to which this invention belongs.

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

The following terms are used to describe the present invention. In instances where a term is not specifically defined herein, that term is given an art-recognized meaning by those of ordinary skill applying that term in context to its use in describing the present invention.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined (i.e., elements that are conjunctively present in some cases and disjunctively present in other cases). Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, for example, to A only (optionally including elements other than B); in another example, to B only (optionally including elements other than A); in yet another example, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of shall be closed or semi-closed transitional phrases, respectively,.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a nonlimiting example, "at least one of A and B" (or, equivalently, "at least one of A or B," or, equivalently "at least one of A and/or B") can refer, for example, to at least one, optionally including more than one, A, with no B present (and optionally including elements other than B); in another example, to at least one, optionally including more than one, B, with no A present (and optionally including elements other than A); in yet another example, to at least one, optionally including more than one, A, and at least one, optionally including more than one, B (and optionally including other elements); etc.

It should also be understood that, in certain methods described herein that include more than one step or act, the order of the steps or acts of the method is not necessarily limited to the order in which the steps or acts of the method are recited unless the context indicates otherwise.

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In certain preferred aspects, one or more of the present compounds described herein, are coadministered in combination with at least one additional bioactive agent, especially including an anticancer agent. In particularly preferred aspects, the co-administration of compounds results in synergistic activity and/or therapy, including anticancer activity.

The term "compound", as used herein, unless otherwise indicated, refers to any specific chemical compound disclosed herein and includes tautomers, regioisomers, geometric isomers, and where applicable, stereoisomers, including optical isomers (enantiomers) and other steroisomers (diastereomers) thereof, as well as pharmaceutically acceptable salts and derivatives (including prodrug forms) thereof where applicable, in context. Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds. The term also refers, in context to prodrug forms of compounds which have been modified to facilitate the administration and delivery of compounds to a site of activity. It is noted that in describing the present compounds, numerous substituents and variables associated with same, among others, are described. It is understood by those of ordinary skill that molecules which are described herein are stable compounds as generally described hereunder. When the bond is shown, both a double bond and single bond are represented within the context of the compound shown.

The term "Ubiquitin Ligase" refers to a family of proteins that facilitate the transfer of ubiquitin to a specific substrate protein, targeting the substrate protein for degradation. For example, cereblon is an E3 Ubiquitin Ligase protein that alone or in combination with an E2 ubiquitin-conjugating enzyme causes the attachment of ubiquitin to a lysine on a target protein, and subsequently targets the specific protein substrates for degradation by the proteasome. Thus, E3 ubiquitin ligase alone or in complex with an E2 ubiquitin conjugating enzyme is responsible for the transfer of ubiquitin to targeted proteins. In general, the ubiquitin ligase is involved in polyubiquitination such that a second ubiquitin is attached to the first; a third is attached to the second, and so forth. Polyubiquitination marks proteins for degradation by the proteasome. However, there are some ubiquitination events that are limited to mono-ubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. Further complicating matters, different lysines on ubiquitin can be targeted by an E3 to make chains. The most common lysine is Lys48 on the ubiquitin chain. This is the lysine used to make polyubiquitin, which is recognized by the proteasome.

The term "patient" or "subject" is used throughout the specification to describe an animal, preferably a human or a domesticated animal, to whom treatment, including prophylactic treatment, with the compositions according to the present invention is provided. For treatment of those infections, conditions or disease states which are specific for a specific animal such as a human patient, the term patient refers to that specific animal, including a domesticated animal such as a dog or cat or a farm animal such as a horse, cow, sheep, etc. In general, in the present invention, the term patient refers to a human patient unless otherwise stated or implied from the context of the use of the term.

The term "effective" is used to describe an amount of a compound, composition or component which, when used within the context of its intended use, effects an intended result. The term effective subsumes all other effective amount or effective concentration terms, which are otherwise described or used in the present application.

### Compounds and Compositions

In one aspect, the description describes compounds comprising an E3 Ubiquitin Ligase binding moiety ("ULM") that is a cereblon E3 Ubiquitin Ligase binding moiety ("CLM"). For example, the CLM may be coupled to a chemical linker (L) according to the structure:

(I) L-CLM

wherein L is a chemical linker group and CLM is a cereblon E3 Ubiquitin Ligase binding moiety. The number and/or relative positions of the moieties in the compounds illustrated herein is provided by way of example only. As would be understood by the skilled artisan, compounds as described herein can be synthesized with any desired number and/or relative position of the respective functional moieties.

The terms ULM and CLM are used in their inclusive sense unless the context indicates otherwise. For example, the term ULM is inclusive of all ULMs, including those that bind cereblon (i.e., CLMs). Further, the term CLM is inclusive of all possible cereblon E3 Ubiquitin Ligase binding moieties.

Also described herein are bifunctional or multifunctional PROTAC compounds useful for regulating protein activity by inducing the degradation of a target protein. For example, the compound may comprise a CLM coupled, e.g., linked covalently, indirectly, to a moiety that binds a target protein (i.e., protein targeting moiety or "PTM"). In certain examples, the CLM and PTM are joined or coupled via a chemical linker (L). The CLM recognizes the cereblon E3 ubiquitin ligase and the PTM recognizes a target protein and the interaction of the respective moieties with their targets facilitates the degradation of the target protein by placing the target protein in proximity to the ubiquitin ligase protein. In general, an exemplary bifunctional compound can be depicted as:

(II) PTM-CLM

In certain examples, the bifunctional compound further comprises a chemical linker ("L"). For example, the bifunctional compound can be depicted as:

(III) PTM-L-CLM

wherein PTM is a protein/polypeptide targeting moiety, L is a linker, and CLM is a cereblon E3 ligase binding moiety.

For example, the compounds as described herein may comprise multiple PTMs (targeting the same or different protein targets), multiple CLMs, one or more ULMs (i.e., moieties that bind specifically to another E3 Ubiquitin Ligase, e.g., VHL) or a combination thereof. For example, the PTMs, CLMs, and ULMs can be coupled directly or via one or more chemical linkers or a combination thereof. For example, where a compound has multiple ULMs, the ULMs can be for the same E3 Ubiquintin Ligase or each respective ULM can bind specifically to a different E3 Ubiquitin Ligase. For example, where a compound has multiple PTMs, the PTMs can bind the same target protein or each respective PTM can bind specifically to a different target protein.

Also described is a compound which comprises a plurality of CLMs coupled directly or via a chemical linker moiety (L). For example, a compound having two CLMs can be depicted as:

(IV) CLM-CLM

or

(V) CLM-L-CLM

For example, where the compound comprises multiple CLMs, the CLMs may be identical. For example, the compound comprising a plurality of CLMs may further comprise at least one PTM coupled to a CLM directly or via a chemical linker (L) or both. For example, the compound comprising a plurality of CLMs may further comprise multiple PTMs. For example, the PTMs may be the same or, optionally, different. For example, wherein the PTMs are different the respective PTMs may bind the same protein target or bind specifically to a different protein target.

Also described is a compound comprising at least two different CLMs coupled directly or via a chemical linker (L) or both. For example, such a compound having two different CLMs can be depicted as:

(VI) CLM-CLM'

or

(VII) CLM-L-CLM'

wherein CLM' indicates a cereblon E3 Ubiquitin Ligase binding moiety that is structurally different from CLM. For example, the compound may comprise a plurality of CLMs and/or a plurality of CLM's. For example, the compound comprising at least two different CLMs, a plurality of CLMs, and/or a plurality of CLM's may further comprise at least one PTM coupled to a CLM or a CLM' directly or via a chemical linker or both. For example, a compound comprising at least two different CLMs can further comprise multiple PTMs. For example, the PTMs may be the same or, optionally, different. For example, wherein the PTMs are different the respective PTMs may bind the same protein target or bind specifically to a different protein target. For example, the PTM itself may be a ULM or CLM (or ULM' or CLM').

For example, the CLM may comprise a moiety that is a ligand of the cereblon E3 Ubiquitin Ligase (CRBN). In certain examples, the CLM comprises a chemotype from the "imide" class of of molecules. In certain additional examples, the CLM comprises a phthalimido group or an analog or derivative thereof. In still additional examples, the CLM comprises a phthalimido-glutarimide group or an analog or derivative thereof. In still other examples, the CLM comprises a member of the group consisting of thalidomide, lenalidomide, pomalidomide, and analogs or derivatives thereof.

Also described are the compounds as described herein including their enantiomers, diastereomers, solvates and polymorphs, including pharmaceutically acceptable salt forms thereof (e.g., acid and base salt forms).

### Neo-imide compounds

Also described herein are compounds useful for binding and/or inhibiting cereblon. For example, the compound may be selected from the group consisting of chemical structures: 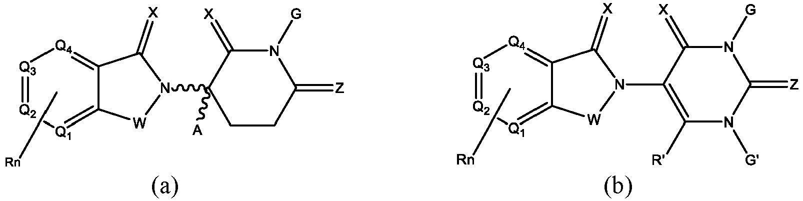 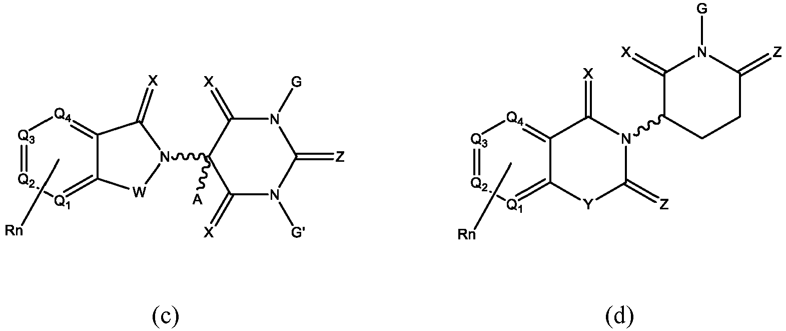 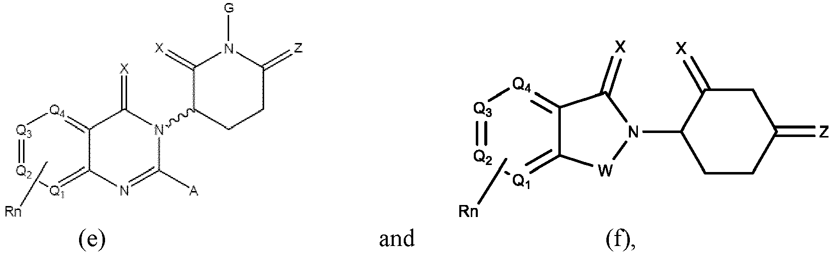 wherein
W is independently selected from the group CH₂, CHR, C=O, SO₂, NH, and N-alkyl;
X is independently selected from the group O, S and H₂;
Y is independently selected from the group NH, N-alkyl, N-aryl, N-heteroaryl, N-cycloalkyl, N-heterocyclyl, O, and S;
Z is independently selected from the group O, and S or H₂ except that both X and Z cannot be H₂;
G and G' are independently selected from the group H, alkyl, OH, CH₂-heterocyclyl optionally substituted with R', and benzyl optionally substituted with R';
Q1 - Q4 represent a carbon C substituted with a group independently selected from R', N or N-oxide;
A is independently selected from the group alkyl, cycloalkyl, Cl and F;
R comprises, but is not limited to: -CONR'R", -OR', -NR'R", -SR', -SO₂R', -SO₂NR'R", - CR'R"-, -CR'NR'R"-, -aryl, -heteroaryl, -alkyl, -cycloalkyl, -heterocyclyl, -P(O)(OR')R", -P(O)R'R", -OP(O)(OR')R", -OP(O)R'R", -Cl, -F, -Br, -I, -CF₃, -CN, -NR'SO₂NR'R", - NR'CONR'R", -CONR'COR", -NR'C(=N-CN)NR'R", -C(=N-CN)NR'R", -NR'C(=N-CN)R", -NR'C(=C-NO₂)NR'R", -SO₂NR'COR", -NO₂, -CO₂R', -C(C=N-OR')R", - CR'=CR'R", -CCR', -S(C=O)(C=N-R')R", -SF₅ and -OCF₃
R' and R" are independently selected from a bond, H, alkyl, cycloalkyl, aryl, heteroaryl, heterocyclyl
n is an integer from 1-4;
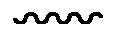 represents a bond that may be stereospecific ((R) or (S)) or non-stereospecific; and
Rₙ comprises 1-4 independent functional groups or atoms.

### Exemplary CLMs

In any of the compounds described herein, the CLM may comprise a chemical structure selected from the group: 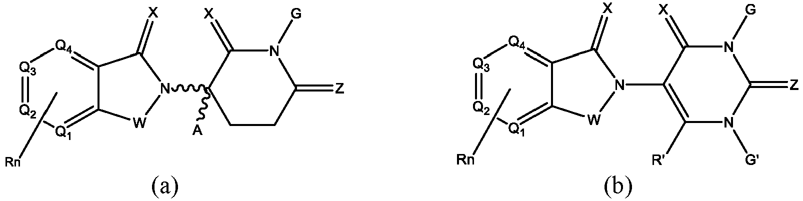 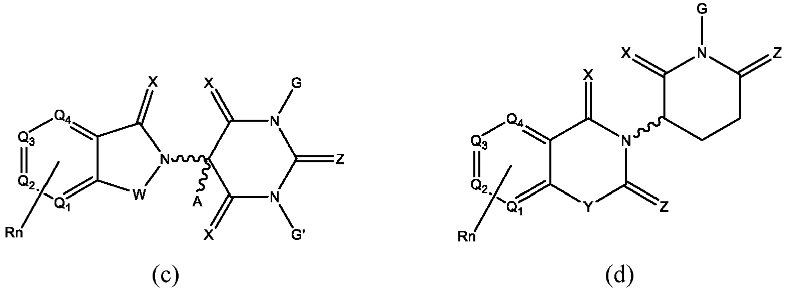 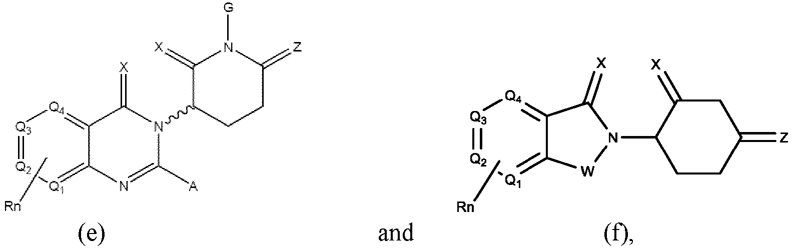 wherein
W is independently selected from the group CH₂, CHR, C=O, SO₂, NH, and N-alkyl;
X is independently selected from the group O, S and H₂;
Y is independently selected from the group NH, N-alkyl, N-aryl, N-heteroaryl, N-cycloalkyl, N-heterocyclyl, O, and S;
Z is independently selected from the group O, and S or H₂ except that both X and Z cannot be H₂;
G and G' are independently selected from the group H, alkyl, OH, CH₂-heterocyclyl optionally substituted with R', and benzyl optionally substituted with R';
Q1 - Q4 represent a carbon C substituted with a group independently selected from R', N or N-oxide;
A is independently selected from the group alkyl, cycloalkyl, Cl and F;
R comprises, but is not limited to: -CONR'R", -OR', -NR'R", -SR', -SO₂R', -SO₂NR'R", - CR'R"-, -CR'NR'R"-, -aryl, -heteroaryl, -alkyl, -cycloalkyl, -heterocyclyl, -P(O)(OR')R", -P(O)R'R", -OP(O)(OR')R", -OP(O)R'R", -Cl, -F, -Br, -I, -CF₃, -CN, -NR'SO₂NR'R", - NR'CONR'R", -CONR'COR", -NR'C(=N-CN)NR'R", -C(=N-CN)NR'R", -NR'C(=N-CN)R", -NR'C(=C-NO₂)NR'R", -SO₂NR'COR", -NO₂, -CO₂R', -C(C=N-OR')R", - CR'=CR'R", -CCR', -S(C=O)(C=N-R')R", -SF₅ and -OCF₃
R' and R" are independently selected from a bond, H, alkyl, cycloalkyl, aryl, heteroaryl, heterocyclyl;
n is an integer from 1-4;
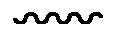 represents a bond that may be stereospecific ((R) or (S)) or non-stereospecific; and
Rn comprises 1-4 independent functional groups or atoms, and optionally, one of which is modified to be covalently joined to a PTM, a chemical linker group (L), a ULM, CLM (or CLM') or combination thereof.

The term "independently" is used herein to indicate that the variable, which is independently applied, varies independently from application to application.

The term "alkyl" shall mean within its context a linear or branch-chainedfully saturated hydrocarbon radical or alkyl group, preferably a C₁-C₁₀, more preferably a C₁-C₆, alternatively a C₁-C₃ alkyl group, which may be optionally substituted. Examples of alkyl groups are methyl, ethyl, *n*-butyl, *sec*-butyl, *n*-hexyl, *n*-heptyl, *n-octyl, n*-nonyl, *n*-decyl, isopropyl, 2-methylpropyl, among others. For example, the alkyl group may be end-capped with a halogen group (At, Br, Cl, F, or I). For example, compounds described herein may be used to covalently bind to dehalogenase enzymes. These compounds generally contain a side chain (often linked through a polyethylene glycol group) which terminates in an alkyl group which has a halogen substituent (often chlorine or bromine) on its distal end which results in covalent binding of the compound containing such a moiety to the protein.

The term "Alkenyl" refers to linear, branch-chained or cyclic C₂-C₁₀ (preferably C₂-C₆) hydrocarbon radicals containing at least one C=C bond.

The term "Alkynyl" refers to linear, branch-chained or cyclic C₂-C₁₀ (preferably C₂-C₆) hydrocarbon radicals containing at least one C≡C bond.

The term "alkylene" when used, refers to a -(CH₂)ₙ- group (n is an integer generally from 0-6), which may be optionally substituted. When substituted, the alkylene group preferably is substituted on one or more of the methylene groups with a C₁-C₆ alkyl group (including a cyclopropyl group or a t-butyl group), but may also be substituted with one or more halo groups, preferably from 1 to 3 halo groups or one or two hydroxyl groups, O-(C₁-C₆ alkyl) groups or amino acid sidechains as otherwise disclosed herein. For example, an alkylene group may be substituted with a urethane or alkoxy group (or other group) which is further substituted with a polyethylene glycol chain (of from 1 to 10, preferably 1 to 6, often 1 to 4 ethylene glycol units) to which is substituted (preferably, but not exclusively on the distal end of the polyethylene glycol chain) an alkyl chain substituted with a single halogen group, preferably a chlorine group. For example, the alkylene (often, a methylene) group, may be substituted with an amino acid sidechain group such as a sidechain group of a natural or unnatural amino acid, for example, alanine, β-alanine, arginine, asparagine, aspartic acid, cysteine, cystine, glutamic acid, glutamine, glycine, phenylalanine, histidine, isoleucine, lysine, leucine, methionine, proline, serine, threonine, valine, tryptophan or tyrosine.

The term "unsubstituted" shall mean substituted only with hydrogen atoms. A range of carbon atoms which includes C₀ means that carbon is absent and is replaced with H. Thus, a range of carbon atoms which is C₀-C₆ includes carbons atoms of 1, 2, 3, 4, 5 and 6 and for C₀, H stands in place of carbon.

The term "substituted" or "optionally substituted" shall mean independently (i.e., where more than substituent occurs, each substituent is independent of another substituent) one or more substituents (independently up to five substitutents, preferably up to three substituents, often 1 or 2 substituents on a moiety in a compound according to the present invention and may include substituents which themselves may be further substituted) at a carbon (or nitrogen) position anywhere on a molecule within context, and includes as substituents hydroxyl, thiol, carboxyl, cyano (C≡N), nitro (NO₂), halogen (preferably, 1, 2 or 3 halogens, especially on an alkyl, especially a methyl group such as a trifluoromethyl), an alkyl group (preferably, C₁-C₁₀, more preferably, C₁-C₆), aryl (especially phenyl and substituted phenyl for example benzyl or benzoyl), alkoxy group (preferably, C₁-C₆ alkyl or aryl, including phenyl and substituted phenyl), thioether (C₁-C₆ alkyl or aryl), acyl (preferably, C₁-C₆ acyl), ester or thioester (preferably, C₁-C₆ alkyl or aryl) including alkylene ester (such that attachment is on the alkylene group, rather than at the ester function which is preferably substituted with a C₁-C₆ alkyl or aryl group), preferably, C₁-C₆ alkyl or aryl, halogen (preferably, F or Cl), amine (including a five- or six-membered cyclic alkylene amine, further including a C₁-C₆ alkyl amine or a C₁-C₆ dialkyl amine which alkyl groups may be substituted with one or two hydroxyl groups) or an optionally substituted -N(C₀-C₆ alkyl)C(O)(O-C₁-C₆ alkyl) group (which may be optionally substituted with a polyethylene glycol chain to which is further bound an alkyl group containing a single halogen, preferably chlorine substituent), hydrazine, amido, which is preferably substituted with one or two C₁-C₆ alkyl groups (including a carboxamide which is optionally substituted with one or two C₁-C₆ alkyl groups), alkanol (preferably, C₁-C₆ alkyl or aryl), or alkanoic acid (preferably, C₁-C₆ alkyl or aryl). Substituents according to the present invention may include, for example -SiR₁R₂R₃ groups where each of R₁ and R₂ is as otherwise described herein and R₃ is H or a C₁-C₆ alkyl group, preferably R₁, R₂, R₃ in this context is a C₁-C₃ alkyl group (including an isopropyl or t-butyl group). Each of the above-described groups may be linked directly to the substituted moiety or alternatively, the substituent may be linked to the substituted moiety (preferably in the case of an aryl or heteraryl moiety) through an optionally substituted -(CH₂)ₘ- or alternatively an optionally substituted -(OCH₂)ₘ-, - (OCH₂CH₂)ₘ- or -(CH₂CH₂O)ₘ- group, which may be substituted with any one or more of the above-described substituents. Alkylene groups -(CH₂)ₘ- or -(CH₂)ₙ- groups or other chains such as ethylene glycol chains, as identified above, may be substituted anywhere on the chain. Preferred substitutents on alkylene groups include halogen or C₁-C₆ (preferably C₁-C₃) alkyl groups, which may be optionally substituted with one or two hydroxyl groups, one or two ether groups (O-C₁-C₆ groups), up to three halo groups (preferably F), or a sidechain of an amino acid as otherwise described herein and optionally substituted amide (preferably carboxamide substituted as described above) or urethane groups (often with one or two C₀-C₆ alkyl substitutents, which group(s) may be further substituted). The alkylene group (often a single methylene group) may be substituted with one or two optionally substituted C₁-C₆ alkyl groups, preferably C₁-C₄ alkyl group, most often methyl or O-methyl groups or a sidechain of an amino acid as otherwise described herein. A moiety in a molecule described herein may be optionally substituted with up to five substituents, preferably up to three substituents. For example, described herein are moieties which are substituted with one or two substituents.

The term "substituted" (each substituent being independent of any other substituent) shall also mean within its context of use C₁-C₆ alkyl, C₁-C₆ alkoxy, halogen, amido, carboxamido, sulfone, including sulfonamide, keto, carboxy, C₁-C₆ ester (oxyester or carbonylester), C₁-C₆ keto, urethane -O-C(O)-NR₁R₂ or -N(R₁)-C(O)-O-R₁, nitro, cyano and amine (especially including a C₁-C₆ alkylene-NR₁R₂, a mono- or di- C₁-C₆ alkyl substituted amines which may be optionally substituted with one or two hydroxyl groups). Each of these groups contain unless otherwise indicated, within context, between 1 and 6 carbon atoms. For example, preferred substituents may include for example, -NH-, -NHC(O)-, -O-, =O, -(CH₂)ₘ-(here, m and n are in context, 1, 2, 3, 4, 5 or 6), -S-, -S(O)-, SO₂- or -NH-C(O)-NH-, - (CH₂)ₙOH, -(CH₂)ₙSH, -(CH₂)ₙCOOH, C₁-C₆ alkyl, -(CH₂)ₙO-(C₁-C₆ alkyl), -(CH₂)ₙC(O)-(C₁-C₆ alkyl), -(CH₂)ₙOC(O)-(C₁-C₆ alkyl), -(CH₂)ₙC(O)O-(C₁-C₆ alkyl), -(CH₂)ₙNHC(O)-R₁, - (CH₂)ₙC(O)-NR₁R₂, -(OCH₂)ₙOH, -(CH₂O)ₙCOOH, C₁-C₆ alkyl, -(OCH₂)ₙO-(C₁-C₆ alkyl), - (CH₂O)ₙC(O)-(C₁-C₆ alkyl), -(OCH₂)ₙNHC(O)-R₁, -(CH₂O)ₙC(O)-NR₁R₂, -S(O)₂-R_{S}, -S(O)-R_{S} (R_{S} is C₁-C₆ alkyl or a -(CH₂)ₘ-NR₁R₂ group), NO₂, CN or halogen (F, Cl, Br, I, preferably F or Cl), depending on the context of the use of the substituent. R₁ and R₂ are each, within context, H or a C₁-C₆ alkyl group (which may be optionally substituted with one or two hydroxyl groups or up to three halogen groups, preferably fluorine). The term "substituted" shall also mean, within the chemical context of the compound defined and substituent used, an optionally substituted aryl or heteroaryl group or an optionally substituted heterocyclic group as otherwise described herein. Alkylene groups may also be substituted as otherwise disclosed herein, preferably with optionally substituted C₁-C₆ alkyl groups (methyl, ethyl or hydroxymethyl or hydroxyethyl is preferred, thus providing a chiral center), a sidechain of an amino acid group as otherwise described herein, an amido group as described hereinabove, or a urethane group O-C(O)-NR₁R₂ group where R₁ and R₂ are as otherwise described herein, although numerous other groups may also be used as substituents. Various optionally substituted moieties may be substituted with 3 or more substituents, preferably no more than 3 substituents and preferably with 1 or 2 substituents. It is noted that in instances where, in a compound at a particular position of the molecule substitution is required (principally, because of valency), but no substitution is indicated, then that substituent is construed or understood to be H, unless the context of the substitution suggests otherwise.

The term "aryl" or "aromatic", in context, refers to a substituted (as otherwise described herein) or unsubstituted monovalent aromatic radical having a single ring (e.g., benzene, phenyl, benzyl) or condensed rings (e.g., naphthyl, anthracenyl, phenanthrenyl, etc.) and can be bound to the compound according to the present invention at any available stable position on the ring(s) or as otherwise indicated in the chemical structure presented.

The term "substituted aryl" refers to an aromatic carbocyclic group comprised of at least one aromatic ring or of multiple condensed rings at least one of which being aromatic, wherein the ring(s) are substituted with one or more substituents. For example, an aryl group can comprise a substituent(s) selected from: -(CH₂)ₙOH, -(CH₂)ₙ-O-(C₁-C₆)alkyl, -(CH₂)ₙ-O-(CH₂)ₙ-(C₁-C₆)alkyl, -(CH₂)ₙ-C(O)(C₀-C₆) alkyl, -(CH₂)ₙ-C(O)O(C₀-C₆)alkyl, -(CH₂)ₙ-OC(O)(C₀-C₆)alkyl, amine, mono- or di-(C₁-C₆ alkyl) amine wherein the alkyl group on the amine is optionally substituted with 1 or 2 hydroxyl groups or up to three halo (preferably F, Cl) groups, OH, COOH, C₁-C₆ alkyl, preferably CH₃, CF₃, OMe, OCF₃, NO₂, or CN group (each of which may be substituted in ortho-, meta- and/or para- positions of the phenyl ring, preferably para-), an optionally substituted phenyl group (the phenyl group itself is preferably substituted with a linker group attached to a PTM group, including a ULM group), and/or at least one of F, Cl, OH, COOH, CH₃, CF₃, OMe, OCF₃, NO₂, or CN group (in ortho-, meta- and/or para- positions of the phenyl ring, preferably para-), a naphthyl group, which may be optionally substituted.

"Carboxyl" denotes the group --C(O)OR, where R is hydrogen, alkyl, substituted alkyl, aryl, substituted aryl, heteroaryl or substituted heteroaryl, whereas these generic substituents have meanings which are identical with definitions of the corresponding groups defined herein.

The term "heteroaryl" can mean but is in no way limited to an optionally substituted quinoline (which may be attached to the pharmacophore or substituted on any carbon atom within the quinoline ring), an optionally substituted indole (including dihydroindole), an optionally substituted indolizine, an optionally substituted azaindolizine (2, 3 or 4-azaindolizine) an optionally substituted benzimidazole, benzodiazole, benzoxofuran, an optionally substituted imidazole, an optionally substituted isoxazole, an optionally substituted oxazole (preferably methyl substituted), an optionally substituted diazole, an optionally substituted triazole, a tetrazole, an optionally substituted benzofuran, an optionally substituted thiophene, an optionally substituted thiazole (preferably methyl and/or thiol substituted), an optionally substituted isothiazole, an optionally substituted triazole (preferably a 1,2,3-triazole substituted with a methyl group, a triisopropylsilyl group, an optionally substituted -(CH₂)ₘ-O-C₁-C₆ alkyl group or an optionally substituted -(CH₂)ₘ-C(O)-O-C₁-C₆ alkyl group), an optionally substituted pyridine (2-, 3-, or 4-pyridine) or a group according to the chemical structure: 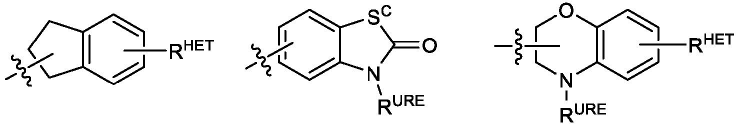 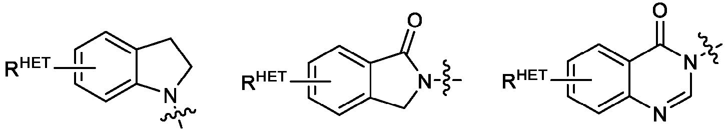 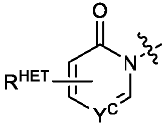 wherein
S^{C} is CHR^{SS}, NR^{URE}, or O;
R^{HET} is H, CN, NO₂, halo (preferably Cl or F), optionally substituted C₁-C₆ alkyl (preferably substituted with one or two hydroxyl groups or up to three halo groups (e.g. CF₃), optionally substituted O(C₁-C₆ alkyl) (preferably substituted with one or two hydroxyl groups or up to three halo groups) or an optionally substituted acetylenic group -C≡C-Rₐ where Rₐ is H or a C₁-C₆ alkyl group (preferably C₁-C₃ alkyl);
R^{SS} is H, CN, NO₂, halo (preferably F or Cl), optionally substituted C₁-C₆ alkyl (preferably substituted with one or two hydroxyl groups or up to three halo groups), optionally substituted O-(C₁-C₆ alkyl) (preferably substituted with one or two hydroxyl groups or up to three halo groups) or an optionally substituted -C(O)(C₁-C₆ alkyl) (preferably substituted with one or two hydroxyl groups or up to three halo groups);
R^{URE} is H, a C₁-C₆ alkyl (preferably H or C₁-C₃ alkyl) or a -C(O)(C₁-C₆ alkyl), each of which groups is optionally substituted with one or two hydroxyl groups or up to three halogen, preferably fluorine groups, or an optionally substituted heterocycle, for example piperidine, morpholine, pyrrolidine, tetrahydrofuran, tetrahydrothiophene, piperidine, piperazine, each of which is optionally substituted, and
Y^{C} is N or C-R^{YC}, where R^{YC} is H, OH, CN, NO₂, halo (preferably Cl or F), optionally substituted C₁-C₆ alkyl (preferably substituted with one or two hydroxyl groups or up to three halo groups (e.g. CF₃), optionally substituted O(C₁-C₆ alkyl) (preferably substituted with one or two hydroxyl groups or up to three halo groups) or an optionally substituted acetylenic group -C≡C-Rₐ where Rₐ is H or a C₁-C₆ alkyl group (preferably C₁-C₃ alkyl).

The terms "aralkyl" and "heteroarylalkyl" refer to groups that comprise both aryl or, respectively, heteroaryl as well as alkyl and/or heteroalkyl and/or carbocyclic and/or heterocycloalkyl ring systems according to the above definitions.

The term "arylalkyl" as used herein refers to an aryl group as defined above appended to an alkyl group defined above. The arylalkyl group is attached to the parent moiety through an alkyl group wherein the alkyl group is one to six carbon atoms. The aryl group in the arylalkyl group may be substituted as defined above.

The term "Heterocycle" refers to a cyclic group which contains at least one heteroatom (e.g., N, O or S), and may be aromatic (heteroaryl) or non-aromatic.

Exemplary heterocyclics include: azetidinyl, benzimidazolyl, 1,4- benzodioxanyl, 1,3-benzodioxolyl, benzoxazolyl, benzothiazolyl, benzothienyl, dihydroimidazolyl, dihydropyranyl, dihydrofuranyl, dioxanyl, dioxolanyl, ethyleneurea, 1,3-dioxolane, 1,3-dioxane, 1,4-dioxane, furyl, homopiperidinyl, imidazolyl, imidazolinyl, imidazolidinyl, indolinyl, indolyl, isoquinolinyl, isothiazolidinyl, isothiazolyl, isoxazolidinyl, isoxazolyl, morpholinyl, naphthyridinyl, oxazolidinyl, oxazolyl, pyridone, 2-pyrrolidone, pyridine, piperazinyl, , N-methylpiperazinyl, piperidinyl, phthalimide, succinimide, pyrazinyl, pyrazolinyl, pyridyl, pyrimidinyl, pyrrolidinyl, pyrrolinyl, pyrrolyl, quinolinyl, tetrahydrofuranyl, tetrahydropyranyl, tetrahydroquinoline, thiazolidinyl, thiazolyl, thienyl, tetrahydrothiophene, oxane, oxetanyl, oxathiolanyl, thiane among others.

Heterocyclic groups can be optionally substituted with a member selected from the group consisting of alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, keto, thioketo, carboxy, carboxyalkyl, thioaryloxy, thioheteroaryloxy, thioheterocyclooxy, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl, oxo (=O), and -SO₂-heteroaryl. Such heterocyclic groups can have a single ring or multiple condensed rings. Examples of nitrogen heterocycles and heteroaryls include, but are not limited to, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, morpholino, piperidinyl, tetrahydrofuranyl, and the like as well as N-alkoxy-nitrogen containing heterocycles. The term "heterocyclic" also includes bicyclic groups in which any of the heterocyclic rings is fused to a benzene ring or a cyclohexane ring or another heterocyclic ring (for example, indolyl, quinolyl, isoquinolyl, tetrahydroquinolyl, and the like).

The term "cycloalkyl" can mean but is in no way limited to univalent groups derived from monocyclic or polycyclic alkyl groups or cycloalkanes, as defined herein, e.g., saturated monocyclic hydrocarbon groups having from three to twenty carbon atoms in the ring, including, but not limited to, cyclopropyl, cyclobutyl, cyclopentyl, cyclohexyl, cycloheptyl and the like. The term "substituted cycloalkyl" can mean but is in no way limited to a monocyclic or polycyclic alkyl group and being substituted by one or more substituents, for example, amino, halogen, alkyl, substituted alkyl, carbyloxy, carbylmercapto, aryl, nitro, mercapto or sulfo, whereas these generic substituent groups have meanings which are identical with definitions of the corresponding groups as defined in this legend.

"Heterocycloalkyl" refers to a monocyclic or polycyclic alkyl group in which at least one ring carbon atom of its cyclic structure being replaced with a heteroatom selected from the group consisting of N, O, S or P. "Substituted heterocycloalkyl" refers to a monocyclic or polycyclic alkyl group in which at least one ring carbon atom of its cyclic structure being replaced with a heteroatom selected from the group consisting of N, O, S or P and the group is containing one or more substituents selected from the group consisting of halogen, alkyl, substituted alkyl, carbyloxy, carbylmercapto, aryl, nitro, mercapto or sulfo, whereas these generic substituent group have meanings which are identical with definitions of the corresponding groups as defined in this legend.

The term "hydrocarbyl" shall mean a compound which contains carbon and hydrogen and which may be fully saturated, partially unsaturated or aromatic and includes aryl groups, alkyl groups, alkenyl groups and alkynyl groups.

In any of the compounds described herein, the W, X, Y, Z, G, G', R, R', R", Q1-Q4, A, and Rn may independently be covalently coupled to a linker and/or a linker to which is attached one or more PTM, ULM, CLM or CLM' groups.

More specifically, non-limiting examples of CLMs include those shown below as well as those 'hybrid' molecules that arise from the combination of 1 or more of the different features shown in the molecules below.

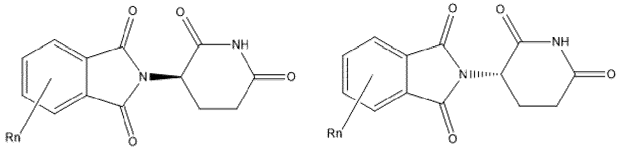

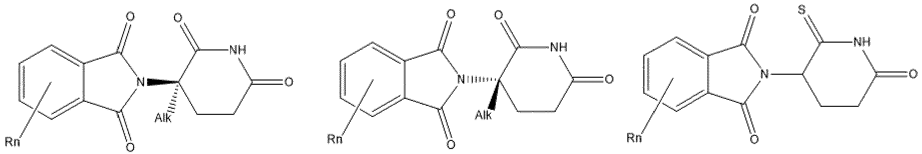

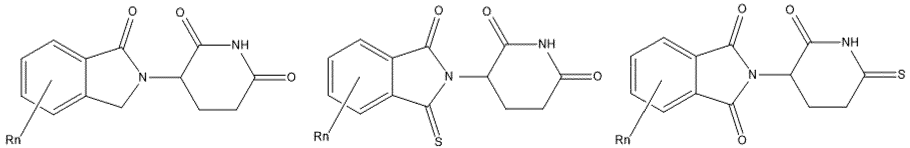

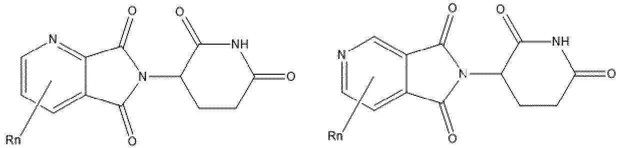

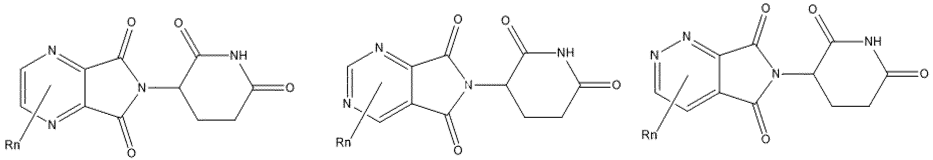

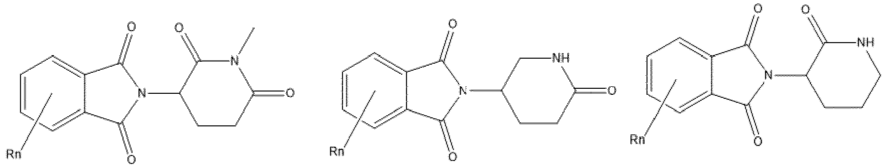

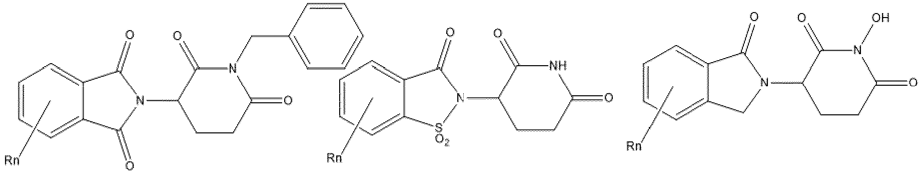

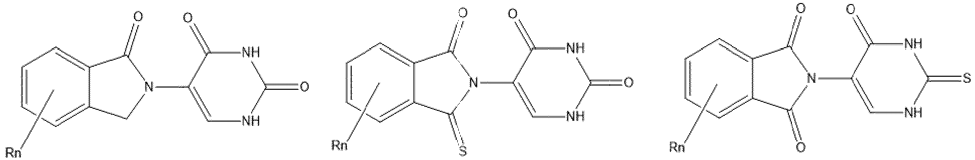

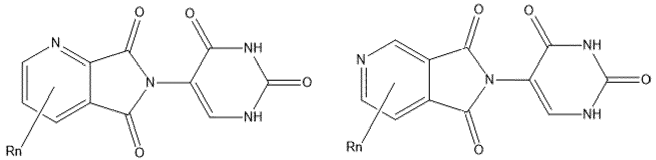

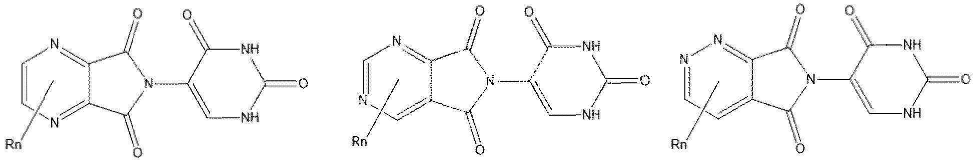

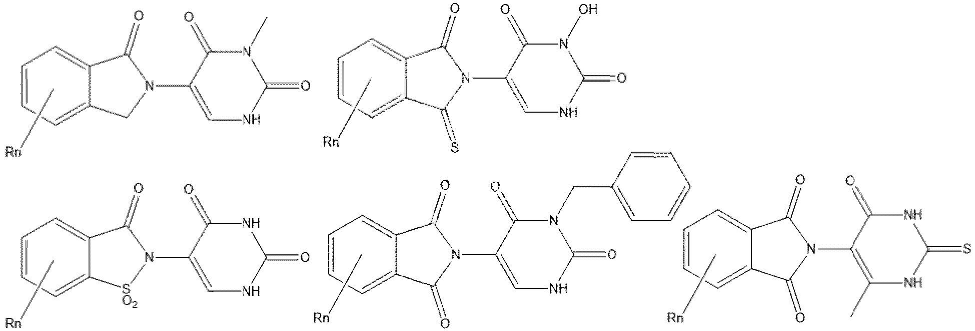

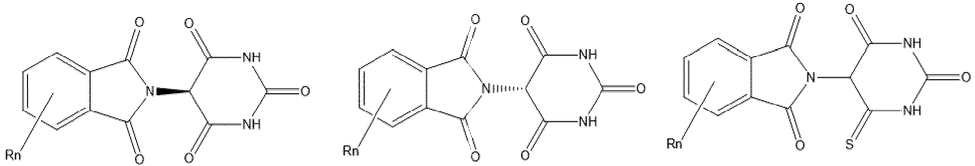

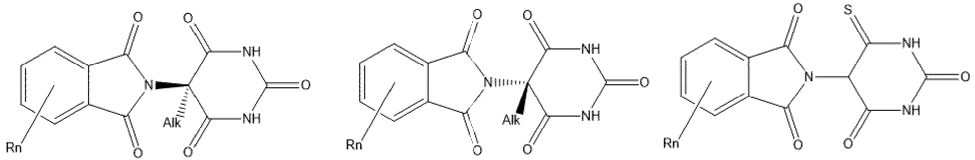

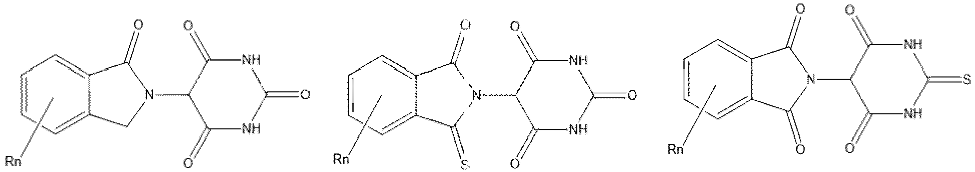

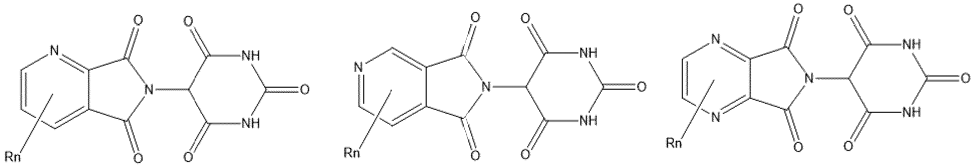

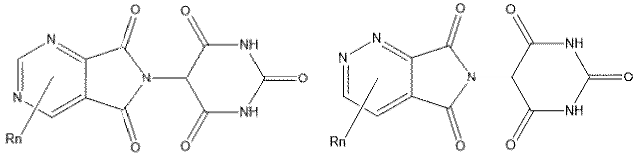

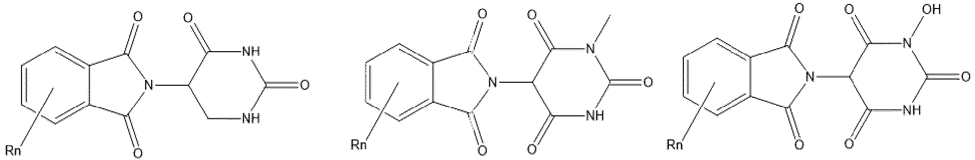

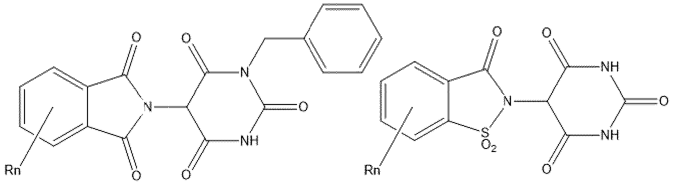

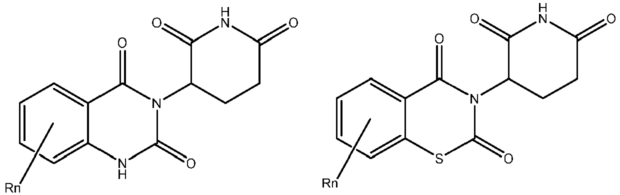

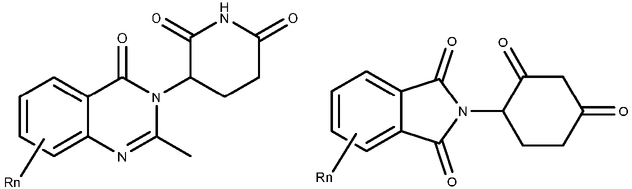

### Exemplary Linkers

The compounds as described herein may be chemically linked or coupled via a chemical linker (L). In general and for example, the linker group L may be a group comprising one or more covalently connected structural units of A (e.g., -A₁...A_{q}- ), wherein A₁ is a group coupled to at least one of a ULM, a PTM, or a combination thereof. For example, A₁ may link a ULM, a PTM, or a combination thereof directly to another ULM, PTM, or combination thereof. For example, A₁ may link a ULM, a PTM, or a combination thereof indirectly to another ULM, PTM, or combination thereof through A_{q}.

For example, A₁ to A_{q} may be, each independently, a bond, CR^{L1}R^{L2}, O, S, SO, SO₂, NR^{L3}, SO₂NR^{L3}, SONR^{L3}, CONR^{L3}, NR^{L3}CONR^{L4}, NR^{L3}SO₂NR^{L4}, Co, CR^{L1}=CR^{L2}, C≡C, SiR^{L1}R^{L2}, P(O)R^{L1}, P(O)OR^{L1}, NR^{L3}C(=NCN)NR^{L4}, NR^{L3}C(=NCN), NR^{L3}C(=CNO₂)NR^{L4}, C₃₋₁₁cycloalkyl optionally substituted with 0-6 R^{L1} and/or R^{L2} groups, C₃₋₁₁heteocyclyl optionally substituted with 0-6 R^{L1} and/or R^{L2} groups, aryl optionally substituted with 0-6 R^{L1} and/or R^{L2} groups, heteroaryl optionally substituted with 0-6 R^{L1} and/or R^{L2} groups, where R^{L1} or R^{L2}, each independently, can be linked to other A groups to form cycloalkyl and/or heterocyclyl moeity which can be further substituted with 0-4 R^{L5} groups; wherein
R^{L1}, R^{L2}, R^{L3}, R^{L4} and R^{L5} are, each independently, H, halo, C₁₋₈alkyl, OC₁₋₈alkyl, SC₁₋₈alkyl, NHC₁₋₈alkyl, N(C₁₋₈alkyl)₂, C₃₋₁₁cycloalkyl, aryl, heteroaryl, C₃₋₁₁heterocyclyl, OC₁₋₈cycloalkyl, SC₁₋₈cycloalkyl, NHC₁₋₈cycloalkyl, N(C₁₋₈cycloalkyl)₂, N(C₁₋₈cycloalkyl)(C₁₋₈alkyl), OH, NH₂, SH, SO₂C₁₋₈alkyl, P(O)(OC₁₋₈alkyl)(C₁₋₈alkyl), P(O)(OC₁₋₈alkyl)₂, CC-C₁₋₈alkyl, CCH, CH=CH(C₁₋₈alkyl), C(C₁₋₈alkyl)=CH(C₁₋₈alkyl), C(C₁₋₈alkyl)=C(C₁₋₈alkyl)₂, Si(OH)₃, Si(C₁₋₈alkyl)₃, Si(OH)(C₁₋₈alkyl)₂, COC₁₋₈alkyl, CO₂H, halogen, CN, CF₃, CHF₂, CH₂F, NO₂, SF₅, SO₂NHC₁₋₈alkyl, SO₂N(C₁₋₈alkyl)₂, SONHC₁₋₈alkyl, SON(C₁₋₈alkyl)₂, CONHC₁₋₈alkyl, CON(C₁₋₈alkyl)₂, N(C₁₋₈alkyl)CONH(C₁₋₈alkyl), N(C₁₋₈alkyl)CON(C₁₋₈alkyl)₂, NHCONH(C₁₋₈alkyl), NHCON(C₁₋₈alkyl)₂, NHCONH₂, N(C₁₋₈alkyl)SO₂NH(C₁₋₈alkyl), N(C₁₋₈alkyl) SO₂N(C₁₋₈alkyl)₂, NH SO₂NH(C₁₋₈alkyl), NH SO₂N(C₁₋₈alkyl)₂, NH SO₂NH₂.

For example, q may be an integer greater than or equal to 0. For example, q may be an integer greater than or equal to 1.

For example, where q is greater than 2, A_{q} may be a group which is connected to a ULM or ULM' moiety, and A₁ and A_{q} are connected via structural units of A (number of such structural units of A: q-2).

For example, where q is 2, A_{q} may be a group which is connected to A₁ and to a ULM or ULM' moiety.

For example, where q is 1, the structure of the linker group L may be -A₁-, and A₁ is a group which is connected to a ULM or ULM' moiety and a PTM moiety.

For example, q may be an integer from 1 to 100, 1 to 90, 1 to 80, 1 to 70, 1 to 60, 1 to 50, 1 to 40, 1 to 30, 1 to 20, or 1 to 10.

For example, the linker (L) is selected from the group consisting of:

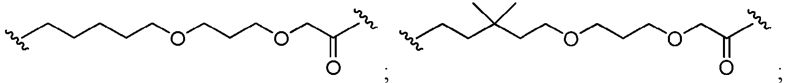

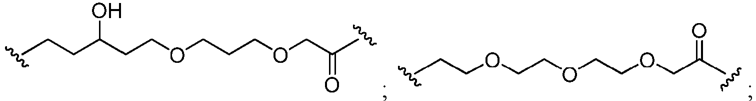

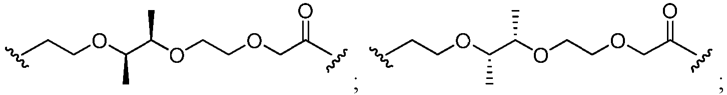

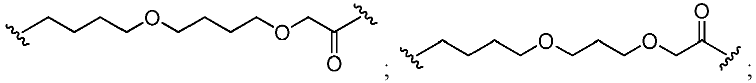

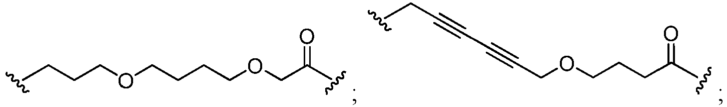

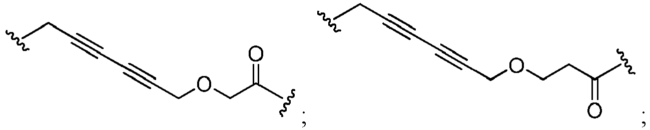

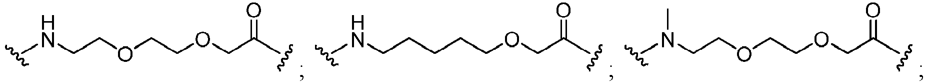

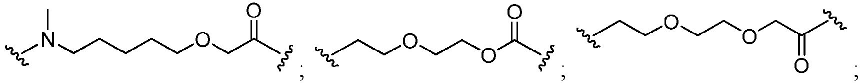

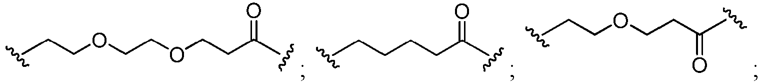

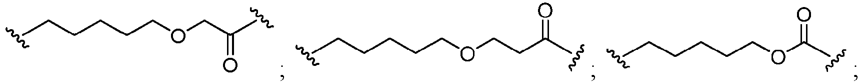

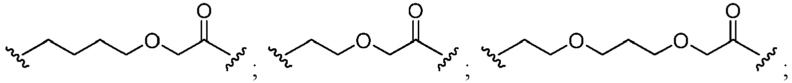

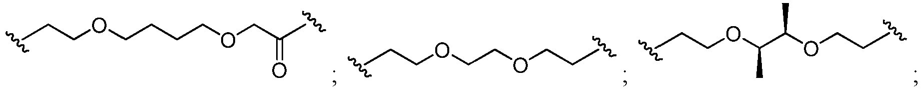

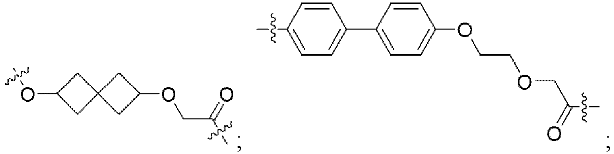

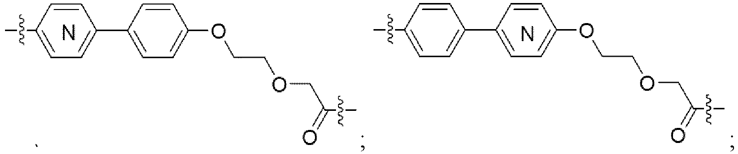

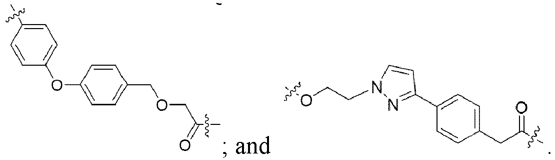

For example, the linker group may be optionally substituted (poly)ethyleneglycol having between 1 and about 100 ethylene glycol units, between about 1 and about 50 ethylene glycol units, between 1 and about 25 ethylene glycol units, between about 1 and 10 ethylene glycol units, between 1 and about 8 ethylene glycol units and 1 and 6 ethylene glycol units, between 2 and 4 ethylene glycol units, or optionally substituted alkyl groups interdispersed with optionally substituted, O, N, S, P or Si atoms. The linker may be substituted with an aryl, phenyl, benzyl, alkyl, alkylene, or heterocycle group. The linker may be asymmetric or symmetrical.

For example, the linker group may be any suitable moiety as described herein. For example, the linker may be a substituted or unsubstituted polyethylene glycol group ranging in size from about 1 to about 12 ethylene glycol units, between 1 and about 10 ethylene glycol units, about 2 about 6 ethylene glycol units, between about 2 and 5 ethylene glycol units, between about 2 and 4 ethylene glycol units.

For example, although the CLM (or ULM) group and PTM group may be covalently linked to the linker group through any group which is appropriate and stable to the chemistry of the linker, the linker may be independently covalently bonded to the CLM group and the PTM group preferably through an amide, ester, thioester, keto group, carbamate (urethane), carbon or ether, each of which groups may be inserted anywhere on the CLM group and PTM group to provide maximum binding of the CLM group on the ubiquitin ligase and the PTM group on the target protein to be degraded. (It is noted, for example, that where the PTM group is a ULM group, the target protein for degradation may be the ubiquitin ligase itself). For example, the linker may be linked to an optionally substituted alkyl, alkylene, alkene or alkyne group, an aryl group or a heterocyclic group on the CLM and/or PTM groups.

### Exemplary PTMs

In some aspects, PTM is a small molecule protein target moiety that binds specifically to estrogen receptor, androgen receptor, or a BET bromodomain protein.

In preferred aspects of the invention, the PTM group is a group, which binds to target proteins. In general and for example, targets of a PTM group are numerous in kind and are selected from proteins that are expressed in a cell such that at least a portion of the sequences is found in the cell and may bind to a PTM group. The term "protein" includes oligopeptides and polypeptide sequences of sufficient length that they can bind to a PTM group. Any protein in a eukaryotic system or a microbial system, including a virus, bacteria or fungus, as otherwise described herein, are targets for ubiquitination mediated by the compounds. For example, preferably, the target protein is a eukaryotic protein. For example, the protein binding moiety may be a haloalkane, preferably a C₁-C₁₀ alkyl group which is substituted with at least one halo group, preferably a halo group at the distal end of the alkyl group (i.e., away from the linker or CLM group), which may covalently bind to a dehalogenase enzyme in a patient or subject or in a diagnostic assay.

For example, described herein are PTM groups being any moiety which binds to a protein specifically (binds to a target protein) and includes the following non-limiting examples of small molecule target protein moieties: Hsp90 inhibitors, kinase inhibitors, HDM2 & MDM2 inhibitors, compounds targeting Human BET Bromodomain-containing proteins, HDAC inhibitors, human lysine methyltransferase inhibitors, angiogenesis inhibitors, nuclear hormone receptor compounds, immunosuppressive compounds, and compounds targeting the aryl hydrocarbon receptor (AHR), among numerous others. The compositions described below exemplify some of the members of these nine types of small molecule target protein binding moieties. Such small molecule target protein binding moieties also include pharmaceutically acceptable salts, enantiomers, solvates and polymorphs of these compositions, as well as other small molecules that may target a protein of interest. For example, these binding moieties may be linked to the ubiquitin ligase binding moiety preferably through a linker in order to present a target protein (to which the protein target moiety is bound) in proximity to the ubiquitin ligase for ubiquitination and degradation.

For example, any protein which can bind to a protein target moiety or PTM group and acted on or degraded by a ubiquitin ligase can be a target protein. In general, target proteins may include, for example, structural proteins, receptors, enzymes, cell surface proteins, proteins pertinent to the integrated function of a cell, including proteins involved in catalytic activity, aromatase activity, motor activity, helicase activity, metabolic processes (anabolism and catrabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein, lipid carbohydrate), receptor activity, cell motility, membrane fusion, cell communication, regulation of biological processes, development, cell differentiation, response to stimulus, behavioral proteins, cell adhesion proteins, proteins involved in cell death, proteins involved in transport (including protein transporter activity, nuclear transport, ion transporter activity, channel transporter activity, carrier activity, permease activity, secretion activity, electron transporter activity, pathogenesis, chaperone regulator activity, nucleic acid binding activity, transcription regulator activity, extracellular organization and biogenesis activity, translation regulator activity. Proteins of interest can include proteins from eurkaryotes and prokaryotes including humans as targets for drug therapy, other animals, including domesticated animals, microbials for the determination of targets for antibiotics and other antimicrobials and plants, and even viruses, among numerous others.

For example, the PTM group may be a haloalkyl group, wherein said alkyl group generally ranges in size from about 1 or 2 carbons to about 12 carbons in length, often about 2 to 10 carbons in length, often about 3 carbons to about 8 carbons in length, more often about 4 carbons to about 6 carbons in length. The haloalkyl groups are generally linear alkyl groups (although branched-chain alkyl groups may also be used) and are end-capped with at least one halogen group, preferably a single halogen group, often a single chloride group. Haloalkyl PT groups, for example, are preferably represented by the chemical structure - (CH₂)ᵥ-Halo where v is any integer from 2 to about 12, often about 3 to about 8, more often about 4 to about 6. Halo may be any halogen, but is preferably Cl or Br, more often Cl.

For example, compounds described herein may be used to treat a number of disease states and/or conditions, including any disease state and/or condition in which proteins are dysregulated and where a patient would benefit from the degradation of proteins.

Also described herein are therapeutic compositions comprising an effective amount of a compound as described herein or salt form thereof, and a pharmaceutically acceptable carrier, additive or excipient, and optionally an additional bioactive agent. The therapeutic compositions modulate protein degradation in a patient or subject, for example, an animal such as a human, and can be used for treating or ameliorating disease states or conditions which are modulated through the degraded protein. For example, the therapeutic compositions as described herein may be used to effectuate the degradation of proteins of interest for the treatment or amelioration of a disease, e.g., cancer. For example, the disease is multiple myeloma.

For example, described herein is a method for treating a disease state or ameliorating the symptoms of a disease or condition in a subject in need thereof by degrading a protein or polypeptide through which a disease state or condition is modulated comprising administering to said patient or subject an effective amount, e.g., a therapeutically effective amount, of at least one compound as described hereinabove, optionally in combination with a pharmaceutically acceptable carrier, additive or excipient, and optionally an additional bioactive agent, wherein the composition is effective for treating or ameliorating the disease or disorder or symptom thereof in the subject. The method may be used to treat a large number of disease states or conditions including cancer, by virtue of the administration of effective amounts of at least one compound described herein. The disease state or condition may be a disease caused by overexpression of a protein, which leads to a disease state and/or condition.

Also described are methods for identifying the effects of the degradation of proteins of interest in a biological system using compounds according to the present invention.

The term "target protein" is used to describe a protein or polypeptide, which is a target for binding to a compound according to the present invention and degradation by ubiquitin ligase hereunder. Such small molecule target protein binding moieties also include pharmaceutically acceptable salts, enantiomers, solvates and polymorphs of these compositions, as well as other small molecules that may target a protein of interest. These binding moieties are linked to CLM groups through linker groups L.

For example, target proteins which may be bound to a protein target moiety and degraded by the ligase to which the ubiquitin ligase binding moiety is bound include any protein or peptide, including fragments thereof, analogues thereof, and/or homologues thereof. For example, target proteins may include proteins and peptides having any biological function or activity including structural, regulatory, hormonal, enzymatic, genetic, immunological, contractile, storage, transportation, and signal transduction. For example, the target proteins may include structural proteins, receptors, enzymes, cell surface proteins, proteins pertinent to the integrated function of a cell, including proteins involved in catalytic activity, aromatase activity, motor activity, helicase activity, metabolic processes (anabolism and catrabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein, lipid carbohydrate), receptor activity, cell motility, membrane fusion, cell communication, regulation of biological processes, development, cell differentiation, response to stimulus, behavioral proteins, cell adhesion proteins, proteins involved in cell death, proteins involved in transport (including protein transporter activity, nuclear transport, ion transporter activity, channel transporter activity, carrier activity, permease activity, secretion activity, electron transporter activity, pathogenesis, chaperone regulator activity, nucleic acid binding activity, transcription regulator activity, extracellular organization and biogenesis activity, translation regulator activity. For example, proteins of interest can include proteins from eurkaryotes and prokaryotes, including microbes, viruses, fungi and parasites, including humans, microbes, viruses, fungi and parasites, among numerous others, as targets for drug therapy, other animals, including domesticated animals, microbials for the determination of targets for antibiotics and other antimicrobials and plants, and even viruses, among numerous others.

For example, a number of drug targets for human therapeutics represent protein targets to which protein target moiety may be bound and incorporated into compounds described herein. These include proteins which may be used to restore function in numerous polygenic diseases, including for example B7.1 and B7, TINFRlm, TNFR2, NADPH oxidase, BclIBax and other partners in the apotosis pathway, C5a receptor, HMG-CoA reductase, PDE V phosphodiesterase type, PDE IV phosphodiesterase type 4, PDE I, PDEII, PDEIII, squalene cyclase inhibitor, CXCR1, CXCR2, nitric oxide (NO) synthase, cyclo-oxygenase 1, cyclo-oxygenase 2, 5HT receptors, dopamine receptors, G Proteins, i.e., Gq, histamine receptors, 5-lipoxygenase, tryptase serine protease, thymidylate synthase, purine nucleoside phosphorylase, GAPDH trypanosomal, glycogen phosphorylase, Carbonic anhydrase, chemokine receptors, JAW STAT, RXR and similar, HIV 1 protease, HIV 1 integrase, influenza, neuramimidase, hepatitis B reverse transcriptase, sodium channel, multi drug resistance (MDR), protein P-glycoprotein (and MRP), tyrosine kinases, CD23, CD124, tyrosine kinase p56 lck, CD4, CD5, IL-2 receptor, IL-1 receptor, TNF-alphaR, ICAM1, Cat+ channels, VCAM, VLA-4 integrin, selectins, CD40/CD40L, newokinins and receptors, inosine monophosphate dehydrogenase, p38 MAP Kinase, RaslRaflMEWERK pathway, interleukin-1 converting enzyme, caspase, HCV, NS3 protease, HCV NS3 RNA helicase, glycinamide ribonucleotide formyl transferase, rhinovirus 3C protease, herpes simplex virus-1 (HSV-I), protease, cytomegalovirus (CMV) protease, poly (ADP-ribose) polymerase, cyclin dependent kinases, vascular endothelial growth factor, oxytocin receptor, microsomal transfer protein inhibitor, bile acid transport inhibitor, 5 alpha reductase inhibitors, angiotensin 11, glycine receptor, noradrenaline reuptake receptor, endothelin receptors, neuropeptide Y and receptor, estrogen receptors, androgen receptors, adenosine receptors, adenosine kinase and AMP deaminase, purinergic receptors (P2Y1, P2Y2, P2Y4, P2Y6, P2X1-7), farnesyltransferases, geranylgeranyl transferase, TrkA a receptor for NGF, beta-amyloid, tyrosine kinase Flk-IIKDR, vitronectin receptor, integrin receptor, Her-21 neu, telomerase inhibition, cytosolic phospholipaseA2 and EGF receptor tyrosine kinase. Additional protein targets include, for example, ecdysone 20-monooxygenase, ion channel of the GABA gated chloride channel, acetylcholinesterase, voltage-sensitive sodium channel protein, calcium release channel, and chloride channels. Still further target proteins include Acetyl-CoA carboxylase, adenylosuccinate synthetase, protoporphyrinogen oxidase, and enolpyruvylshikimate-phosphate synthase.

For example, haloalkane dehalogenase enzymes are another target of some specific compounds described herein. Said compounds, which contain chloroalkane peptide binding moieties (C₁-C₁₂ often about C₂-C₁₀ alkyl halo groups) may be used to inhibit and/or degrade haloalkane dehalogenase enzymes which are used in fusion proteins or related dioagnostic proteins as described in PCT/US2012/063401 filed December 6, 2011 and published as WO 2012/078559 on June 14, 2012.

These various protein targets may be used in screens that identify compound moieties which bind to the protein, the level of activity of the protein may be altered for therapeutic end result.

The term "protein target moiety" or PTM is used to describe a small molecule which binds to a target protein or other protein or polypeptide of interest and places/presents that protein or polypeptide in proximity to an ubiquitin ligase such that degradation of the protein or polypeptide by ubiquitin ligase may occur. For example, non-limiting examples of small molecule target protein binding moieties include Hsp90 inhibitors, kinase inhibitors, MDM2 inhibitors, compounds targeting Human BET Bromodomain-containing proteins, HDAC inhibitors, human lysine methyltransferase inhibitors, angiogenesis inhibitors, immunosuppressive compounds, and compounds targeting the aryl hydrocarbon receptor (AHR), among numerous others. The compositions described below exemplify some of the members of these nine types of small molecule target protein.

Exemplary protein target moieties given for example include, haloalkane halogenase inhibitors, Hsp90 inhibitors, kinase inhibitors, MDM2 inhibitors, compounds targeting Human BET Bromodomain-containing proteins. HDAC inhibitors, human lysine methyltransferase inhibitors, angiogenesis inhibitors, immunosuppressive compounds, and compounds targeting the aryl hydrocarbon receptor (AHR).

The compositions described below exemplify some of the members of these types of small molecule target protein binding moieties. Such small molecule target protein binding moieties also include pharmaceutically acceptable salts, enantiomers, solvates and polymorphs of these compositions, as well as other small molecules that may target a protein of interest. Examples below which do not form part of the present invention are given for reference purposes only and are marked accordingly with "reference".

### I. Heat Shock Protein 90 (HSP90) Inhibitors (reference):

HSP90 inhibitors as used herein include, but are not limited to:
1. The HSP90 inhibitors identified in Vallee, et al., "Tricyclic Series of Heat Shock Protein 90 (HSP90) Inhibitors Part I: Discovery of Tricyclic Imidazo[4,5-C]Pyridines as Potent Inhibitors of the HSP90 Molecular Chaperone (2011) J.Med.Chem. 54: 7206, including YKB (N-[4-(3H-imidazo[4,5-C]Pyridin-2-yl)-9H-Fluoren-9-yl]-succinamide): 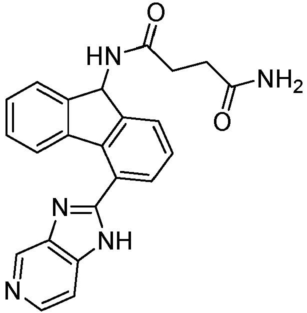 derivatized where a linker group L or a -(L-CLM) group is attached, for example, via the terminal amide group;
**2**. The HSP90 inhibitor p54 (modified) (8-[(2,4-dimethylphenyl)sulfanyl]-3]pent-4-yn-1-yl-3H-purin-6-amine): 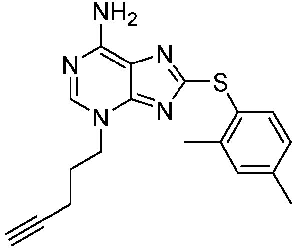 where a linker group L or a -(L-CLM) group is attached, for example, via the terminal acetylene group;
**3**. The HSP90 inhibitors (modified) identified in Brough, et al., "4,5-Diarylisoxazole HSP90 Chaperone Inhibitors: Potential Therapeutic Agents for the Treatment of Cancer", J.MED.CHEM. vol: 51, pag: 196 (2008), including the compound 2GJ (5-[2,4-dihydroxy-5-(1-methylethyl)phenyl]-n-ethyl-4-[4-(morpholin-4-ylmethyl)phenyl]isoxazole-3-carboxamide) having the structure: 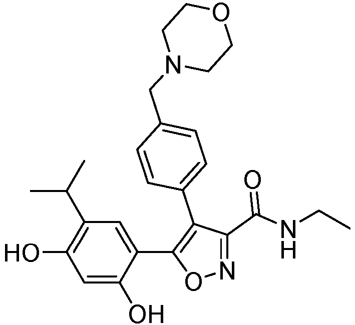 derivatized, where a linker group L or a -(L-CLM) group is attached, for example, via the amide group (at the amine or at the alkyl group on the amine);
**4**. The HSP90 inhibitors (modified) identified in Wright, et al., Structure-Activity Relationships in Purine-Based Inhibitor Binding to HSP90 Isoforms, Chem Biol. 2004 Jun;11(6):775-85, including the HSP90 inhibitor PU3 having the structure: 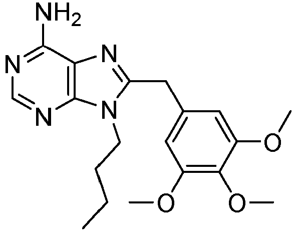 where a linker group L or -(L-CLM) is attached, for example, via the butyl group; and
**5**. The HSP90 inhibitor geldanamycin ((4*E*,6*Z*,8*S*,9*S*,10*E*,12*S*,13*R*,14*S*,16*R*)-13-hydroxy-8,14,19-trimethoxy-4,10,12,16-tetramethyl-3,20,22-trioxo-2-azabicyclo[16.3.1] (derivatized) or any of its derivatives (e.g. 17-alkylamino-17-desmethoxygeldanamycin ("17-AAG") or 17-(2-dimethylaminoethyl)amino-17-desmethoxygeldanamycin ("17-DMAG")) (derivatized, where a linker group L or a-(L-CLM) group is attached, for example, via the amide group).

### II. Kinase and Phosphatase Inhibitors (reference):

Kinase inhibitors as used herein include, but are not limited to:
**1**. Erlotinib Derivative Tyrosine Kinase Inhibitor: 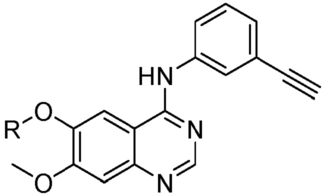 where R is a linker group L or a -(L-CLM) group attached, for example, via the ether group;
**2**. The kinase inhibitor sunitinib (derivatized): 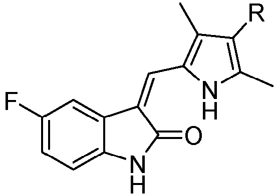 (derivatized where R is a linker group L or a -(L-CLM) group attached, for example, to the pyrrole moiety);
**3**. Kinase Inhibitor sorafenib (derivatized): 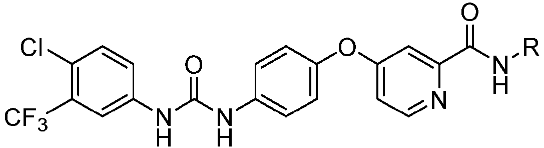 (derivatized where R is a linker group L or a -(L-CLM) group attached, for example, to the amide moiety);
**4**. The kinase inhibitor desatinib (derivatized): 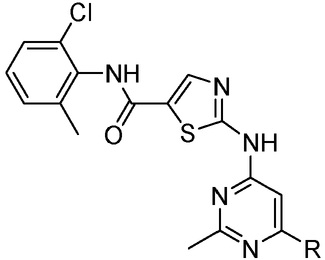 (derivatized where R is a linker group Lor a-(L-CLM) attached, for example, to the pyrimidine);
**5**. The kinase inhibitor lapatinib (derivatized): 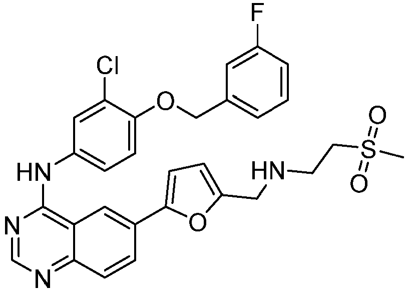 (derivatized where a linker group L or a-(L-CLM) group is attached, for example, via the terminal methyl of the sulfonyl methyl group);
**6**. The kinase inhibitor U09-CX-5279 (derivatized): 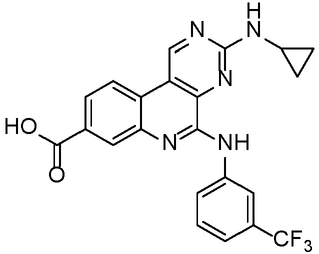 derivatized where a linker group L or a -(L-CLM) group is attached, for example, via the amine (aniline), carboxylic acid or amine alpha to cyclopropyl group, or cyclopropyl group;
**7**. The kinase inhibitors identified in Millan, et al., Design and Synthesis of Inhaled P38 Inhibitors for the Treatment of Chronic Obstructive Pulmonary Disease, J.MED.CHEM. vol:54, pag:7797 (2011), including the kinase inhibitors Y1W and Y1X (Derivatized) having the structures: 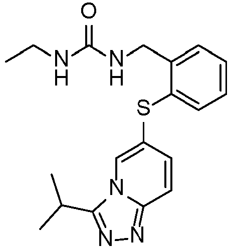 YIX (1-ethyl-3-(2-{[3-(1-methylethyl)[1,2,4]triazolo[4,3-a]pyridine-6-yl]sulfanyl}benzyl)urea
   derivatized where a linker group L or a-(L-CLM) group is attached, for example, via the ⁱpropyl group; 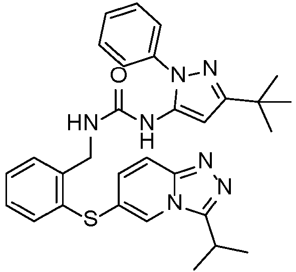 Y1W 1-(3-tert-butyl-1-phenyl-1H-pyrazol-5-yl)-3-(2-{[3-(1-methylethyl)[1,2,4] triazolo[4,3-a]pyridin-6-yl]sulfanyl }benzyl)urea derivatized where a linker group L or a -(L-CLM) group is attached, for example, preferably via either the i-propyl group or the t-butyl group;
**8**. The kinase inhibitors identified in Schenkel, et al., Discovery of Potent and Highly Selective Thienopyridine Janus Kinase 2 Inhibitors J. Med. Chem., 2011, 54 (24), pp 8440-8450, including the compounds 6TP and 0TP (Derivatized) having the structures: 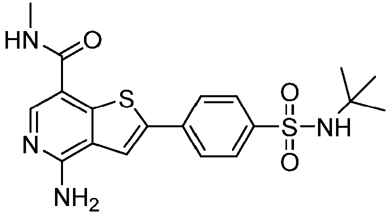 6TP 4-amino-2-[4-(tert-butylsulfamoyl)phenyl]-N-methylthieno[3,2-c]pyridine-7-carboxamide Thienopyridine 19
   derivatized where a linker group L or a -(L-CLM) group is attached, for example, via the terminal methyl group bound to amide moiety; 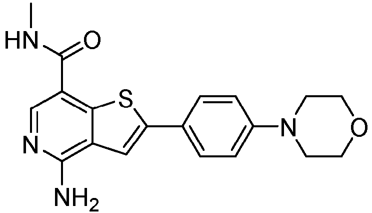 0TP 4-amino-N-methyl-2-[4-(morpholin-4-yl)phenyl]thieno[3,2-c]pyridine-7-carboxamide Thienopyridine 8
   derivatized where a linker group L or a -(L-CLM)group is attached, for example, via the terminal methyl group bound to the amide moiety;
**9**. The kinase inhibitors identified in Van Eis, et al., "2,6-Naphthyridines as potent and selective inhibitors of the novel protein kinase C isozymes", Biorg. Med. Chem. Lett.2011 Dec 15;21(24):7367-72, including the kinase inhibitor 07U having the structure: 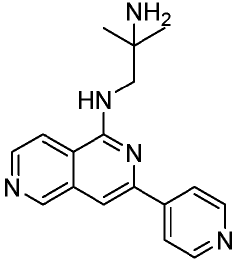 07U 2-methyl-N~1~[3-(pyridin-4-yl)-2,6-naphthyridin-1-yl]propane-1,2-diamine derivatized where a linker group L or a -(L-CLM)group is attached, for example, via the secondary amine or terminal amino group;
**10**. The kinase inhibitors identified in Lountos, et al., "Structural Characterization of Inhibitor Complexes with Checkpoint Kinase 2 (Chk2), a Drug Target for Cancer Therapy", J.STRUCT.BIOL. vol:176, pag:292 (2011), including the kinase inhibitor YCF having the structure: 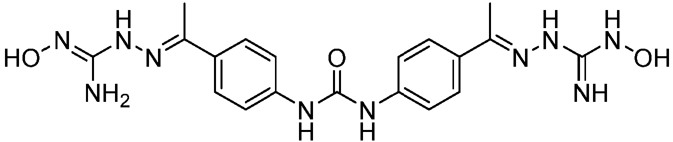 derivatized where a linker group L or a -(L-CLM) group is attached, for example, via either of the terminal hydroxyl groups;
**11**. The kinase inhibitors identified in Lountos, et al., "Structural Characterization of Inhibitor Complexes with Checkpoint Kinase 2 (Chk2), a Drug Target for Cancer Therapy", J.STRUCT.BIOL. vol:176, pag:292 (2011), including the kinase inhibitors XK9 and NXP (derivatized) having the structures: 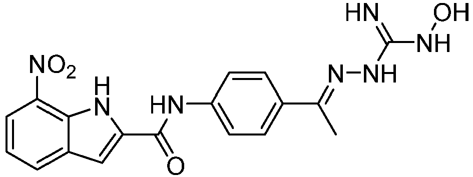 XK9 N-{4-[(1E)-N-(N-hydroxycarbamimidoyl)ethanehydrazonoyl]phenyl}-7-nitro-1H-indole-2-carboxamide; 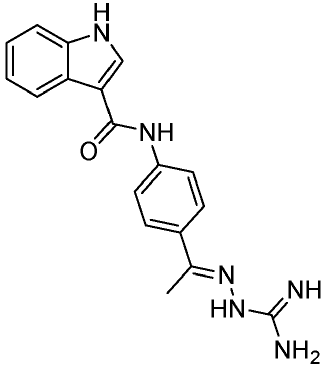 NXP N-{4-[(1E)-N-CARBAMIMIDOYLETHANEHYDRAZONOYL]PHENYL}-1H-INDOLE-3-CARBOXAMIDE derivatized where a linker group L or a -(L-CLM) group is attached, for example, via the terminal hydroxyl group (XK9) or the hydrazone group (NXP);
**12**. The kinase inhibitor afatinib (derivatized) (*N*-[4-[(3-chloro-4-fluorophenyl)amino]-7-[[(3*S*)-tetrahydro-3-furanyl]oxy]-6-quinazolinyl]-4(dimethylamino)-2-butenamide) (Derivatized where a linker group L or a -(L-CLM) group is attached, for example, via the aliphatic amine group);
**13**. The kinase inhibitor fostamatinib (derivatized) ([6-({5-fluoro-2-[(3,4,5-trimethoxyphenyl)amino]pyrimidin-4-yl}amino)-2,2-dimethyl-3-oxo-2,3-dihydro-4H-pyrido[3,2-b]-1,4-oxazin-4-yl]methyl disodium phosphate hexahydrate) (Derivatized where a linker group L or a -(L-CLM) group is attached, for example, via a methoxy group);
**14**. The kinase inhibitor gefitinib (derivatized) (*N*-(3-chloro-4-fluoro-phenyl)-7-methoxy-6-(3-morpholin-4-ylpropoxy)quinazolin-4-amine): 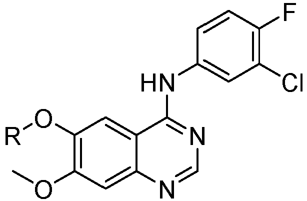 (derivatized where a linker group L or a -(L-CLM) group is attached, for example, via a methoxy or ether group);
**15**. The kinase inhibitor lenvatinib (derivatized) (4-[3-chloro-4-(cyclopropylcarbamoylamino)phenoxy]-7-methoxy-quinoline-6-carboxamide) (derivatized where a linker group L or a -(L-CLM) group is attached, for example, via the cyclopropyl group);
**16**. The kinase inhibitor vandetanib (derivatized) (*N*-(4-bromo-2-fluorophenyl)-6-methoxy-7-[(1-methylpiperidin-4-yl)methoxy]quinazolin-4-amine) (derivatized where a linker group L or a -(L-CLM) group is attached, for example, via the methoxy or hydroxyl group);
**17**. The kinase inhibitor vemurafenib (derivatized) (propane-1-sulfonic acid {3-[5-(4-chlorophenyl)-1H-pyrrolo[2,3-b]pyridine-3-carbonyl]-2,4-difluoro-phenyl}-amide) (derivatized where a linker group L or a -(L-CLM) group is attached, for example, via the sulfonyl propyl group);
**18**. The kinase inhibitor Gleevec (derivatized): 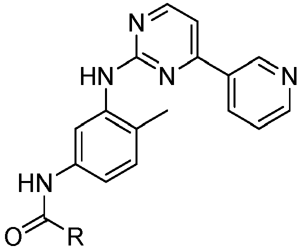 (derivatized where R as a linker group L or a-(L-CLM) group is attached, for example, via the amide group or via the aniline amine group);
**19**. The kinase inhibitor pazopanib (derivatized) (VEGFR3 inhibitor): 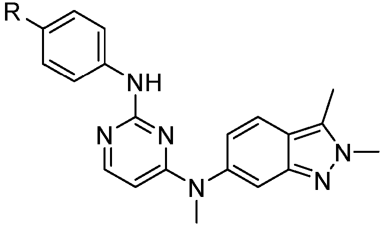 (derivatized where R is a linker group L or a -(L-CLM) group attached, for example, to the phenyl moiety or via the aniline amine group);
**20**. The kinase inhibitor AT-9283 (Derivatized) Aurora Kinase Inhibitor 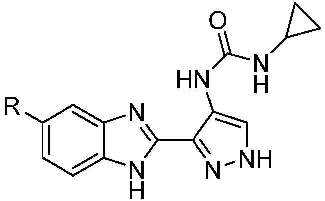 (where R is a linker group L or a -(L-CLM) group attached, for example, to the phenyl moiety);
**21**. The kinase inhibitor TAE684 (derivatized) ALK inhibitor 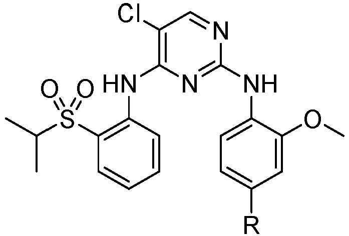 (where R is a linker group L or a -(L-CLM) group attached, for example, to the phenyl moiety);
**22**. The kinase inhibitor nilotanib (derivatized) Abl inhibitor: 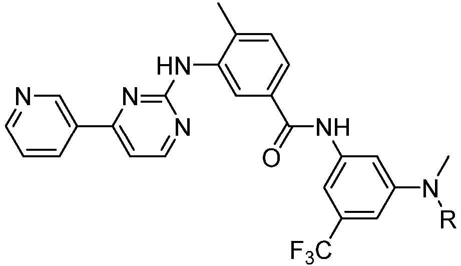 (derivatized where R is a linker group L or a -(L-CLM) group attached, for example, to the phenyl moiety or the aniline amine group);
**23**. Kinase Inhibitor NVP-BSK805 (derivatized) JAK2 Inhibitor 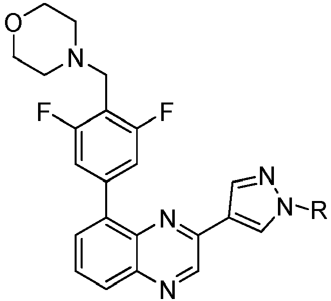 (derivatized where R is a linker group L or a -(L-CLM) group attached, for example, to the phenyl moiety or the diazole group);
**24**. Kinase Inhibitor crizotinib Derivatized Alk Inhibitor 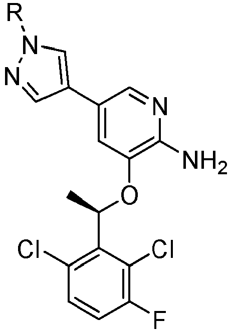 (derivatized where R is a linker group L or a -(L-CLM) group attached, for example, to the phenyl moiety or the diazole group);
**25**. Kinase Inhibitor JNJ FMS (derivatized) Inhibitor 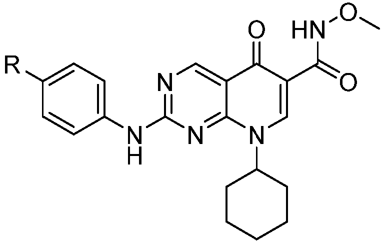 (derivatized where R is a linker group L or a -(L-CLM) group attached, for example, to the phenyl moiety);
**26**. The kinase inhibitor foretinib (derivatized) Met Inhibitor 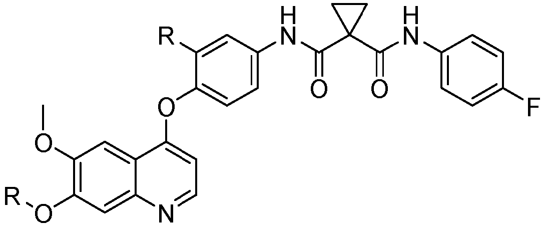 (derivatized where R is a linker group L or a -(L-CLM)group attached, for example, to the phenyl moiety or a hydroxyl or ether group on the quinoline moiety);
**27**. The allosteric Protein Tyrosine Phosphatase Inhibitor PTP1B (derivatized): 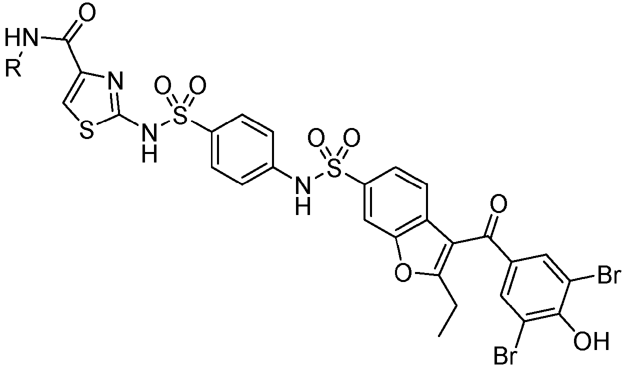 derivatized where a linker group L or a -(L-CLM) group is attached, for example, at R, as indicated;
**28**. The inhibitor of SHP-2 Domain of Tyrosine Phosphatase (derivatized): 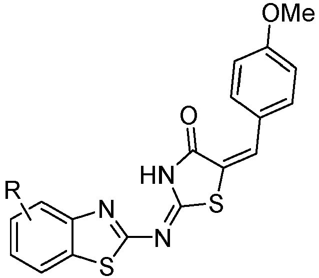 derivatized where a linker group L or a -(L-CLM) group is attached, for example, at R;
**29**. The inhibitor (derivatized) of BRAF (BRAF^{V600E})/MEK: 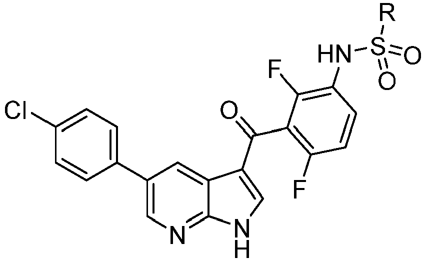 derivatized where a linker group L or a-(L-CLM) group is attached, for example, at R;
**30**. Inhibitor (derivatized) of Tyrosine Kinase ABL 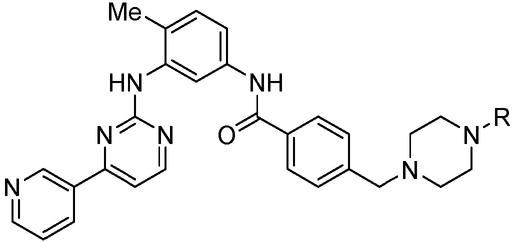 derivatized where a linker group L or a-(L-CLM) group is attached, for example, at R;
**31**. The kinase inhibitor OSI-027 (derivatized) mTORC1/2 inhibitor 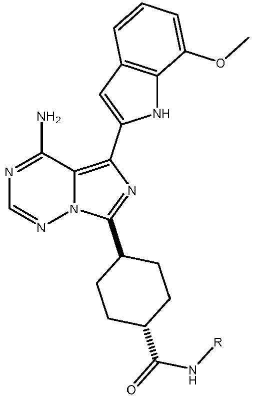 derivatized where a linker group L or a-(L-CLM) group is attached, for example, at R;
**32**. The kinase inhibitor OSI-930 (derivatized) c-Kit/KDR inhibitor 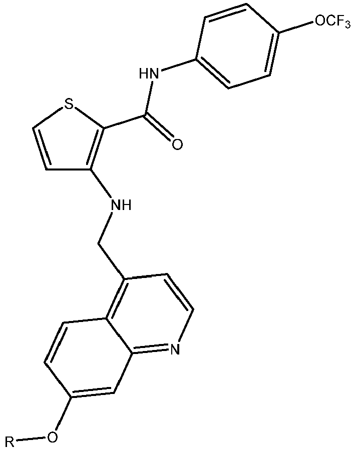 derivatized where a linker group L or a-(L-CLM) group is attached, for example, at R; and
**33**. The kinase inhibitor OSI-906 (derivatized) IGF1R/IR inhibitor 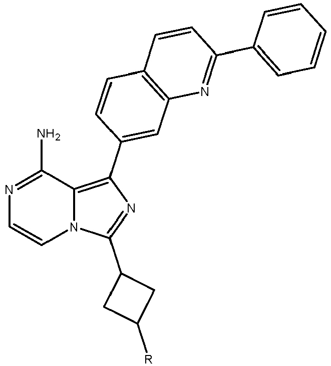 derivatized where a linker group L or a-(L-CLM) group is attached, for example, at R; (derivatized where "R" designates a site for attachment of a linker group L or a -(L-CLM)group on the piperazine moiety).

### III. HDM2/MDM2 Inhibitors (reference):

HDM2/MDM2 inhibitors as used herein include, but are not limited to:
**1**. The HDM2/MDM2 inhibitors identified in Vassilev, et al., In vivo activation of the p53 pathway by small-molecule antagonists of MDM2, SCIENCE vol:303, pag:844-848 (2004), and Schneekloth, et al., Targeted intracellular protein degradation induced by a small molecule: En route to chemical proteomics, Bioorg. Med. Chem. Lett. 18 (2008) 5904-5908, including (or additionally) the compounds nutlin-3, nutlin-2, and nutlin-1 (derivatized) as described below, as well as all derivatives and analogs thereof: 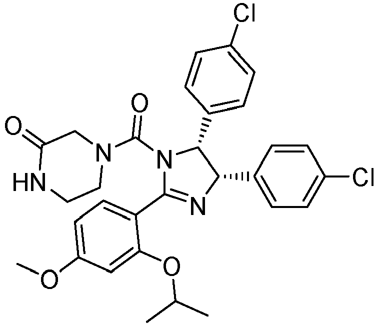 (derivatized where a linker group L or a -(L-CLM)group is attached, for example, at the methoxy group or as a hydroxyl group); 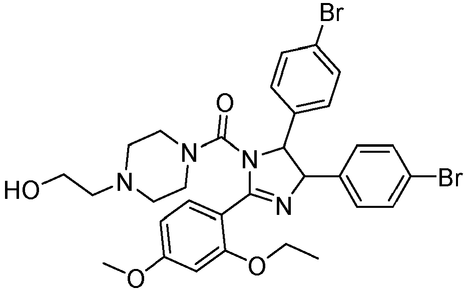 (derivatized where a linker group L or a -(L-CLM) group is attached, for example, at the methoxy group or hydroxyl group); 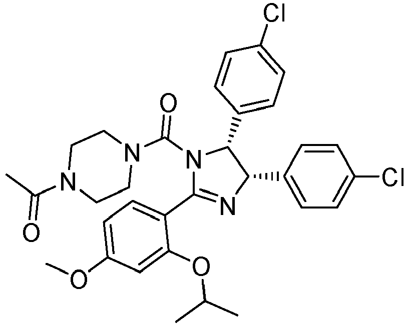 (derivatized where a linker group L or a -(L-CLM) group is attached, for example, via the methoxy group or as a hydroxyl group); and
**2.** Trans-4-Iodo-4'-Boranyl-Chalcone 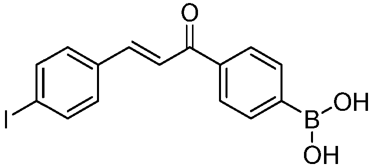 (derivatized where a linker group L or a a linker group L or a-(L-CLM) group is attached, for example, via a hydroxy group).

### IV. Compounds Targeting Human BET Bromodomain-containing proteins:

Compounds targeting Human BET Bromodomain-containing proteins include, but are not limited to the compounds associated with the targets as described below, where "R" designates a site for linker group L or a-(L-CLM) group attachment, for example:
**1.** JQ1, Filippakopoulos et al. Selective inhibition of BET bromodomains. Nature (2010*):* 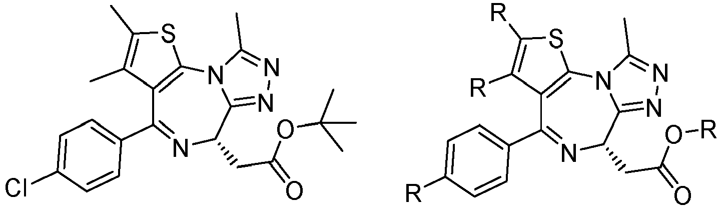
**2.** I-BET, Nicodeme et al. Supression of Inflammation by a Synthetic Histone Mimic. Nature (2010). Chung et al. Discovery and Characterization of Small Molecule Inhibitors of the BET Family Bromodomains. J. Med Chem. (2011): 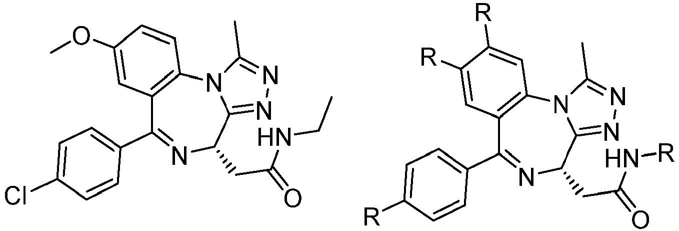
**3.** Compounds described in Hewings et al. 3,5-Dimethylisoxazoles Act as Acetyl-lysine Bromodomain Ligands. J. Med. Chem. (2011) 54 6761-6770. 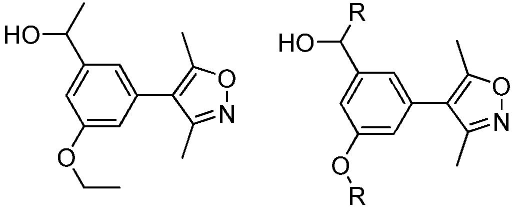
**4.** I-BET151, Dawson et al. Inhibition of BET Recruitment to Chromatin as an Efective Treatment for MLL-fusion Leukemia. Nature (2011): 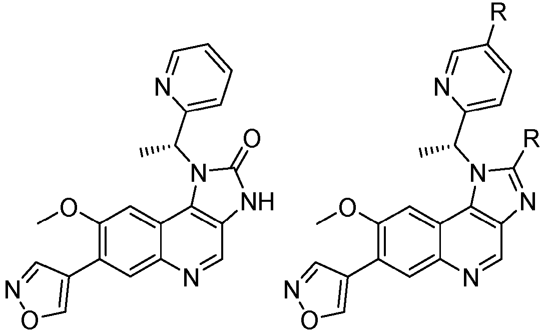
(Where R, in each instance, designates a site for attachment, for example, of a linker group L or a -(L-CLM) group).

### V. HDAC Inhibitors (reference):

HDAC Inhibitors (derivatized) include, but are not limited to:
**1.** Finnin, M. S. et al. Structures of Histone Deacetylase Homologue Bound to the TSA and SAHA Inhibitors. Nature 40, 188-193 (1999). 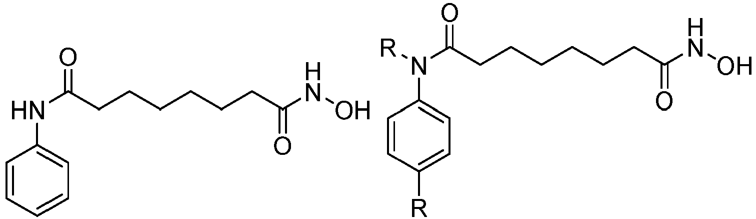 (Derivatized where "R" designates a site for attachment, for example, of a linker group L or a -(L-CLM) group); and
**2.** Compounds as defined by formula (I) of PCT WO0222577 ("DEACETYLASE INHIBITORS") (Derivatized where a linker group L or a - (L-CLM) group is attached, for example, via the hydroxyl group);

### VI. Human Lysine Methyltransferase Inhibitors (reference):

Human Lysine Methyltransferase inhibitors include, but are not limited to:
**1.** Chang et al. Structural Basis for G9a-Like protein Lysine Methyltransferase Inhibition by BIX-1294. Nat. Struct. Biol. (2009) 16(3) 312. 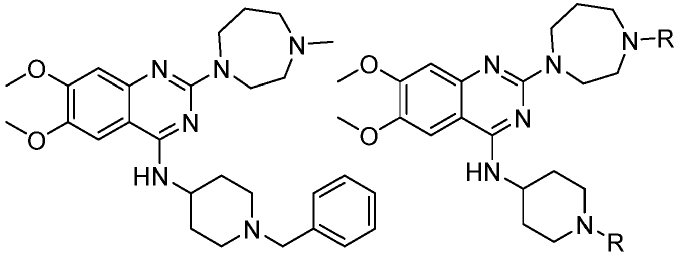 (Derivatized where "R" designates a site for attachment, for example, of a linker group L or a -(L-CLM) group);
**2.** Liu, F. et al Discovery of a 2,4-Diamino-7-aminoalkoxyquinazoline as a Potent and Selective Inhibitor of Histone Methyltransferase G9a. J. Med. Chem. (2009) 52(24) 7950. 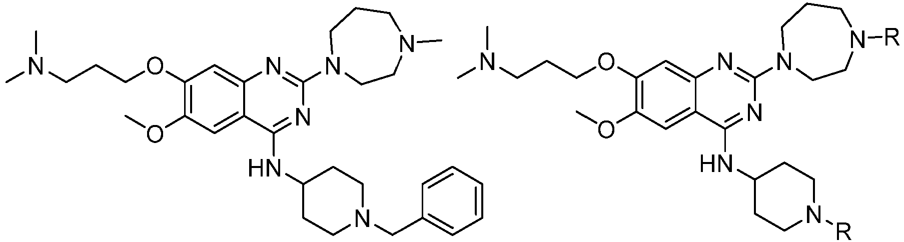 (Derivatized where "R" designates a potential site for attachment, for example, of a linker group L or a -(L-CLM) group);
**3.** Azacitidine (derivatized) (4-amino-1-β-D-ribofuranosyl-1,3,5-triazin-2(1H)-one) (Derivatized where a linker group L or a -(L-CLM) group is attached, for example, via the hydroxy or amino groups); and
**4.** Decitabine (derivatized) (4-amino-1-(2-deoxy-b-D-erythro-pentofuranosyl)-1, 3, 5-triazin-2(1*H*)-one) (Derivatized where a linker group L or a -(L-CLM) group is attached, for example, via either of the hydroxy groups or at the amino group).

### VII. Angiogenesis Inhibitors (reference):

Angiogenesis inhibitors include, but are not limited to:
**1.** GA-1 (derivatized) and derivatives and analogs thereof, having the structure(s) and binding to linkers as described in Sakamoto, et al., Development of Protacs to target cancer-promoting proteins for ubiquitination and degradation, Mol Cell Proteomics 2003 Dec;2(12):1350-8;
**2.** Estradiol (derivatized), which may be bound to a linker group L or a - (L-CLM) group as is generally described in Rodriguez-Gonzalez, et al., Targeting steroid hormone receptors for ubiquitination and degradation in breast and prostate cancer, Oncogene (2008) 27, 7201-7211;
**3.** Estradiol, testosterone (derivatized) and related derivatives, including but not limited to DHT and derivatives and analogs thereof, having the structure(s) and binding to a linker group L or a -(L-CLM) group as generally described in Sakamoto, et al., Development of Protacs to target cancer-promoting proteins for ubiquitination and degradation, Mol Cell Proteomics 2003 Dec; 2(12): 1350-8; and
**4.** Ovalicin, fumagillin (derivatized), and derivatives and analogs thereof, having the structure(s) and binding to a linker group L or a -(L-CLM) group as is generally described in Sakamoto, et al., Protacs: chimeric molecules that target proteins to the Skp1-Cullin-F box complex for ubiquitination and degradation Proc Natl Acad Sci USA. 2001 Jul 17;98(15):8554-9 and United States Patent No. 7,208,157.

### VIII. Immunosuppressive Compounds (reference):

Immunosuppressive compounds include, but are not limited to:
**1.** AP21998 (derivatized), having the structure(s) and binding to a linker group L or a -(L-CLM) group as is generally described in Schneekloth, et al., Chemical Genetic Control of Protein Levels: Selective in Vivo Targeted Degradation, J. AM. CHEM. SOC. 2004, 126, 3748-3754;
**2.** Glucocorticoids (e.g., hydrocortisone, prednisone, prednisolone, and methylprednisolone) (Derivatized where a linker group L or a -(L-CLM) group is to bound, e.g. to any of the hydroxyls) and beclometasone dipropionate (Derivatized where a linker group or a -(L-CLM) is bound, e.g. to a proprionate);
**3.** Methotrexate (Derivatized where a linker group or a -(L-CLM) group can be bound, e.g. to either of the terminal hydroxyls);
**4.** Ciclosporin (Derivatized where a linker group or a -(L-CLM) group can be bound, e.g. at any of the butyl groups);
**5.** Tacrolimus (FK-506) and rapamycin (Derivatized where a linker group L or a -(L-CLM) group can be bound, e.g. at one of the methoxy groups); and
**6.** Actinomycins (Derivatized where a linker group L or a -(L-CLM) group can be bound, e.g. at one of the isopropyl groups).

### IX. Compounds targeting the aryl hydrocarbon receptor (AHR) (reference):

Compounds targeting the aryl hydrocarbon receptor (AHR) include, but are not limited to:
**1.** Apigenin (Derivatized in a way which binds to a linker group L or a -(L-CLM) group as is generally illustrated in Lee, et al., Targeted Degradation of the Aryl Hydrocarbon Receptor by the PROTAC Approach: A Useful Chemical Genetic Tool, ChemBioChem Volume 8, Issue 17, pages 2058-2062, November 23, 2007); and
**2.** SR1 and LGC006 (derivatized such that a linker group L or a -(L-CLM) is bound), as described in Boitano, et al., Aryl Hydrocarbon Receptor Antagonists Promote the Expansion of Human Hematopoietic Stem Cells, Science 10 September 2010: Vol. 329 no. 5997 pp. 1345-1348.

### X. Compounds targeting RAF Receptor (Kinase) (reference):

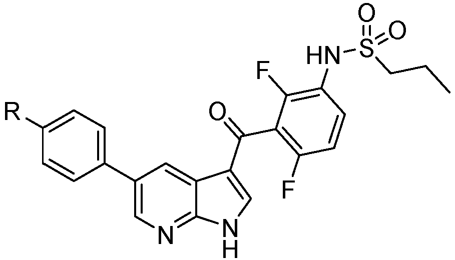

PLX4032
(Derivatized where "R" designates a site for linker group L or -(L-CLM) group attachment, for example).

### XI. Compounds Targeting FKBP (reference):

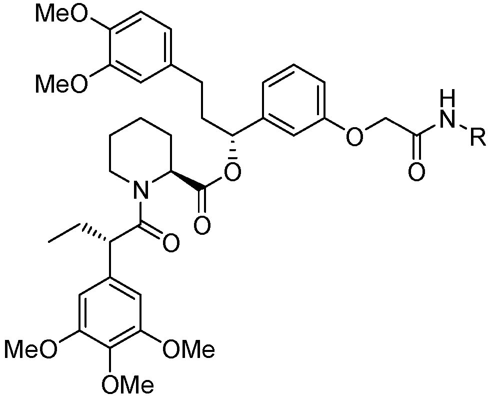

(Derivatized where "R" designates a site for a linker group L or a -(L-CLM) group attachment, for example).

### XII. Compounds Targeting Androgen Receptor (AR)

### 1. RU59063 Ligand (derivatized) of Androgen Rceptor

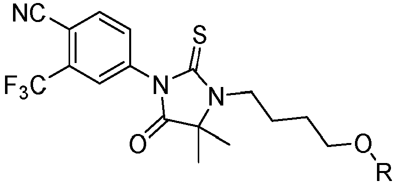

(Derivatized where "R" designates a site for a linker group L or a -(L-CLM) group attachment, for example).

### 2. SARM Ligand (derivatized) of Androgen Receptor

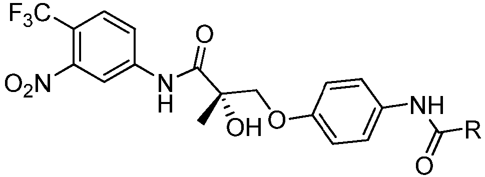

(Derivatized where "R" designates a site for a linker group L or a-(L-CLM) group attachment, for example).

### 3. Androgen Receptor Ligand DHT (derivatized)

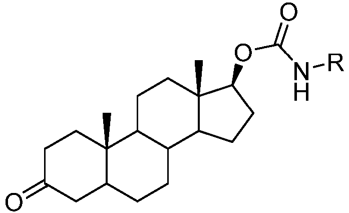

(Derivatized where "R" designates a site for a linker group L or -(L-CLM) group attachment, for example).

### 4. MDV3100 Ligand (derivatized)

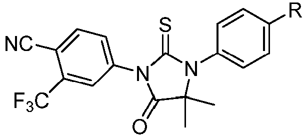

### 5. ARN-509 Ligand (derivatized)

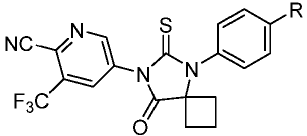

### 6. Hexahydrobenzisoxazoles

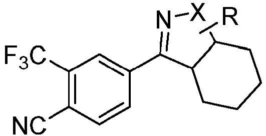

### 7. Tetramethylcyclobutanes

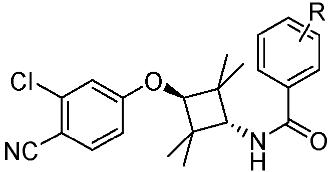

### XIII. Compounds Targeting Estrogen Receptor (ER) ICI-182780

### 1. Estrogen Receptor Ligand

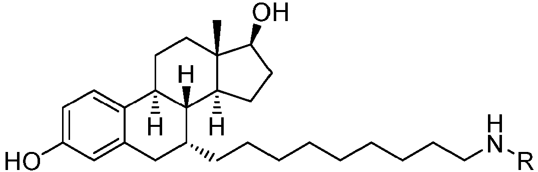

(Derivatized where "R" designates a site for linker group L or -(L-CLM) group attachment).

### XIV. Compounds Targeting Thyroid Hormone Receptor (TR) (reference)

### 1. Thyroid Hormone Receptor Ligand (derivatized)

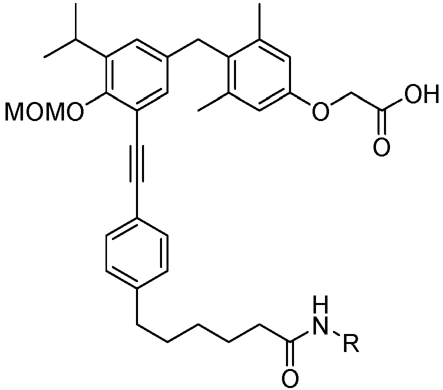

(Derivatized where "R" designates a site for linker group L or -(L-CLM) group attachment and MOMO indicates a methoxymethoxy group).

### XV. Compounds targeting HIV Protease (reference)

### 1. Inhibitor of HIV Protease (derivatized)

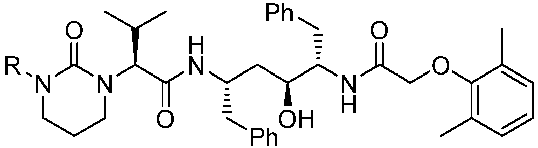

(Derivatized where "R" designates a site for linker group L or-(L-CLM) group attachment). See, J. Med. Chem. 2010, 53, 521-538.

### 2. Inhibitor of HIV Protease

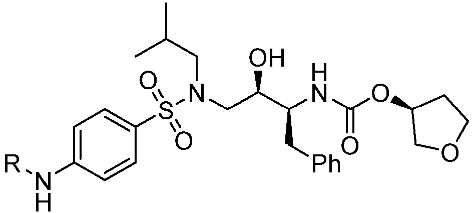

(Derivatized where "R" designates a potential site for linker group L or -(L-CLM) group attachment). See, J. Med. Chem. 2010, 53, 521-538.

### XVI. Compounds targeting HIV Integrase (reference)

### 1. Inhibitor of HIV Integrase (derivatized)

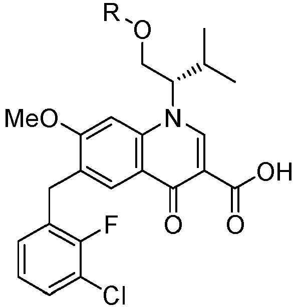

(Derivatized where "R" designates a site for linker group L or -(L-CLM) group attachment). See, J. Med. Chem. 2010, 53, 6466.

### 2. Inhibitor of HIV Integrase (derivatized)

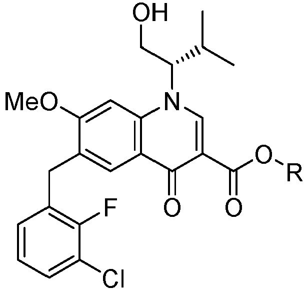

### 3. Inhibitor of HIV integrase Isetntress (derivatized)

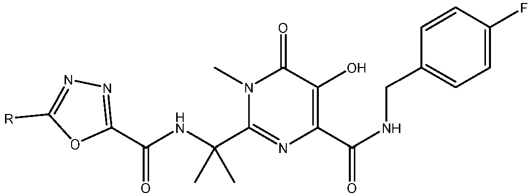

(Derivatized where "R" designates a site for linker group L or -(L-CLM) group attachment). See, J. Med. Chem. 2010, 53, 6466.

### XVII. Compounds targeting HCV Protease (reference)

### 1. Inhibitors of HCV Protease (derivatized)

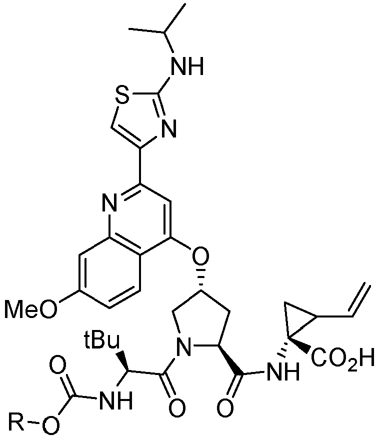

(Derivatized where "R" designates a site for linker group L or -(L-CLM) group attachment).

### XVIII. Compounds targeting Acyl-protein Thioesterase-1 and -2 (APT1 and APT2) (reference)

### 1. Inhibitor of APT1 and APT2 (derivatized)

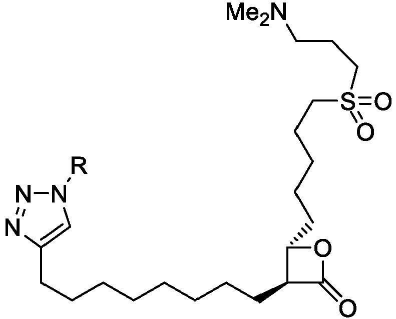

(Derivatized where "R" designates a site for linker group L or -(L-CLM) group attachment). See, Angew. Chem. Int. Ed. 2011, 50, 9838 -9842, where L is a linker group as otherwise described herein and said CLM group is as otherwise described herein such that -(L-CLM) binds the CLM group to a PTM group as otherwise described herein.

### Therapeutic Compositions

Pharmaceutical compositions comprising combinations of an effective amount of at least one bifunctional compound as described herein, and one or more of the compounds otherwise described herein, all in effective amounts, in combination with a pharmaceutically effective amount of a carrier, additive or excipient, represents a further aspect of the present disclosure.

The present disclosure includes, where applicable, the compositions comprising the pharmaceutically acceptable salts, in particular, acid or base addition salts of compounds as described herein. The acids which are used to prepare the pharmaceutically acceptable acid addition salts of the aforementioned base compounds useful according to this aspect are those which form non-toxic acid addition salts (i.e., salts containing pharmacologically acceptable anions), such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3 naphthoate)]salts, among numerous others.

Pharmaceutically acceptable base addition salts may also be used to produce pharmaceutically acceptable salt forms of the compounds or derivatives according to the present disclosure. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g., potassium and sodium) and alkaline earth metal cations (e.g, calcium, zinc and magnesium), ammonium or water-soluble amine addition salts such as N-methylglucamine-(meglumine), and the lower alkanolammonium and other base salts of pharmaceutically acceptable organic amines, among others.

The compounds as described herein may, in accordance with the disclosure, be administered in single or divided doses by the oral, parenteral or topical routes. Administration of the active compound may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal, sublingual and suppository administration, among other routes of administration. Enteric coated oral tablets may also be used to enhance bioavailability of the compounds from an oral route of administration. The most effective dosage form will depend upon the pharmacokinetics of the particular agent chosen as well as the severity of disease in the patient. Administration of compounds according to the present disclosure as sprays, mists, or aerosols for intra-nasal, intra-tracheal or pulmonary administration may also be used. The present disclosure therefore also is directed to pharmaceutical compositions comprising an effective amount of compound as described herein, optionally in combination with a pharmaceutically acceptable carrier, additive or excipient. Compounds according to the present disclosure may be administered in immediate release, intermediate release or sustained or controlled release forms. Sustained or controlled release forms are preferably administered orally, but also in suppository and transdermal or other topical forms. Intramuscular injections in liposomal form may also be used to control or sustain the release of compound at an injection site.

The compositions as described herein may be formulated in a conventional manner using one or more pharmaceutically acceptable carriers and may also be administered in controlled-release formulations. Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions as described herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions as described herein may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions as described herein may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions as described herein may be administered in the form of suppositories for rectal administration. These can be prepared by mixing the agent with a suitable non-irritating excipient, which is solid at room temperature but liquid at rectal temperature and therefore will melt in the rectum to release the drug. Such materials include cocoa butter, beeswax and polyethylene glycols.

The pharmaceutical compositions as described herein may also be administered topically. Suitable topical formulations are readily prepared for each of these areas or organs. Topical application for the lower intestinal tract can be effected in a rectal suppository formulation (see above) or in a suitable enema formulation. Topically-acceptable transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. In certain preferred aspects of the invention, the compounds may be coated onto a stent which is to be surgically implanted into a patient in order to inhibit or reduce the likelihood of occlusion occurring in the stent in the patient.

Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions as described herein may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of compound in a pharmaceutical composition as described herein that may be combined with the carrier materials to produce a single dosage form will vary depending upon the host and disease treated, the particular mode of administration. Preferably, the compositions should be formulated to contain between about 0.05 milligram to about 750 milligrams or more, more preferably about 1 milligram to about 600 milligrams, and even more preferably about 10 milligrams to about 500 milligrams of active ingredient, alone or in combination with at least one other compound according to the present invention.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or condition being treated.

A patient or subject in need of therapy using compounds according to the methods described herein can be treated by administering to the patient (subject) an effective amount of the compound according to the present invention including pharmaceutically acceptable salts, solvates or polymorphs, thereof optionally in a pharmaceutically acceptable carrier or diluent, either alone, or in combination with other known erythopoiesis stimulating agents as otherwise identified herein.

These compounds can be administered by any appropriate route, for example, orally, parenterally, intravenously, intradermally, subcutaneously, or topically, including transdermally, in liquid, cream, gel, or solid form, or by aerosol form.

The active compound is included in the pharmaceutically acceptable carrier or diluent in an amount sufficient to deliver to a patient a therapeutically effective amount for the desired indication, without causing serious toxic effects in the patient treated. A preferred dose of the active compound for all of the herein-mentioned conditions is in the range from about 10 ng/kg to 300 mg/kg, preferably 0.1 to 100 mg/kg per day, more generally 0.5 to about 25 mg per kilogram body weight of the recipient/patient per day. A typical topical dosage will range from 0.01-5% wt/wt in a suitable carrier.

The compound is conveniently administered in any suitable unit dosage form, including but not limited to one containing less than 1mg, 1 mg to 3000 mg, preferably 5 to 500 mg of active ingredient per unit dosage form. An oral dosage of about 25-250 mg is often convenient.

The active ingredient is preferably administered to achieve peak plasma concentrations of the active compound of about 0.00001-30 mM, preferably about 0.1-30 µM. This may be achieved, for example, by the intravenous injection of a solution or formulation of the active ingredient, optionally in saline, or an aqueous medium or administered as a bolus of the active ingredient. Oral administration is also appropriate to generate effective plasma concentrations of active agent.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

Oral compositions will generally include an inert diluent or an edible carrier. They may be enclosed in gelatin capsules or compressed into tablets. For the purpose of oral therapeutic administration, the active compound or its prodrug derivative can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or enteric agents.

The active compound or pharmaceutically acceptable salt thereof can be administered as a component of an elixir, suspension, syrup, wafer, chewing gum or the like. A syrup may contain, in addition to the active compounds, sucrose as a sweetening agent and certain preservatives, dyes and colorings and flavors.

The active compound or pharmaceutically acceptable salts thereof can also be mixed with other active materials that do not impair the desired action, or with materials that supplement the desired action, such as erythropoietin stimulating agents, including EPO and darbapoietin alfa, among others. In certain preferred aspects of the invention, one or more compounds according to the present invention are coadministered with another bioactive agent, such as an erythropoietin stimulating agent or a would healing agent, including an antibiotic, as otherwise described herein.

Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

For example, the active compounds may be prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions may also be pharmaceutically acceptable carriers. These may be prepared according to methods known to those skilled in the art, for example, as described in U.S. Pat. No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound are then introduced into the container. The container is then swirled by hand to free lipid material from the sides of the container and to disperse lipid aggregates, thereby forming the liposomal suspension.

### Therapeutic Methods

Also described are therapeutic compositions comprising an effective amount of a compound as described herein or salt form thereof, and a pharmaceutically acceptable carrier. The therapeutic compositions modulate protein degradation in a patient or subject, for example, an animal such as a human, and can be used for treating or ameliorating disease states or conditions which are modulated through the degraded protein.

The terms "treat", "treating", and "treatment", etc., as used herein, refer to any action providing a benefit to a patient for which the present compounds may be administered, including the treatment of any disease state or condition which is modulated through the protein to which the present compounds bind. Disease states or conditions, including cancer, which may be treated using compounds according to the present invention are set forth hereinabove.

Also described are therapeutic compositions as described herein for effectuating the degradation of proteins of interest for the treatment or amelioration of a disease (e.g., cancer). In certain examples, the disease is multiple myeloma. As such, in another aspect, the description provides bifunctional compound as described herein comprising, e.g., a CLM and a PTM, preferably linked through a linker moiety, as otherwise described herein, wherein the CLM is coupled to the PTM and wherein the CLM recognizes a ubiquitin pathway protein (e.g., an ubiquitin ligase, preferably an E3 ubiquitin ligase such as, e.g., cereblon) and the PTM recognizes the target protein such that degradation of the target protein will occur when the target protein is placed in proximity to the ubiquitin ligase, thus resulting in degradation/inhibition of the effects of the target protein and the control of protein levels, for use in a method of ubiquitinating/ degrading a target protein in a cell. The control of protein levels provides treatment of a disease state or condition, which is modulated through the target protein by lowering the level of that protein in the cell (e.g., cell of a patient). For example, the method may comprise administering an effective amount of a compound as described herein, optionally including a pharamaceutically acceptable excipient, carrier, adjuvant, another bioactive agent or combination thereof.

Also described are methods for treating or emeliorating a disease, disorder or symptom thereof in a subject or a patient (e.g., an animal such as a human), comprising administering to a subject in need thereof a composition comprising an effective amount (e.g., a therapeutically effective amount) of a compound as described herein or salt form thereof, and a pharmaceutically acceptable excipient, carrier, adjuvant, another bioactive agent or combination thereof, wherein the composition is effective for treating or ameliorating the disease or disorder or symptom thereof in the subject.

Also described are methods for identifying the effects of the degradation of proteins of interest in a biological system using compounds according to the present invention.

Also described is a method of treating a human patient in need for a disease state or condition modulated through a protein where the degradation of that protein will produce a therapeutic effect in that patient., the method comprising administering to a patient in need an effective amount of a compound as described herein, optionally in combination with another bioactive agent. The disease state or condition may be a disease caused by a microbial agent or other exogenous agent such as a virus, bacteria, fungus, protozoa or other microbe or may be a disease state, which is caused by overexpression of a protein, which leads to a disease state and/or condition.

The term "disease state or condition" is used to describe any disease state or condition wherein protein dysregulation (i.e., the amount of protein expressed in a patient is elevated) occurs and where degradation of one or more proteins in a patient may provide beneficial therapy or relief of symptoms to a patient in need thereof. In certain instances, the disease state or condition may be cured.

Disease states or conditions which may be treated using compounds of the present invention include cancer, namely large B-cell lymphoma, prostate cancer, ovarian cancer, breast cancer, multiple myeloma, uterine cancer, colon cancer or leukemia.

In general and for example, disease states of conditions which may be treated using compounds described herein include, for example, asthma, autoimmune diseases such as multiple sclerosis, various cancers, ciliopathies, cleft palate, diabetes, heart disease, hypertension, inflammatory bowel disease, mental retardation, mood disorder, obesity, refractive error, infertility, Angelman syndrome, Canavan disease, Coeliac disease, Charcot-Marie-Tooth disease, Cystic fibrosis, Duchenne muscular dystrophy, Haemochromatosis, Haemophilia, Klinefelter's syndrome, Neurofibromatosis, Phenylketonuria, Polycystic kidney disease, (PKD1) or 4 (PKD2) Prader-Willi syndrome, Sickle-cell disease, Tay-Sachs disease, Turner syndrome.

For example, further disease states or conditions which may be treated by compounds described herein include Alzheimer's disease, Amyotrophic lateral sclerosis (Lou Gehrig's disease), Anorexia nervosa, Anxiety disorder, Atherosclerosis, Attention deficit hyperactivity disorder, Autism, Bipolar disorder, Chronic fatigue syndrome, Chronic obstructive pulmonary disease, Crohn's disease, Coronary heart disease, Dementia, Depression, Diabetes mellitus type 1, Diabetes mellitus type 2, Epilepsy, Guillain-Barré syndrome, Irritable bowel syndrome, Lupus, Metabolic syndrome, Multiple sclerosis, Myocardial infarction, Obesity, Obsessive-compulsive disorder, Panic disorder, Parkinson's disease, Psoriasis, Rheumatoid arthritis, Sarcoidosis, Schizophrenia, Stroke, Thromboangiitis obliterans, Tourette syndrome, Vasculitis.

For example, still additional disease states or conditions which can be treated by compounds described herein include aceruloplasminemia, Achondrogenesis type II, achondroplasia, Acrocephaly, Gaucher disease type 2, acute intermittent porphyria, Canavan disease, Adenomatous Polyposis Coli, ALA dehydratase deficiency, adenylosuccinate lyase deficiency, Adrenogenital syndrome, Adrenoleukodystrophy, ALA-D porphyria, ALA dehydratase deficiency, Alkaptonuria, Alexander disease, Alkaptonuric ochronosis, alpha 1-antitrypsin deficiency, alpha-1 proteinase inhibitor, emphysema, amyotrophic lateral sclerosis Alström syndrome, Alexander disease, Amelogenesis imperfecta, ALA dehydratase deficiency, Anderson-Fabry disease, androgen insensitivity syndrome, Anemia Angiokeratoma Corporis Diffusum, Angiomatosis retinae (von Hippel-Lindau disease) Apert syndrome, Arachnodactyly (Marfan syndrome), Stickler syndrome, Arthrochalasis multiplex congenital (Ehlers-Danlos syndrome arthrochalasia type) ataxia telangiectasia, Rett syndrome, primary pulmonary hypertension, Sandhoff disease, neurofibromatosis type II, Beare-Stevenson cutis gyrata syndrome, Mediterranean fever, familial, Benjamin syndrome, beta-thalassemia, Bilateral Acoustic Neurofibromatosis (neurofibromatosis type II), factor V Leiden thrombophilia, Bloch-Sulzberger syndrome (incontinentia pigmenti), Bloom syndrome, X-linked sideroblastic anemia, Bonnevie-Ullrich syndrome (Turner syndrome), Bourneville disease (tuberous sclerosis), prion disease, Birt-Hogg-Dubé syndrome, Brittle bone disease (osteogenesis imperfecta), Broad Thumb-Hallux syndrome (Rubinstein-Taybi syndrome), Bronze Diabetes/Bronzed Cirrhosis (hemochromatosis), Bulbospinal muscular atrophy (Kennedy's disease), Burger-Grutz syndrome (lipoprotein lipase deficiency), CGD Chronic granulomatous disorder, Campomelic dysplasia, biotinidase deficiency, Cardiomyopathy (Noonan syndrome), Cri du chat, CAVD (congenital absence of the vas deferens), Caylor cardiofacial syndrome (CBAVD), CEP (congenital erythropoietic porphyria), cystic fibrosis, congenital hypothyroidism, Chondrodystrophy syndrome (achondroplasia), otospondylomegaepiphyseal dysplasia, Lesch-Nyhan syndrome, galactosemia, Ehlers-Danlos syndrome, Thanatophoric dysplasia, Coffin-Lowry syndrome, Cockayne syndrome, (familial adenomatous polyposis), Congenital erythropoietic porphyria, Congenital heart disease, Methemoglobinemia/Congenital methaemoglobinaemia, achondroplasia, X-linked sideroblastic anemia, Connective tissue disease, Conotruncal anomaly face syndrome, Cooley's Anemia (beta-thalassemia), Copper storage disease (Wilson's disease), Copper transport disease (Menkes disease), hereditary coproporphyria, Cowden syndrome, Craniofacial dysarthrosis (Crouzon syndrome), Creutzfeldt-Jakob disease (prion disease), Cockayne syndrome, Cowden syndrome, Curschmann-Batten-Steinert syndrome (myotonic dystrophy), Beare-Stevenson cutis gyrata syndrome, primary hyperoxaluria, spondyloepimetaphyseal dysplasia (Strudwick type), muscular dystrophy, Duchenne and Becker types (DBMD), Usher syndrome, Degenerative nerve diseases including de Grouchy syndrome and Dejerine-Sottas syndrome, developmental disabilities, distal spinal muscular atrophy, type V, androgen insensitivity syndrome, Diffuse Globoid Body Sclerosis (Krabbe disease), Di George's syndrome, Dihydrotestosterone receptor deficiency, androgen insensitivity syndrome, Down syndrome, Dwarfism, erythropoietic protoporphyria Erythroid 5-aminolevulinate synthetase deficiency, Erythropoietic porphyria, erythropoietic protoporphyria, erythropoietic uroporphyria, Friedreich's ataxia,, familial paroxysmal polyserositis, porphyria cutanea tarda, familial pressure sensitive neuropathy, primary pulmonary hypertension (PPH), Fibrocystic disease of the pancreas, fragile X syndrome, galactosemia, genetic brain disorders, Giant cell hepatitis (Neonatal hemochromatosis), Gronblad-Strandberg syndrome (pseudoxanthoma elasticum), Gunther disease (congenital erythropoietic porphyria), haemochromatosis, Hallgren syndrome, sickle cell anemia, hemophilia, hepatoerythropoietic porphyria (HEP), Hippel-Lindau disease (von Hippel-Lindau disease), Huntington's disease, Hutchinson-Gilford progeria syndrome (progeria), Hyperandrogenism, Hypochondroplasia, Hypochromic anemia, Immune system disorders, including X-linked severe combined immunodeficiency, Insley-Astley syndrome, Jackson-Weiss syndrome, Joubert syndrome, Lesch-Nyhan syndrome, Jackson-Weiss syndrome, Kidney diseases, including hyperoxaluria, Klinefelter's syndrome, Kniest dysplasia, Lacunar dementia,Langer-Saldino achondrogenesis, ataxia telangiectasia, Lynch syndrome, Lysyl-hydroxylase deficiency, Machado-Joseph disease, Metabolic disorders, including Kniest dysplasia, Marfan syndrome, Movement disorders, Mowat-Wilson syndrome, cystic fibrosis, Muenke syndrome, Multiple neurofibromatosis, Nance-Insley syndrome, Nance-Sweeney chondrodysplasia, Niemann-Pick disease, Noack syndrome (Pfeiffer syndrome), Osler-Weber-Rendu disease, Peutz-Jeghers syndrome, Polycystic kidney disease, polyostotic fibrous dysplasia (McCune-Albright syndrome), Peutz-Jeghers syndrome, Prader-Labhart-Willi syndrome, hemochromatosis, primary hyperuricemia syndrome (Lesch-Nyhan syndrome), primary pulmonary hypertension, primary senile degenerative dementia, prion disease, progeria (Hutchinson Gilford Progeria Syndrome), progressive chorea, chronic hereditary (Huntington) (Huntington's disease), progressive muscular atrophy, spinal muscular atrophy, propionic acidemia, protoporphyria, proximal myotonic dystrophy, pulmonary arterial hypertension, PXE (pseudoxanthoma elasticum), Rb (retinoblastoma), Recklinghausen disease (neurofibromatosis type I), Recurrent polyserositis, Retinal disorders, Retinoblastoma, Rett syndrome, RFALS type 3, Ricker syndrome, Riley-Day syndrome, Roussy-Levy syndrome, severe achondroplasia with developmental delay and acanthosis nigricans (SADDAN), Li-Fraumeni syndrome, sarcoma, breast, leukemia, and adrenal gland (SBLA) syndrome, sclerosis tuberose (tuberous sclerosis), SDAT, SED congenital (spondyloepiphyseal dysplasia congenita), SED Strudwick (spondyloepimetaphyseal dysplasia, Strudwick type), SEDc (spondyloepiphyseal dysplasia congenita) SEMD, Strudwick type (spondyloepimetaphyseal dysplasia, Strudwick type), Shprintzen syndrome, Skin pigmentation disorders, Smith-Lemli-Opitz syndrome, South-African genetic porphyria (variegate porphyria), infantile-onset ascending hereditary spastic paralysis, Speech and communication disorders, sphingolipidosis, Tay-Sachs disease, spinocerebellar ataxia, Stickler syndrome, stroke, androgen insensitivity syndrome, tetrahydrobiopterin deficiency, beta-thalassemia, Thyroid disease, Tomaculous neuropathy (hereditary neuropathy with liability to pressure palsies), Treacher Collins syndrome, Triplo X syndrome ( triple X syndrome), Trisomy 21 (Down syndrome), Trisomy X, VHL syndrome (von Hippel-Lindau disease), Vision impairment and blindness (Alström syndrome), Vrolik disease, Waardenburg syndrome, Warburg Sjo Fledelius Syndrome, Weissenbacher-Zweymüller syndrome, Wolf-Hirschhorn syndrome, Wolff Periodic disease, Weissenbacher-Zweymüller syndrome and Xeroderma pigmentosum, among others.

The term "neoplasia" or "cancer" is used throughout the specification to refer to the pathological process that results in the formation and growth of a cancerous or malignant neoplasm, i.e., abnormal tissue that grows by cellular proliferation, often more rapidly than normal and continues to grow after the stimuli that initiated the new growth cease. Malignant neoplasms show partial or complete lack of structural organization and functional coordination with the normal tissue and most invade surrounding tissues, metastasize to several sites, and are likely to recur after attempted removal and to cause the death of the patient unless adequately treated. As used herein, the term neoplasia is used to describe all cancerous disease states and embraces or encompasses the pathological process associated with malignant hematogenous, ascitic and solid tumors. For example, exemplary cancers which may be treated by compounds described herein, either alone or in combination with at least one additional anti-cancer agent include squamous-cell carcinoma, basal cell carcinoma, adenocarcinoma, hepatocellular carcinomas, and renal cell carcinomas, cancer of the bladder, bowel, breast, cervix, colon, esophagus, head, kidney, liver, lung, neck, ovary, pancreas, prostate, and stomach; leukemias; benign and malignant lymphomas, particularly Burkitt's lymphoma and Non-Hodgkin's lymphoma; benign and malignant melanomas; myeloproliferative diseases; sarcomas, including Ewing's sarcoma, hemangiosarcoma, Kaposi's sarcoma, liposarcoma, myosarcomas, peripheral neuroepithelioma, synovial sarcoma, gliomas, astrocytomas, oligodendrogliomas, ependymomas, gliobastomas, neuroblastomas, ganglioneuromas, gangliogliomas, medulloblastomas, pineal cell tumors, meningiomas, meningeal sarcomas, neurofibromas, and Schwannomas; bowel cancer, breast cancer, prostate cancer, cervical cancer, uterine cancer, lung cancer, ovarian cancer, testicular cancer, thyroid cancer, astrocytoma, esophageal cancer, pancreatic cancer, stomach cancer, liver cancer, colon cancer, melanoma; carcinosarcoma, Hodgkin's disease, Wilms' tumor and teratocarcinomas. Additional cancers which may be treated using compounds described herein include, for example, T-lineage Acute lymphoblastic Leukemia (T-ALL), T-lineage lymphoblastic Lymphoma (T-LL), Peripheral T-cell lymphoma, Adult T-cell Leukemia, Pre-B ALL, Pre-B Lymphomas, Large B-cell Lymphoma, Burkitts Lymphoma, B-cell ALL, Philadelphia chromosome positive ALL and Philadelphia chromosome positive CML.

The term "bioactive agent" is used to describe an agent, other than a compound according to the present invention, which is used in combination with the present compounds as an agent with biological activity to assist in effecting an intended therapy, inhibition and/or prevention/prophylaxis for which the present compounds are used. Preferred bioactive agents for use herein include those agents which have pharmacological activity similar to that for which the present compounds are used or administered and include for example, anti-cancer agents, antiviral agents, especially including anti-HIV agents. and anti-HCV agents.

The term "additional anti-cancer agent" is used to describe an anti-cancer agent, which may be combined with compounds according to the present invention to treat cancer. These agents are selected from the group consisting of FLT-3 inhibitor, a VEGFR inhibitor, an EGFR TK inhibitor, an aurora kinase inhibitor, a PIK-1 modulator, a Bcl-2 inhibitor, an HDAC inhbitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, an mTORC1/2 inhibitor, a JAK/STAT inhibitor, a checkpoint-1 or 2 inhibitor, a focal adhesion kinase inhibitor, a Map kinase kinase (mek) inhibitor, a VEGF trap antibody, and mixtures thereof. In general and for example, further possible anticancer agents include everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR₁ KRX-0402, lucanthone, LY317615, neuradiab, vitespan, Rta 744, Sdx 102, talampanel, atrasentan, Xr 311, romidepsin, ADS-100380, sunitinib, 5-fluorouracil, vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N-[4-[2-(2-amino-4,7-dihydro-4-oxo-1H- pyrrolo[2,3-d]pyrimidin-5-yl)ethyl]benzoyl]-, disodium salt, heptahydrate, camptothecin, PEG-labeled irinotecan, tamoxifen, toremifene citrate, anastrazole, exemestane, letrozole, DES(diethylstilbestrol), estradiol, estrogen, conjugated estrogen, bevacizumab, IMC-1C11, CHIR-258); 3-[5-(methylsulfonylpiperadinemethyl)- indolyl-quinolone, vatalanib, AG-013736, AVE-0005, goserelin acetate, leuprolide acetate, triptorelin pamoate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, Ionafarnib, BMS-214662, tipifarnib; amifostine, NVP-LAQ824, suberoyl analide hydroxamic acid, valproic acid, trichostatin A, FK-228, SU11248, sorafenib, KRN951 , aminoglutethimide, arnsacrine, anagrelide, L-asparaginase, Bacillus Calmette-Guerin (BCG) vaccine, adriamycin, bleomycin, buserelin, busulfan, carboplatin, carmustine, chlorambucil, cisplatin, cladribine, clodronate, cyproterone, cytarabine, dacarbazine, dactinomycin, daunorubicin, diethylstilbestrol, epirubicin, fludarabine, fludrocortisone, fluoxymesterone, flutamide, gleevec, gemcitabine, hydroxyurea, idarubicin, ifosfamide, imatinib, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291 , squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox,gefitinib, bortezimib, paclitaxel, cremophor-free paclitaxel, docetaxel, epithilone B, BMS- 247550, BMS-310705, droloxifene, 4-hydroxytamoxifen, pipendoxifene, ERA-923, arzoxifene, fulvestrant, acolbifene, lasofoxifene, idoxifene, TSE-424, HMR- 3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonist, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa, darbepoetin alfa and mixtures thereof.

The term "anti-HIV agent" or "additional anti-HIV agent" includes, for example, nucleoside reverse transcriptase inhibitors (NRTI), other non-nucloeoside reverse transcriptase inhibitors (i.e., those which are not representative of the present invention), protease inhibitors, fusion inhibitors, among others, exemplary compounds of which may include, for example, 3TC (Lamivudine), AZT (Zidovudine), (-)-FTC, ddI (Didanosine), ddC (zalcitabine), abacavir (ABC), tenofovir (PMPA), D-D4FC (Reverset), D4T (Stavudine), Racivir, L-FddC, L-FD4C, NVP (Nevirapine), DLV (Delavirdine), EFV (Efavirenz), SQVM (Saquinavir mesylate), RTV (Ritonavir), IDV (Indinavir), SQV (Saquinavir), NFV (Nelfinavir), APV (Amprenavir), LPV (Lopinavir), fusion inhibitors such as T20, among others, fuseon and mixtures thereof, including anti-HIV compounds presently in clinical trials or in development.

Other anti-HIV agents which may be used in coadministration with compounds according to the present invention include, for example, other NNRTI's (i.e., other than the NNRTI's according to the present invention) may be selected from the group consisting of nevirapine (BI-R6-587), delavirdine (U-90152S/T), efavirenz (DMP-266), UC-781 (N-[4-chloro-3-(3-methyl-2-butenyloxy)phenyl]-2methyl3-furancarbothiamide), etravirine (TMC125), Trovirdine (Ly300046.HCl), MKC-442 (ernivirine, coactinon), HI-236, HI-240, HI-280, HI-281, rilpivirine (TMC-278), MSC-127, HBY 097, DMP266, Baicalin (TJN-151) ADAM-II (Methyl 3',3'-dichloro-4',4"-dimethoxy-5',5"-bis(methoxycarbonyl)-6,6-diphenylhexenoate), Methyl 3-Bromo-5-(1-5-bromo-4-methoxy-3-(methoxycarbonyl)phenyl)hept-1-enyl)-2-methoxybenzoate (Alkenyldiarylmethane analog, Adam analog), (5-chloro-3-(phenylsulfinyl)-2'-indolecarboxamide), AAP-BHAP (U-104489 or PNU-104489), Capravirine (AG-1549, S-1153), atevirdine (U-87201E), aurin tricarboxylic acid (SD-095345), 1-[(6-cyano-2-indolyl)carbonyl]-4-[3-(isopropylamino)-2-pyridinyl]piperazine, 1-[5-[[N-(methyl)methylsulfonylamino]-2-indolylcarbonyl-4-[3-(isopropylamino)-2-pyridinyl]piperazine, 1-[3-(Ethylamino)-2-[pyridinyl]-4-[(5-hydroxy-2-indolyl)carbonyl]piperazine, 1-[(6-Formyl-2-indolyl)carbonyl]-4-[3-(isopropylamino)-2-pyridinyl]piperazine, 1-[[5-(Methylsulfonyloxy)-2-indoyly)carbonyl]-4-[3-(isopropylamino)-2-pyridinyl]piperazine, U88204E, Bis(2-nitrophenyl)sulfone (NSC 633001), Calanolide A (NSC675451), Calanolide B, 6-Benzyl-5-methyl-2-(cyclohexyloxy)pyrimidin-4-one (DABO-546), DPC 961, E-EBU, E-EBU-dm, E-EPSeU, E-EPU, Foscarnet (Foscavir), HEPT (1-[(2-Hydroxyethoxy)methyl]-6-(phenylthio)thymine), HEPT-M (1-[(2-Hydroxyethoxy)methyl]-6-(3-methylphenyl)thio)thymine), HEPT-S (1-[(2-Hydroxyethoxy)methyl]-6-(phenylthio)-2-thiothymine), Inophyllum P, L-737,126, Michellamine A (NSC650898), Michellamine B (NSC649324), Michellamine F, 6-(3,5-Dimethylbenzyl)-1-[(2-hydroxyethoxy)methyl]-5-isopropyluracil, 6-(3,5-Dimethylbenzyl)-1-(ethyoxymethyl)-5-isopropyluracil, NPPS, E-BPTU (NSC 648400), Oltipraz (4-Methyl-5-(pyrazinyl)-3H-1,2-dithiole-3-thione), N-{2-(2-Chloro-6-fluorophenethyl]-N'-(2-thiazolyl)thiourea (PETT Cl, F derivative), N-{2-(2,6-Difluorophenethyl]-N'-[2-(5-bromopyridyl)]thiourea {PETT derivative), N-{2-(2,6-Difluorophenethyl]-N'-[2-(5-methylpyridyl)]thiourea {PETT Pyridyl derivative), N-[2-(3-Fluorofuranyl)ethyl]-N'-[2-(5-chloropyridyl)]thiourea, N-[2-(2-Fluoro-6-ethoxyphenethyl)]-N'-[2-(5-bromopyridyl)]thiourea, N-(2-Phenethyl)-N'-(2-thiazolyl)thiourea (LY-73497), L-697,639, L-697,593, L-697,661, 3-[2-(4,7-Difluorobenzoxazol-2-yl)ethyl}-5-ethyl-6-methyl(pypridin-2(1H)-thione (2-Pyridinone Derivative), 3-[[(2-Methoxy-5,6-dimethyl-3-pyridyl)methyl]amine]-5-ethyl-6-methyl(pypridin-2(1H)-thione, R82150, R82913, R87232, R88703, R89439 (Loviride), R90385, S-2720, Suramin Sodium, TBZ (Thiazolobenzimidazole, NSC 625487), Thiazoloisoindol-5-one, (+)(R)-9b-(3,5-Dimethylphenyl-2,3-dihydrothiazolo[2,3-a]isoindol-5(9bH)-one, Tivirapine (R86183), UC-38 and UC-84, among others.

The term "pharmaceutically acceptable salt" is used throughout the specification to describe, where applicable, a salt form of one or more of the compounds described herein which are presented to increase the solubility of the compound in the gastic juices of the patient's gastrointestinal tract in order to promote dissolution and the bioavailability of the compounds. Pharmaceutically acceptable salts include those derived from pharmaceutically acceptable inorganic or organic bases and acids, where applicable. Suitable salts include those derived from alkali metals such as potassium and sodium, alkaline earth metals such as calcium, magnesium and ammonium salts, among numerous other acids and bases well known in the pharmaceutical art. Sodium and potassium salts are particularly preferred as neutralization salts of the phosphates according to the present invention.

The term "pharmaceutically acceptable derivative" is used throughout the specification to describe any pharmaceutically acceptable prodrug form (such as an ester, amide other prodrug group), which, upon administration to a patient, provides directly or indirectly the present compound or an active metabolite of the present compound.

### General Synthetic Approach

The synthetic realization and optimization of the bifunctional molecules as described herein may be approached in a step-wise or modular fashion. For example, identification of compounds that bind to the target molecules can involve high or medium throughput screening campaigns if no suitable ligands are immediately available. It is not unusual for initial ligands to require iterative design and optimization cycles to improve suboptimal aspects as identified by data from suitable in vitro and pharmacological and/or ADMET assays. Part of the optimization/SAR campaign would be to probe positions of the ligand that are tolerant of substitution and that might be suitable places on which to attach the linker chemistry previously referred to herein. Where crystallographic or NMR structural data are available, these can be used to focus such a synthetic effort.

In a very analogous way one can identify and optimize ligands for an E3 Ligase (i.e. ULMs/CLMs).

For example, with PTMs and ULMs (e.g. CLMs) in hand one skilled in the art can use known synthetic methods for their combination with a linker moiety. Linker moieties can be synthesized with a range of compositions, lengths and flexibility and functionalized such that the PTM and ULM groups can be attached sequentially to distal ends of the linker. As with the PTM and ULM groups, the final bifunctional molecules can be subject to iterative design and optimization cycles in order to identify molecules with desirable properties.

Some non-limiting exemplary methods to generate the CLMs as described herein are summarized as shown below.

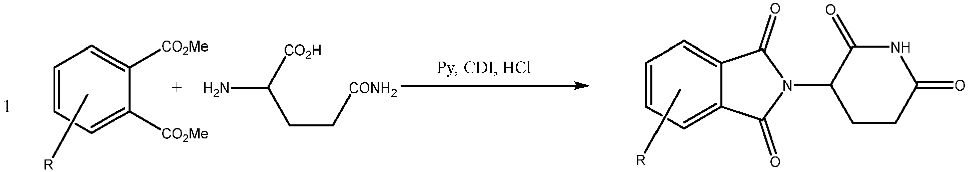

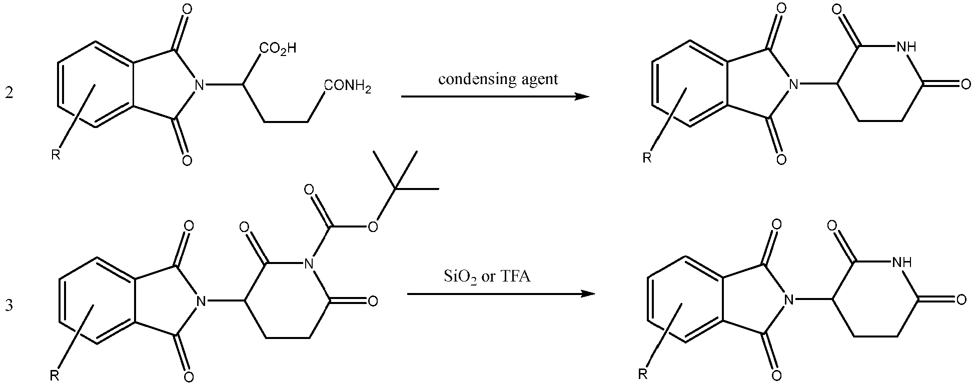

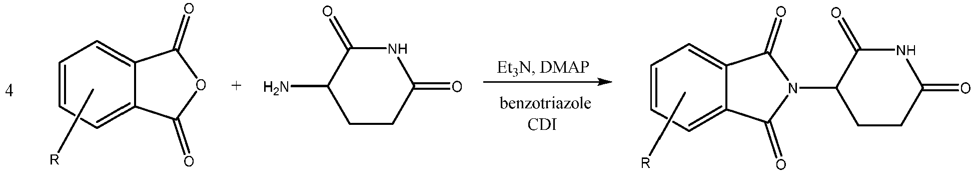

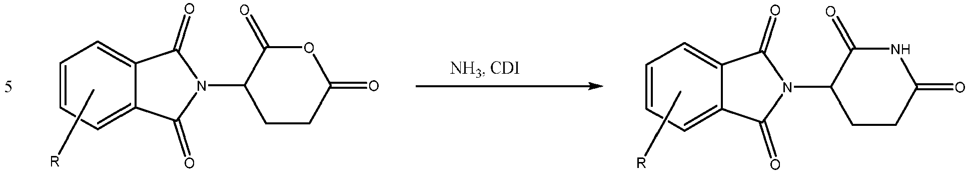

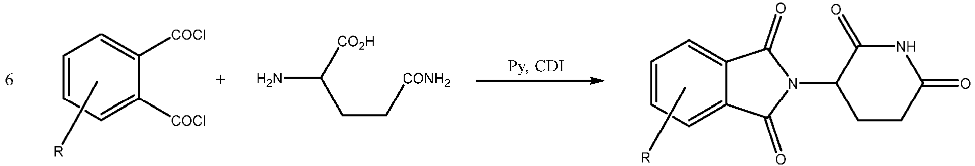

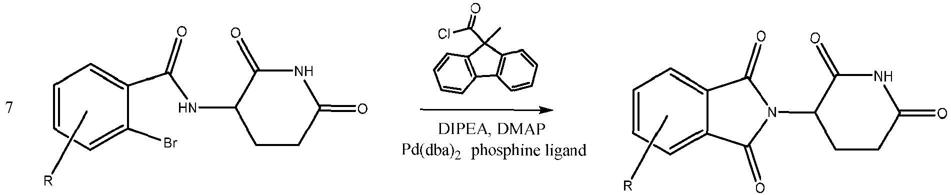

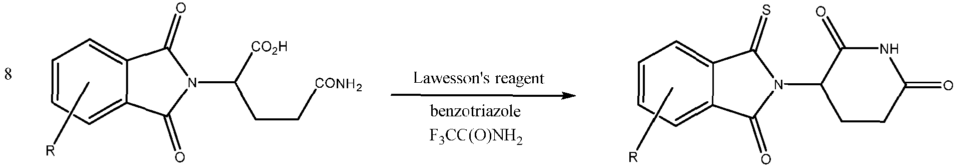

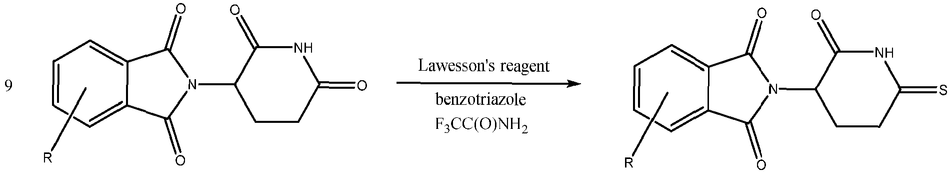

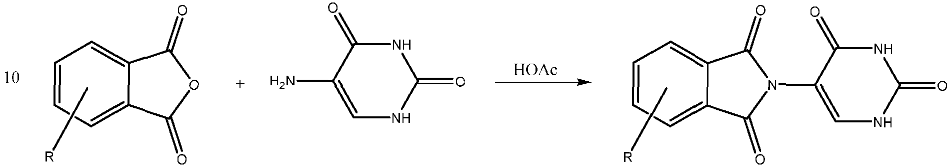

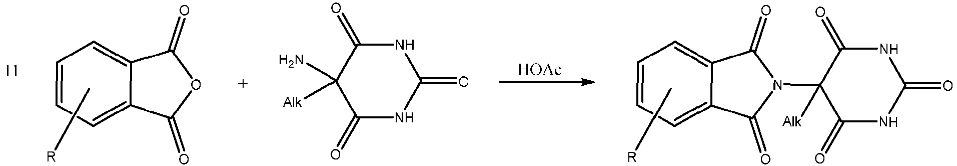

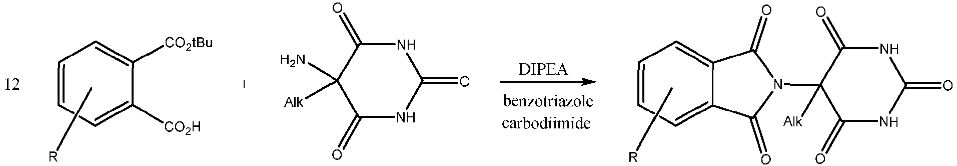

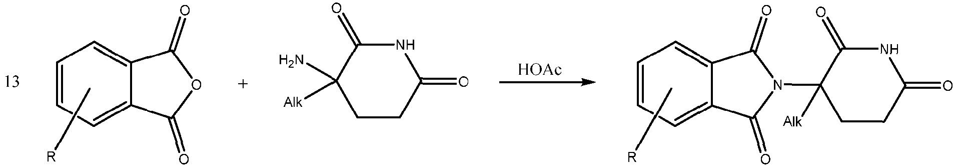

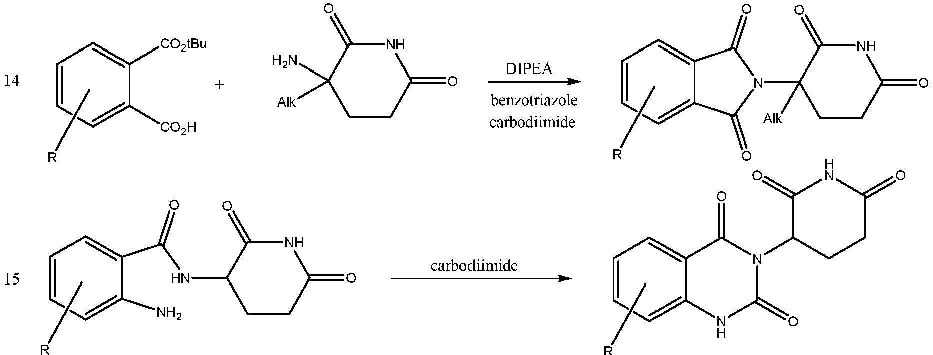

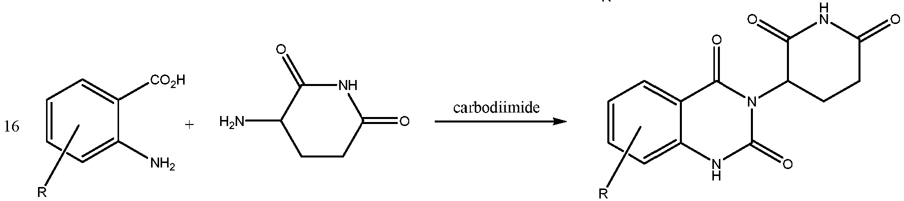

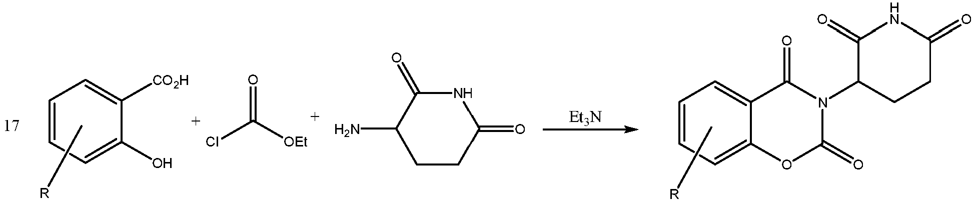

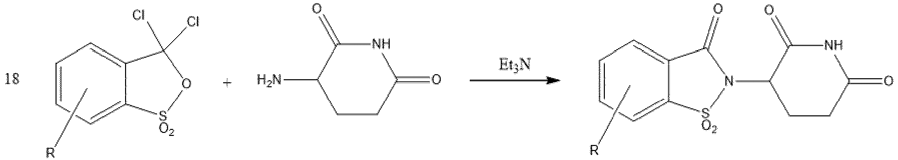

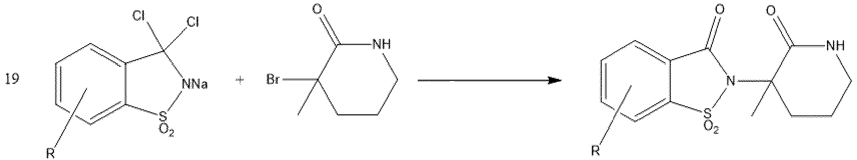

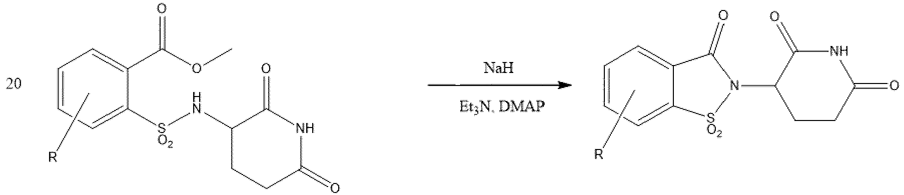

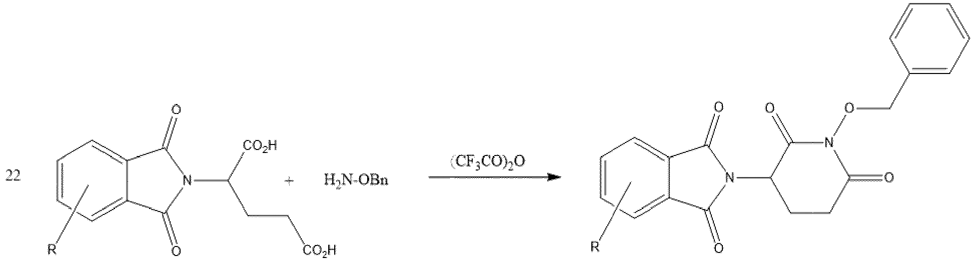

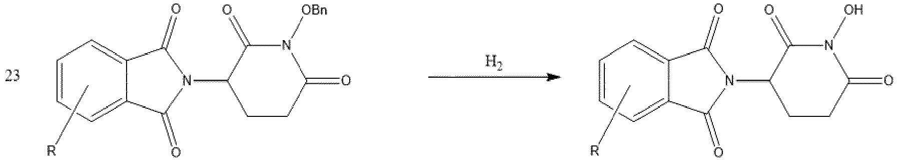

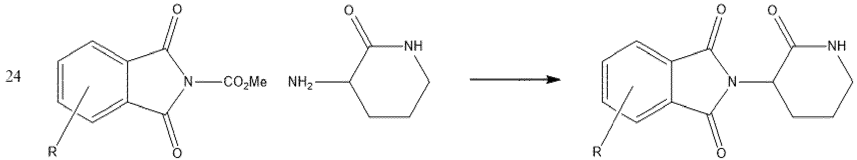

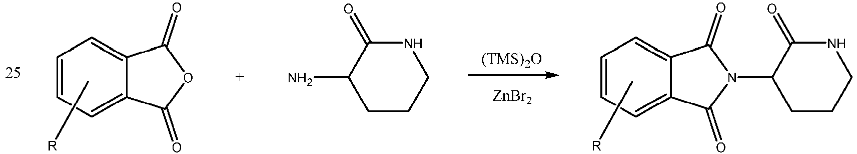

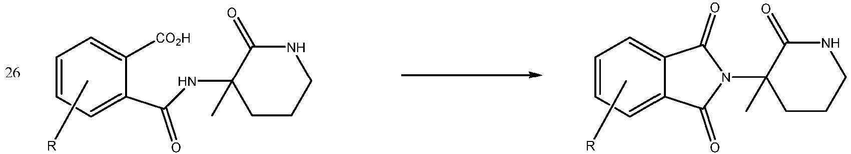

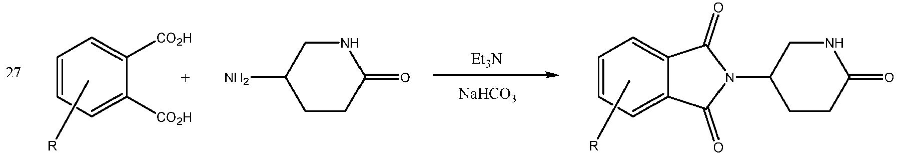

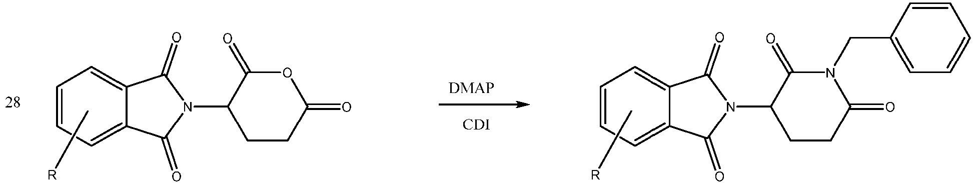

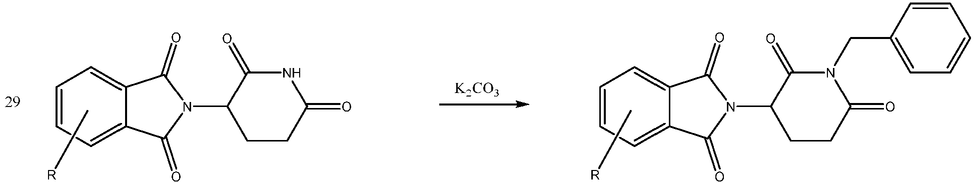

As shown in representative reaction 1, dimethyl phthalate derivatives can be condensed with glutamine (racemate or enantiomer) or glutamine analogs then further reacted with agents such as carbonyl diimidazole to form 2-(2,6-dioxopiperidin-3-yl)-2,3-dihydro-1H-isoindole-1,3-dione derivatives.

Alternatively as shown in representative reaction 2, the intermediate phthalimide produced in the initial condensation described above may be separately prepared and/or isolated and then reacted with dehydrating agents such as trifluoroacetamide, POCl₃ or acetic anhydride to form the desired 2-(2,6-dioxopiperidin-3-yl)-2,3-dihydro-1H-isoindole-1,3-dione derivatives. The same type of intermediate phthalimide can also be reacted with Lawesson's reagent prior to the dehydration step to provide thio analogs such as that shown in representative reactions 8 and 9.

Protected examples of 2-(2,6-dioxopiperidin-3-yl)-2,3-dihydro-1H-isoindole-1,3-dione derivatives such as the N¹-BOC species shown in representative example 3 can be deprotected to reveal the target 2-(2,6-dioxopiperidin-3-yl)-2,3-dihydro-1H-isoindole-1,3-dione derivatives by using, in this case, reagents such as TFA or silica.

Phthalic anhydrides such as that shown in representative example 4 can be ring-opened by reaction with amines such as 3-aminopiperidine-2,6-dione to form an intermediate carboxylate species, that on treatment with carbonyldiimidazole and benzotriazole will form the target 2-(2,6-dioxopiperidin-3-yl)-2,3-dihydro-1H-isoindole-1,3-dione derivatives. Alternatively, the two components may be combined in the presence of acetic acid to provide desired product as shown in representative reaction 13.

In an analogous reaction, anhydride derivatives like those shown in representative reaction 5 may be reacted with amines (ammonia in the example shown) then carbonyldiimidazoleto form the desired 2-(2,6-dioxopiperidin-3-yl)-2,3-dihydro-1H-isoindole-1,3-dione derivatives.

Where phthaloyl chlorides are available, direct condensation with glutamine (racemate or enantiomer) or glutamine analogs is possible, followed by further reaction with agents such as carbonyl diimidazole to form 2-(2,6-dioxopiperidin-3-yl)-2,3-dihydro-1H-isoindole-1,3-dione derivatives as shown in representative reaction 6.

o-Bromobenzamides can be reacted with a source of CO such as the acid chloride shown in representative reaction 7 in the presence of a palladium catalyst and associated phosphine ligand to produce the desired 2-(2,6-dioxopiperidin-3-yl)-2,3-dihydro-1H-isoindole-1,3-dione derivatives. Alternatively CO gas itself may be used in conjunction with rhodium (II) catalysts and silver carbonate to provide the desired products.

2-(2,4-dioxo-1,2,3,4-tetrahydropyrimidin-5-yl)-2,3-dihydro-1H-isoindole-1,3-dione, and 5-(1,3-dioxo-2,3-dihydro-1H-isoindol-2-yl)-1,3-diazinane-2,4,6-trione derivatives can be prepared by analogous means to some of the methods described above for 2-(2,6-dioxopiperidin-3-yl)-2,3-dihydro-1H-isoindole-1,3-dione derivatives. In representative reactions 20 and 21, a phthalic anhydride can be reacted with 5-amino-1,2,3,4-tetrahydropyrimidine-2,4-dione or 5-amino-1,3-diazinane-2,4,6-trione derivatives, respectively, in the presence of acetic acid to form the desired products.

Alternatively, 5-(1,3-dioxo-2,3-dihydro-1H-isoindol-2-yl)-1,3-diazinane-2,4,6-trione derivatives can be prepared by reaction of 5-amino-1,3-diazinane-2,4,6-trione derivatives with phthalic acid mono tert-butyl esters in the presence of Hünig's base, a carbodiimide and benzotriazole as shown in representative reaction 12. Similar conditions can be employed for the preparation of 2-(2,6-dioxopiperidin-3-yl)-2,3-dihydro-1H-isoindole-1,3-dione derivatives from phthalic acid mono tert-butyl esters as shown in representative reaction 14.

Compounds such as 3-(2,6-dioxopiperidin-3-yl)-1,2,3,4-tetrahydroquinazoline-2,4-dione can be prepared from anthranilic acid derivatives by reaction of 3-aminopiperidine-2,6-diones with a carbodiimide as in representative reaction 16. The intermediate benzamide product may be isolated (or separately produced) and further reacted with a carbodiimide to produce 3-(2,6-dioxopiperidin-3-yl)-1,2,3,4-tetrahydroquinazoline-2,4-dione derivatives as shown in representative reaction 15.

3-(2,6-Dioxopiperidin-3-yl)-3,4-dihydro-2H-1,3-benzoxazine-2,4-dione analogs can be prepared by activation of salicylic acids with chloroformates then condensation with 3-aminopiperidine-2,6-diones as shown in representative reaction 17.

3,3-Dichloro-2,1λ⁶-benzoxathiole-1,1-diones as shown in representative reaction 18 can be prepared by reaction of 2-sulfobenzoic acids with POCl₃ and PCl₅. These compounds can be reacted with amino derivatives to produce, for example, desired 2-(2,6-dioxopiperidin-3-yl)-2,3-dihydro-1λ⁶,2-benzothiazole-1,1,3-trione derivatives.

As shown in representative reaction 19, anions of saccharin derivatives can be alkylated with electrophiles such as the 3-bromo-3-methylpiperidin-2-one to produce targeted 2-(3-methyl-2-oxopiperidin-3-yl)-2,3-dihydro-1λ⁶,2-benzothiazole-1,1,3-trione derivatives.

Analogs of 2-(2,6-dioxopiperidin-3-yl)-2,3-dihydro-1λ⁶,2-benzothiazole-1,1,3-trione may also be prepared by reaction of methyl 2-[(2,6-dioxopiperidin-3-yl)sulfamoyl]benzoate with strong bases such as sodium hydride (see representative reaction 20).

Preparation of N¹-substituted compounds such as 2-[1-(benzyloxy)-2,6-dioxopiperidin-3-yl]-2,3-dihydro-1H-isoindole-1,4-dione (representative reaction 22) can be achieved by reaction of 2-(1,3-dioxo-2,3-dihydro-1H-isoindol-2-yl)pentanedioic acid with N-benzylhydroxylamine and with trifluoroacetic anhydride.

In turn molecules such as 2-[1-(benzyloxy)-2,6-dioxopiperidin-3-yl]-2,3-dihydro-1H-isoindole-1,4-dione (representative reaction 23) maybe subject to benzyl removal under hydrogenation conditions to yield N¹-hydroxy analogs such as 2-(1-hydroxy-2,6-dioxopiperidin-3-yl)-2,3-dihydro-1H-isoindole-1,3-dione.

In representative reaction 24, methyl 1,3-dioxo-2,3-dihydro-1H-isoindole-2-carboxylate (and analogs) is reacted with 3-aminopiperidin-2-one to provide 2-(2-oxopiperidin-3-yl)-2,3-dihydro-1H-isoindole-1,3-diones.

The same amine can also be reacted with phthalic anhydride derivatives in the presence of a Lewis acid such as zinc bromide and trimethylsilyl ether to yield the same type of product as shown in representative reaction 25. Intermediate products from this reaction if isolated or otherwise prepared (representative reaction 26) can be pushed to full cyclization through use of a dehydrating agent.

The isomeric derivatives such as 2-(6-oxopiperidin-3-yl)-2,3-dihydro-1H-isoindole-1,3-dione shown in representative reaction 27 are attainable through reaction of phthalic acid with 5-aminopiperidin-2-one.

Preparation of N¹-substituted compounds such as 2-(1-benzyl-2,6-dioxopiperidin-3-yl)-2,3-dihydro-1H-isoindole-1,4-dione (representative reactions 28 and 29) can be achieved through multiple routes. For example the anhydride (2-(2,6-dioxooxan-3-yl)-2,3-dihydro-1H-isoindole-1,3-dione) can be condensed with 3-aminopiperidine-2,6-dione in the presence of DMAP and carbonyldiimidazole (representative reaction 28), or 2-(2,6-dioxopiperidin-3-yl)-2,3-dihydro-1H-isoindole-1,3-dione derivatives can be alkylated with electrophiles such as benzyl bromide in the presence of base as shown in representative reaction 29.

In some instances, protecting group strategies and/or functional group interconversions (FGIs) may be required to facilitate the preparation of the desired materials. Such chemical processes are well known to the synthetic organic chemist and many of these may be found in texts such as "Greene's Protective Groups in Organic Synthesis" Peter G. M. Wuts and Theodora W. Greene (Wiley), and "Organic Synthesis: The Disconnection Approach" Stuart Warren and Paul Wyatt (Wiley).

### Protein Level Control

Also described are methods for the control of protein levels with a cell. This is based on the use of compounds as described herein, which are known to interact with a specific target protein such that degradation of a target protein *in vivo* will result in the control of the amount of protein in a biological system, prerferably to a particular therapeutic benefit.

The following examples are used to assist in describing the present invention, but should not be seen as limiting the present invention in any way.

### Specific Embodiments of the Present Disclosure

The present disclosure encompasses the following specific embodiments. In some embodiments, the compound of the present invention is selected from the group consisting of:
4-{3-[4-({l-[2-(2,6-dioxopiperidin-3-yl)-l,3-dioxo-2,3-dihydro-lH-isoindol-4-yl]-4,7,10-trioxa-l-azatridecan-13-yl}oxy)phenyl]-4,4-dimethyl-5-oxo-2-sulfanylideneimidazolidin-1-yl}-2-(trifluorornethyl)benzonitrile;
4-{3-[4-({l-[2-(2,6-dioxopiperidin-3-yl)-l,3-dioxo-2,3-dihydro-lH-isoindol-4-yl]-4,7,10-trioxa-l-azadodecan-12-yl}oxy)phenyl]-4,4-dimethyl-5-oxo-2-sulfanylideneimidazolidin-1-yl}-2-(trifluorornethyl)benzonitrile;
4-(3-{4-[(l-{2-[(3S)-2,6-dioxopiperidin-3-yl]-l,3-dioxo-2,3-dihydro-lH-isoindol-4-yl}-4,7,10-trioxa-l-azadodecan-12-yl)oxy]phenyl }-4,4-dimethyl-5-oxo-2-sulfanylideneimidazolidin-l-yl)-2-(trifluoromethyl)benzonitrile;
4-(3-{4-[(l-{2-[(3R)-2,6-dioxopiperidin-3-yl]-l,3-dioxo-2,3-dihydro-lH-isoindol-4-yl}-4,7,10-trioxa-l-azadodecan-12-yl)oxy]phenyl }-4,4-dimethyl-5-oxo-2-sulfanylideneimidazolidin-l-yl)-2-(trifluoromethyl)benzonitrile;
4-{3-[4-({l-[2-(2,6-dioxopiperidin-3-yl)-1,3-dioxo-2,3-dihydro-lH-isoindol-4-yl]-4,7,10,13,16-pentaoxa-l-azaoctadecan-18-yl}oxy)phenyl]-4,4-dimethyl-5-oxo-2-sulfanylideneimidazolidin-l-yl}-2-(trifluoromethyl)benzonitrile;
4-[3-(4-{2-[2-(2-{[2-(2,6-dioxopiperidin-3-yl)-l,3-dioxo-2,3-dihydro-lH-isoindol-4-yl]amino}ethoxy)ethoxy]ethoxy}phenyl)-4,4-dimethyl-5-oxo-2-sulfanylideneimidazolidin-l-yl]-2-(trifluoromethyl)benzonitrile;
4-[3-(4-{3-[2-(2-{[2-(2,6-dioxopiperidin-3-yl)-l,3-dioxo-2,3-dihydro-lH-isoindol-4-yl]amino}ethoxy)ethoxy]propoxy}phenyl)-4,4-dimethyl-5-oxo-2-sulfanylideneimidazolidin-1-yl]-2-(trifluoromethyl)benzonitrile;
4-{3-[4-({l-[2-(2,6-dioxopiperidin-3-yl)-l,3-dioxo-2,3-dihydro-lH-isoindol-4-yl]-4,7,10-trioxa-l-azatetradecan-14-yl}oxy)phenyl]-4,4-dimethyl-5-oxo-2-sulfanylideneimidazolidin-l-yl}-2-(trifluorornethyl)benzonitrile;
4-{4,4-dimethyl-3-[4-({l-[2-(3-methyl-2,6-dioxopiperidin-3-yl)-l,3-dioxo-2,3-dihydro-1H-isoindol-4-yl]-4,7,10-trioxa-1-azatridecan-13-yl}oxy)phenyl]-5-oxo-2-sulfanylideneimidazolidin-l-yl}-2-(trifluoromethyl)benzonitrile;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[4-({l-[2-(2,6-dioxopiperidin-3-yl)-l,3-dioxo-2,3-dihydro-lH-isoindol-4-yl]-4,7,10-trioxa-l-azadodecan-12-y1} oxy)phenyl] acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[4-({l-[2-(2,6-dioxopiperidin-3-yl)-l,3-dioxo-2,3-dihydro-lH-isoindol-4-yl]-4,7,10,13-tetraoxa-lazapentadecan-15-yl}oxy)phenyl]acetamide:
   2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-(4-{2-[2-(2-{[2-(2,6-dioxopiperidin-3-yl)-1,3-dioxo-2,3-dihydro-1H-isoindol-4-yl] amino} ethoxy)ethoxy] ethoxy } phenyl)acetamide;
   2-[(9R)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[4-({l-[2-(2,6-dioxopiperidin-3-yl)-l-oxo-2,3-dihydro-lH-isoindol-4-yl]-4,7,10-trioxa-l-azadodecan-12-yl}oxy)phenyl]acetamide; and
   4-(4-{[(5Z)-3-{2-[2-(2-{[2-(2,6-dioxopiperidin-3-yl)-1,3-dioxo-2,3-dihydro-1H-isoindol-4-yl]amino}ethoxy)ethoxy]ethyl}-2,4-dioxo-1,3-thiazolidin-5-ylidene]methyl}-2-methoxyphenoxy)-3-(trifluoromethyl)benzonitrile.
In some embodiments, the compound of the present invention is selected from the group consisting of:
4-[3-(4-{3-[3-(2-{[2-(2,6-dioxopiperidin-3-yl)-l,3-dioxo-2,3-dihydro-lH-isoindol-4-yl]amino}ethoxy)propoxy]propoxy}phenyl)-4,4-dimethyl-5-oxo-2-sulfanylideneimidazolidin-l-yl]-2-(trifluoromethyl)benzonitrile;
4-(3-{4-[2-(2-{[2-(2,6-dioxopiperidin-3-yl)-l,3-dioxo-2,3-dihydro-lH-isoindol-4-yl]amino}ethoxy)ethoxy]phenyl}-4,4-dimethyl-5-oxo-2-sulfanylideneimidazolidin-l-yl)-2-(trifluoromethyl)benzonitrile;
4-{[5-(3-{[2-(2,6-dioxopiperidin-3-yl)-l,3-dioxo-2,3-dihydro-lH-isoindol-4-yl]amino}propoxy)pentyl]oxy}-N-[trans-3-(3-chloro-4-cyanophenoxy)-2,2,4,4-tetramethyl cyclobutyl]benzamide;
4-[3-(4-{4-[(5-{[2-(2,6-dioxopiperidin-3-yl)-l,3-dioxo-2,3-dihydro-lH-isoindol-4-yl]amino}pentyl)oxy]pheny}phenyl)-4,4-dimethyl-5-oxo-2-sulfanylideneimidazolidin-l-yl]-2-(trifluoromethyl)benzonitrile;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[(lS)-l-[4-(4-{[2-(2,6-dioxopiperidin-3-yl)-1,3-dioxo-2,3-dihydro-1H-isoindol-4-yl]amino}butoxy)phenyl]ethyl]acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[3-(3-{[2-(2,6-dioxopiperi din-3-yl)-1,3-dioxo-2,3-dihydro-1H-isoindol-4-yl]amino}propoxy)propyl]acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-(3-{[2-(2,6-dioxopiperidin-3-yl)-l,3-dioxo-2,3-dihydro-lH-isoindol-4-yl]amino}propyl)acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[(lS)-l-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[2-(2-{[2-(2,6-dioxopiperidin-3-yl)-l,3-dioxo-2,3-dihydro-lH-isoindol-4-yl]amino}ethoxy)ethyl]acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[(lR)-l-[4-(4-{[2-(2,6-dioxopiperidin-3-yl)-1,3-dioxo-2,3-dihydro-1H-isoindol-4-yl]amino}butoxy)phenyl]ethyl] acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-[(lR)-l-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamide;
2-[(9S)-7-(4-chlorophenyl)-4,5,13-trimethyl-3-thia-l,8,11,12-tetraazatricyclo[8.3. 0.0²,⁶]trideca-2(6),4,7,10,12-pentaen-9-yl]-N-[(lR)-l-[4-(3-{[2-(2,6-dioxopiperidin-3-yl)-1,3-dioxo-2,3-dihydro-1H-isoindol-4-yl]amino}propoxy)phenyl]ethyl]acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-{2-[4-(3-{[2-(2,6-dioxopiperidin-3-yl)-1,3-dioxo-2,3-dihydro-1H-isoindol-4-yl]amino}propoxy)phenyl]pyrimidin-5-yl}acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-{4-[3-(2-{[2-(2,6-dioxopiperidin-3-yl)-l,3-dioxo-2,3-dihydro-lH-isoindol-4-yl]amino}ethoxy)propoxy]-3-fluorophenyl}acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-l,3-dioxo-2,3-dihydro-lH-isoindol-4-yl]amino}propoxy)butoxy]-2-fluorophenyl}acetamide;
2-[(9S)-7-(4-chlorophenyl)-4,5,13-trimethyl-3-thia-l,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-l,3-dioxo-2,3-dihydro-lH-isoindol-4-yl]amino}propoxy)butoxy]-3-fluorophenyl}acetamide;
4-(4-{[(5Z)-3-[2-(2-{[2-(2,6-dioxopiperidin-3-yl)-1,3-dioxo-2,3-dihydro-1H-isoindol-4-yl]amino}ethoxy)ethyl]-2,4-dioxo-1,3-thiazolidin-5-ylidene]methyl}-2-methoxyphenoxy)-3-(trifluoromethyl)benzonitrile; and
4-(4-{[(5Z)-3-[3-(2-{[2-(2,6-dioxopiperidin-3-yl)-1,3-dioxo-2,3-dihydro-1H-isoindol-4-yl]amino}ethoxy)propyl]-2,4-dioxo-1,3-thiazolidin-5-ylidene]methyl}-2-methoxyphenoxy)-3-(trifluoromethyl)benzonitrile

In certain embodiments, described herein is a pharmaceutical composition comprising the compound of the present invention, or a pharmaceutically acceptable salt, enantiomer or stereoisomer thereof, and a pharmaceutically acceptable carrier, additive, and/or excipient. In certain embodiments, the pharmaceutical composition further comprises a bioactive agent. In certain embodiments, the pharmaceutical composition comprises a bioactive agent that is an antiviral agent. In certain embodiments, the pharmaceutical composition comprises an antiviral agent that is an anti-HIV agent. In certain embodiments, the pharmaceutical composition comprises an anti-HIV agent that is a nucleoside reverse transcriptase inhibitor (NRTI), a non-nucleoside reverse transcriptase inhibitor, a protease inhibitor, a fusion inhibitor, or a mixture thereof. In certain embodiments, the pharmaceutical composition comprises an antiviral agent that is an anti-HCV agent. In certain embodiments, the pharmaceutical composition comprises a bioactive agent selected from the group consisting of an antiinflammation agent, an immunological agent, a cardiovascular agent, and a neurological agent. In certain embodiments, the pharmaceutical composition comprises a bioactive agent that is an anticancer agent.

In certain embodiments, the pharmaceutical composition comprises an anticancer agent selected from the group consisting of a FLT-3 inhibitor, a VEGFR inhibitor, an EGFR TK inhibitor, an aurora kinase inhibitor, a PIK-1 modulator, a Bcl-2 inhibitor, an HDAC inhbitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, an mTORC1/2 inhibitor, a JAK/STAT inhibitor, a checkpoint-1 or 2 inhibitor, a focal adhesion kinase inhibitor, a Map kinase kinase (mek) inhibitor, a VEGF trap antibody and mixtures thereof.

In certain embodiments, disclosed herein is an effective amount of the compound of the present invention for use in a method of inducing degradation of a target protein in a cell. In certain embodiements, disclosed herein is an effective amount of the compound of the present invention for use in a method of inducing degradation of a target protein in a patient. In certain embodiments, disclosed herein is an effective amount of the compound of the present invention for use in a method of treating a disease state or condition in a patient wherein dysregulated protein activity is responsible for said disease state or condition.

In certain embodiments, disclosed herein is a pharmaceutical composition of the present invention, comprising an anti-cancer agent, for use in a method of treating cancer. In certain embodiments, the cancer treated is selected from the group consisting of ; leukemias; multiple myeloma, breast cancer, prostate cancer, uterine cancer, ovarian cancer, colon cancer and Large B-cell Lymphoma.

### EXAMPLES

### A. Assays

### 1. CRBN assay - Cloning, expression and purification of human CRBN and DDB1

The proceedure is standard to one versed in the art, as typified by the description in Lopez-Girona et al. (Cereblon is a direct protein target for immunomodulatory and antiproliferative activities of lenalidomide and pomalidomide, A Lopez-Girona, D Mendy, T Ito, K Miller, A K Gandhi, J Kang, S Karasawa, G Carmel, P Jackson, M Abbasian, A Mahmoudi, B Cathers, E Rychak, S Gaidarova, R Chen, P H Schafer, H Handa, T O Daniel, J F Evans and R Chopra, Leukemia 26: 2326-2335, 2012).

The cDNAs for the CRBN and DDB1 genes can be amplified by PCR using Pfusion (NEB) as the polymerase and the following primer sequences:

| **Primer** | **Sequence** |
|---|---|
| CRBN-Forward | GTGCCGCGTGGCTCCATGGCCGGCGAAGGAGATCAGCAGGA (SEQ ID NO: 1) |
| CRBN-Rev | GCTTCCTTTCGGGCTTATTACAAGCAAAGTATTACTTTGTC (SEQ ID NO: 2) |
| DDB1-Forward | TCGGGCGCGGCTCTCGGTCCGAAAAGGATGTCGTACAACTACGTGGTAAC (SEQ ID NO: 3) |
| DDB1-Rev | GCTTCCTTTCGGGCTTATTTTTCGAACTGCGGGTGGCTCCAATGGATCCGAGTTAGCTCCT (SEQ ID NO: 4) |
| CRBN-Flag-Rev | GCTTCCTTTCGGGCTTACTTATCGTCATCGTCCTTGTAGTCCAAGCAAAGTATTACTTTGT (SEQ ID NO: 5) |

CRBN can be cloned into pBV-ZZ-HT-LIC, pBV-GST-LIC, pMA-HT-LIC, and DDB1 into pBV-notag-LIC, using ligation-independent cloning 26. For cloning into the mammalian vector pMA-HT-LIC, the CRBN-Flag-Reverse oligo adds a C-terminal FLAG tag for immunodetection. The DDB1-Rev adds a StrepTag 27. A ZZ-tag 28 is necessary to achieve high expression of soluble CRBN; without it, the His-CRBN expressed at low level, while a GST-CRBN results in aggregated protein. Recombinant baculovirus of ZZ-His-CRBN and DDB 1-StrepTag (ST) are generated and amplified using Bac-to-Bac baculovirus expression system from Invitrogen in Sf9 insect cells. ZZ-His-CRBN and DDB1-ST are co-expressed in High Five (Tni) insect in 10L wave bags at 27 °C using un-supplemented ESF921 media from Expression Systems. Cells are harvested 48 hours post infection by centrifugation and paste re-suspended in PBS plus5X Protease Inhibitor cocktail (Roche, Indianapolis, IN).

All subsequent protein purification steps are carried out at 4 °C. Frozen cells are thawed, resuspended in 5 volumes of lysis buffer (50 mM Tris HCl pH 8.0, 0.5 M NaCl, 10% glycerol, 2 mM DTT ) plus 20 mM imidazole and protease inhibitors, lysed and centrifuged to yield a clear supernatant. The CRBN-DDB1 is purified on a ÄKTA-xpress system (GE Healthcare) using a Nickel-Sepharose and S200 Sephacryl chromatography. The complex is then further purified using anion exchange chromatography on an 8 ml MonoQ column and a second pass on a S-200 gel filtration. CRBN-DDB1 is identified by SDS-PAGE and the CRBN-DDB1 containing fractions were pooled and stored at -70 °C.

### 2. Fluorescence thermal melt assay to measure binding of compounds to recombinant CRBN

The assay is standard to one versed in the art, as typified by the description in Lopez-Girona et al. (Cereblon is a direct protein target for immunomodulatory and antiproliferative activities of lenalidomide and pomalidomide, A Lopez-Girona, D Mendy, T Ito, K Miller, A K Gandhi, J Kang, S Karasawa, G Carmel, P Jackson, M Abbasian, A Mahmoudi, B Cathers, E Rychak, S Gaidarova, R Chen, P H Schafer, H Handa, T O Daniel, J F Evans and R Chopra, Leukemia 26: 2326-2335, 2012).

Thermal stabilities of CRBN-DDB1 in the presence or absence of test compounds are done in the presence of Sypro Orange in a microplate format according to Pantoliano et al. (Pantoliano MW, Petrella EC, Kwasnoski JD, Lobanov VS, Myslik J, Graf E et al. High-density miniaturized thermal shift assays as a general strategy for drug discovery. J Biomol Screen 2001; 6: 429-440.) Two mg of protein in 20 ml of assay buffer (25 mM Tris HCl, pH 8.0, 150 mM NaCl, 2 uM Sypro Orange) are subjected to stepwise increase of temperature from 20 to 70 °C and the fluorescence read at every 1 °C on an ABIPrism 7900HT (Applied Biosystems, Carlsbad, CA, USA). Compounds are dissolved in DMSO (1% final in assay) and tested in quadruplicate at a concentration range between 30 nM to 1000 uM; controls contained 1% DMSO only.

### 3. LCMS Method

The analysis is conducted on a Poroshell 120 EC C18 column (50mm x 3.0mm internal diameter 2.7µm packing diameter) at 45°C.

The solvents employed are:
A = 0.1% v/v solution of formic acid in water.
B = 0.1% v/v solution of formic acid in acetonitrile.

The gradient employed are as follows:

| **Time (minutes)** | **Flow Rate (mL/min)** | **%A** | **%B** |
|---|---|---|---|
| 0 | 1 | 95 | 5 |
| 0.5 | 1 | 95 | 5 |
| 3.0 | 1 | 1 | 99 |
| 3.75 | 1 | 1 | 99 |
| 4.0 | 1 | 95 | 5 |

The UV detection is an averaged signal from wavelength of 210nm to 350nm and mass spectra are recorded on a mass spectrometer using positive mode electrospray ionization.

The following illustrates the mobile phases and gradients used when compounds undergo purification by preparative HPLC.

### 4. Preparative HPLC (Formic Acid Modifier)

The HPLC analysis is conducted on an X Bridge RP18 OBD column (150mm x 19mm internal diameter, 5µm packing diameter) at ambient temperature.

The solvents employed are:
A = 0.1% v/v solution of formic acid in water.
B = acetonitrile.

### 5. Preparative HPLC (Ammonium Bicarbonate Modifier)

The HPLC analysis is conducted on an X Bridge RP18 OBD column (150mm x 19mm internal diameter, 5µm packing diameter) at ambient temperature.

The solvents employed are:
A = 10 mM ammonium bicarbonate in water.
B = acetonitrile.

For each of the preparative purifications, irrespective of the modifier used, the gradient employed is dependent upon the retention time of the particular compound undergoing purification as recorded in the analytical LCMS. The flow rate is 20 mL/min.

The UV detection is a signal from wavelength of 254nm or 220nm.

While preferred embodiments of the invention have been shown and described herein, it will be understood that such embodiments are provided by way of example only.

### B. Synthesis:

The synthetic details for the examples included below are representative of the general procedures that inform on the synthesis of the broader example set. Syntheses of compounds not claimed are included for reference and are marked accordingly with "reference".

### 1. 2-(2,6-dioxopiperidin-3-yl)-4-fluoro-2,3-dihydro-1H-isoindole-1,3-dione

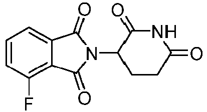

### Step 1: 4-fluoroisobenzofuran-1,3-dione

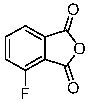

A mixture of 3-fluorophthalic acid (50 g, 271.7 mmol) in acetic anhydride (400 mL) was refluxed for 2 h. The volatiles were removed by vacuum, and the residues were crystallized in acetic anhydride to afford 4-fluoroisobenzofuran-1,3-dione (40 g, crude) as a brown solid. LC-MS: 167.1 [MH]⁺. ¹H NMR (400 MHz, CDCl₃): δ 7.58 (t, *J =* 8.0 Hz, 1H), 7.86 (d, *J=* 7.2 Hz, 1H), 7.92-7.97 (m, 1H).

### Step 2: 5-amino-2-(4-fluoro-1,3-dioxoisoindolin-2-yl)-5-oxopentanoic acid

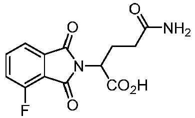

A mixture of the above 4-fluoroisobenzofuran-1,3-dione (40 g, crude) and L-glutamine (35 g, 239 mmol) in dry DMF (200 mL) was stirred at 90 °C for 8 h. The solvent was removed under reduced pressure. The residue was re-dissolved in 4N HCl (200 mL) and stirred for additional 8 h. The resulting precipitation was collected by filtration, washed with water, and dried to afford 5-amino-2-(4-fluoro-1,3-dioxoisoindolin-2-yl)-5-oxopentanoic acid (37 g, crude) as an off-white solid. LC-MS: 295.2 [MH]⁺. ¹H NMR (400 MHz, CDCl₃): δ 2.16-2.20 (m. 2H), 2.31-2.43 (m, 2H), 4.79-4.83 (m, 1H), 6.79 (br, 1H), 7.26(br, 1H), 7.77-7.85 (m, 2H), 7.98-8.03 (m, 1H), 13.32(br, 1H).

### Step 3: 2-(2,6-dioxopiperidin-3-yl)-4-fluoro-2,3-dihydro-1H-isoindole-1,3-dione

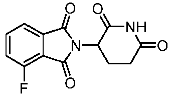

A mixture of the above 5-amino-2-(4-fluoro-1,3-dioxoisoindolin-2-yl)-5-oxopentanoic acid (37 g, crude), 1,1'-carbonyldiimidazole (CDI) (24.2 g, 149.4 mmol) and 4-dimethylaminopyridine (DMAP) (1.3 g, 11.5 mmol) in acetonitrile (80 mL) was refluxed for 5 h. The reaction mixture was cooled to room temperature. The resulting solid was collected by filtration, and washed with acetonitrile (100 mL) to afford the crude product, which was purified by silica gel chromatography using 1-10% MeOH in DCM as eluent to afford 2-(2,6-dioxopiperidin-3-yl)-4-fluoroisoindoline-1,3-dione (9.0 g, 12% yield over three steps ) as a light yellow solid. LC-MS: 277.2 [MH]⁺. ¹H NMR (400 MHz, CDCl₃): δ 2.14-2.19 (m, 1H), 2.75-2.95 (m, 3H), 4.97-5.01 (m, 1H), 7.43 (t, J = 8.4 Hz, 1H), 7.10-7.81 (m, 2H), 8.08 (br, 1H).

### 2. N-(3-(5-bromo-2-chloropyrimidin-4-ylamino)propyl)-N-methylcyclobutane carboxamide

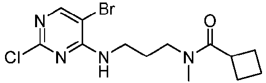

### Step 1: tert-butyl N-{3-[(5-bromo-2-chloropyrimidin-4-yl)amino]propyl}-N-methylcarbamate

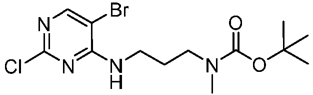

A mixture of *tert*-butyl N-(3-aminopropyl)-N-methylcarbamate (826 mg, 4.40 mmol) and 5-bromo-2,4-dichloropyrimidine (400 mg, 1.76 mmol) in MeOH (10 mL) was stirred at rt for 1 h. The reaction mixture was then concetrated *in vacuo,* and the residue was purfied using a Teledyne ISCO Chromatography [0→35% EtOAc/Heptanes] to afford *tert*-butyl N-{3-[(5-bromo-2-chloropyrimidin-4-yl)amino]propyl}-N-methylcarbamate (615 mg*,* 92% yield). LC-MS (ES⁺): *m*/*z* = 381.05/383.05 [MH⁺], *t_{R}* = 2.55 min.

### Step 2: {3-[(5-bromo-2-chloropyrimidin-4-yl)amino]propyl}(methyl)amine

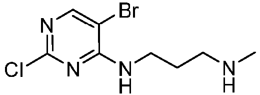

To a solution of *tert*-butyl N-{3-[(5-bromo-2-chloropyrimidin-4-yl)amino]propyl}-N-methylcarbamate (615 mg, 1.62 mmoL) in DCM (5 mL) was added trifluoroacetic acid (0.54 mL, 6.5 mmol) at rt. After the mixture was stirred for 1 h, it was concetrated *in vacuo.* The residue was purified using a Teledyne ISCO Chromatography [0 → 15% methanol in DCM] to afford {3-[(5-bromo-2-chloropyrimidin-4-yl)amino]propyl}(methyl)amine (371 mg, 82% yield). LC-MS (ES⁺): *m*/*z* = 280.99/282.99 [MH⁺], *t_{R} =* 1.13 min.

### Step 3: N-{3-[(5-bromo-2-chloropyrimidin-4-yl)amino]propyl}-N-methylcyclobutanecarboxamide

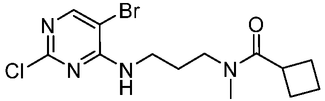

To a solution of {3-[(5-bromo-2-chloropyrimidin-4-yl)amino]propyl}(methyl)amine (371 mg, 1.33 mmol) and cyclobutanecarbonyl chloride (188 mg, 1.60 mmol) in DCM (10 mL) at rt was added triethyl amine (0.41 mL, 2.92 mmol). The reaction mixture was left to stir at rt for 16 h, then concetrated *in vacuo.* The residue was purified using a Teledyne ISCO Chromatography [0 → 100% EtOAc/Heptanes] to afford N-{3-[(5-bromo-2-chloropyrimidin-4-yl)amino]propyl}-N-methylcyclobutane carboxamide (268 mg, 56%). LC-MS (ES⁺): *m*/*z* = 363.04/365.04 [MH⁺], *t_{R}* = 2.18 min.

### 3. (S)-2-(4-(4-chlorophenyl)-2,3,9-trimethyl-6H-thieno [3,2-f] [1,2,4]triazolo[4,3-a][1,4]diazepin-6-yl)acetic acid

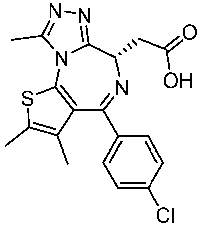

The title compound was prepared according to the procedures described in WO2011/143660

### 4. (Z)-4-(4-((2,4-dioxothiazolidin-5-ylidene)methyl)-2-methoxyphenoxy)-3-(trifluoromethyl)benzonitrile

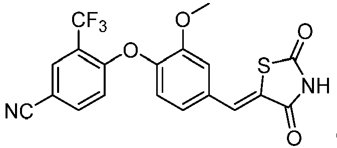

The title compound was prepared according to the procedures described in Patch, R. J. et al J. Med. Chem. 2011, 54, 788-808.

### 5. 4-[3-(4-hydroxyphenyl)-4,4-dimethyl-5-oxo-2-sulfanylideneimidazolidin-1-yl]-2-(trifluoromethyl)benzonitrile

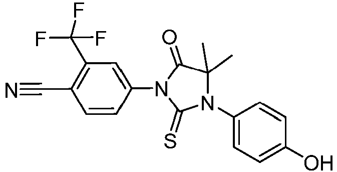

The title compound was prepared according to the procedures described in Jung, M. E. et al J. Med. Chem. 2010, 53, 2779-2796.

### 6. 2-chloro-4-(trans-3-amino-2,2,4,4-tetramethylcyclobutoxy)benzonitrile hydrogen chloride salt

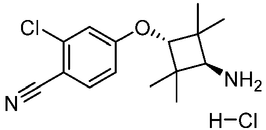

The title compound was prepared according to the procedures described in Guo, C. et al J. Med. Chem. 2011, 54, 7693-7704.

### 7. [N-(3-(5-bromo-2-(4-(2-(2-(2-(2-(2-(2,6-dioxopiperidin-3-yl)-1,3-dioxoisoindolin-4-ylamino)ethoxy)ethoxy)ethoxy)ethoxy)phenylamino)pyrimidin-4-ylamino)propyl)-N-methylcyclobutanecarboxamide] (reference)

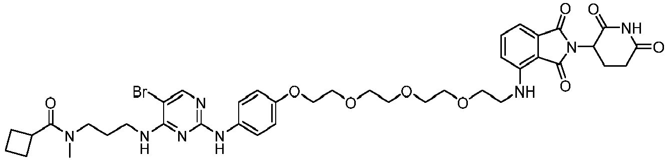

(Compound Structure #17 shown in Table 1)

### Step 1: 2-(2-(2-(2-(4-nitrophenoxy)ethoxy)ethoxy)ethoxy)ethyl 4-methylbenzenesulfonate

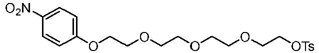

A mixture of 2,2'-(2,2'-oxybis(ethane-2,1-diyl)bis(oxy))bis(ethane-2,1-diyl) bis(4-methylbenzenesulfonate) (3 g, 5.96 mmol), 4-nitrophenol (813 mg, 5.84 mmol) and potassium carbonate (1.65 g, 11.94 mmol) in dry N,N-dimethylformamide (20 mL) was stirred at 50 °C overnight. The mixture was cooled to room temperature and poured into water (60 mL), then extracted with ethyl acetate (80 mL x 3). The combined organic phases were washed with water (50 mL) and brine (50 mL), dried over anhydrous sodium sulfate, and concentrated under reduced pressure. The residue was purified by silica gel flash column chromatography (eluted with 10-20% ethyl acetate in hexane) to afford 2-(2-(2-(2-(4-nitrophenoxy)ethoxy)ethoxy)ethoxy)ethyl 4-methylbenzenesulfonate (2.65 g , 95% yield) as a yellow oil. LC-MS (ES⁺): m/z 470.2 [MH⁺] (t_{R} = 2.83 min)

### Step 2: [1-(2-(2-(2-(2-azidoethoxy)ethoxy)ethoxy)ethoxy)-4-nitrobenzene]

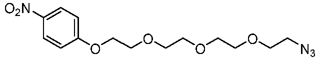

A mixture of 2-(2-(2-(2-(4-nitrophenoxy)ethoxy)ethoxy)ethoxy)ethyl 4-methylbenzenesulfonate (2.65 g, 5.64 mmol) and sodium azide (734 mg, 11.29 mmol) in ethanol (30 mL) was refluxed for 16 h. The mixture was cooled to room temperature, quenched with water (50 mL), and extracted with dichloromethane (50 mL x 3). The combined organic phases were washed with water (50 mL) and brine (40 mL), dried over anhydrous sodium sulfate, and concentrated under reduced pressure to afford the crude 1-(2-(2-(2-(2-azidoethoxy)ethoxy)ethoxy)ethoxy)-4-nitrobenzene (865 mg) as a yellow oil.

### Step 3: [2-(2-(2-(2-(4-nitrophenoxy)ethoxy)ethoxy)ethoxy)ethanamine]

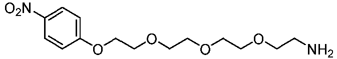

A mixture of the above 1-(2-(2-(2-(2-azidoethoxy)ethoxy)ethoxy)ethoxy)-4-nitrobenzene (865 mg, 2.54 mmol), triphenylphosphine (999 mg, 3.81 mmol) and water (69 mg, 3.83 mmol) in tetrahydrofuran (10 mL) was stirred at room temperature for 14 h under nitrogen atmosphere. The volatiles were removed under reduced pressure to afford a crude residue, which was purified by silica gel flash column chromatography (eluted with 3-5% methanol in dichloromethane) to afford 2-(2-(2-(2-(4-nitrophenoxy)ethoxy)ethoxy)ethoxy)ethanamine (661 mg , 83% yield) as a yellow oil. ¹H NMR (400 MHz, CDCl₃): δ 2.86 (t, J = 5.2 Hz, 2H), 3.51 (t, J = 5.6 Hz, 2H), 3.63-3.75 (m, 8H), 3.90 (t, J = 4.4 Hz, 2H), 4.23 (t, J = 4.8 Hz, 2H), 6.97-6.99 (m, 2H), 8.18-8.22 (m, 2H).

### Step 4: tert-butyl 2-(2-(2-(2-(4-nitrophenoxy)ethoxy)ethoxy)ethoxy)ethylcarbamate

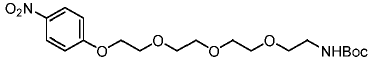

A mixture of 2-(2-(2-(2-(4-nitrophenoxy)ethoxy)ethoxy)ethoxy)ethanamine (661 mg, 2.1 mmol), triethylamine (449 mg, 4.43 mmol) and di-tert-butyl dicarbonate(505 mg, 2.31 mmol) in dichloromethane (25 mL) was stirred at room temperature for 2 h. The mixture was diluted with dichloromethane (100 mL), washed with water (30 mL x 2) and brine (30 mL), dried over anhydrous sodium sulfate, and concentrated under reduced pressure. The residue was purified by silica gel flash column chromatography (eluted with 20-40% ethyl acetate in hexane) to afford tert-butyl 2-(2-(2-(2-(4-nitrophenoxy)ethoxy)ethoxy)ethoxy)ethylcarbamate (818 mg, 94% yield) as a yellow oil. ¹H NMR (400 MHz, CDCl₃): δ 1.44 (s, 9H), 3.37 (d, J = 5.2 Hz, 2H), 3.54 (t, J = 5.2 Hz, 2H), 3.62-3.70 (m, 6H), 3.73-3.76 (m, 2H), 3.90 (t, J = 4.4 Hz, 2H), 4.23 (t, J = 4.8 Hz, 2H), 5.01(br, 1H), 6.96-7.00 (m, 2H), 8.18-8.22 (m, 2H).

### Step 5: tert-butyl 2-(2-(2-(2-(4-aminophenoxy)ethoxy)ethoxy)ethoxy)ethylcarbamate

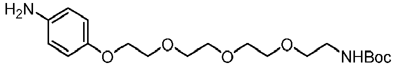

A mixture of 2-(2-(2-(2-(4-nitrophenoxy)ethoxy)ethoxy)ethoxy)ethylcarbamate (818 mg, 1.97 mmol), iron powder (1.1 g, 0.65 mmol), and ammonium chloride (528 mg, 9.87 mmol) in ethanol (20 mL) and water (5 mL) was stirred at 80 °C for 1 h. The mixture was cooled to room temperature, the solid precipitate was removed by filtration and washed with ethyl acetate (20 mL x 2). The filtrate was partitioned between ethyl acetate (120 mL) and water (30 mL). The organic phase was washed with brine (30 mL), dried over anhydrous sodium sulfate, and concentrated under reduced pressure. The residue was purified by silica gel chromatography (eluted with 30-40% ethyl acetate in hexane) to afford tert-butyl 2-(2-(2-(2-(4-aminophenoxy)ethoxy)ethoxy)ethoxy)ethylcarbamate (512 mg, 67% yield) as a yellow oil.

### Step 6: tert-butyl 2-(2-(2-(2-(4-(5-bromo-4-(3- (N-methylcyclobutanecarboxamido) propylamino)pyrimidin-2-ylamino)phenoxy)ethoxy)ethoxy)ethoxy)ethylcarbamate

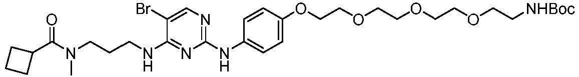

A mixture of tert-butyl 2-(2-(2-(2-(4-aminophenoxy)ethoxy)ethoxy)ethoxy)ethyl carbamate (130 mg, 0.34 mmol), N-(3-(5-bromo-2-chloropyrimidin-4-ylamino)propyl)-N-methylcyclobutanecarboxamide (24 mg, 0.06 mmol) and *p*-toluenesulfonic acid (11.6 mg, 0.07 mmol) in dioxane (1.5 mL) was refluxed for 16 h. The reaction mixture was cooled to room temperature, quenched with aqueous sodium bicarbonate solution (1.0 N, 30 mL), and extracted with ethyl acetate (30 mL x 3). The combined organic phases were washed with water (30 mL) and brine (30 mL), dried over anhydrous sodium sulfate, and concentrated under reduced pressure. The crude residue was purified by silica gel flash column chromatography (eluted with 50% ethyl acetate in hexane) to afford *tert*-butyl 2-(2-(2-(2-(4-(5-bromo-4-(3-(N-methylcyclobutanecarboxamido)propylamino)pyrimidin-2-ylamino)phenoxy)ethoxy)ethoxy)ethoxy)ethylcarbamate (40 mg, 17% yield) as a yellow oil.

### Step 7: N-(3-(2-(4-(2-(2-(2-(2-aminoethoxy)ethoxy) ethoxy)ethoxy)phenylamino)-5-bromopyrimidin-4-ylamino)propyl)-N-methylcyclobutanecarboxamide

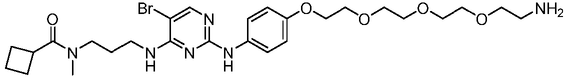

A mixture of *tert*-butyl 2-(2-(2-(2-(4-(5-bromo-4-(3-(N-methylcyclobutanecarboxamido) propylamino)pyrimidin-2-ylamino)phenoxy) ethoxy)ethoxy)ethoxy)ethylcarbamate (40 mg, 0.06 mmol) in 2,2,2-trifluoroacetic acid (1 mL) and dichloromethane (1 mL) was stirred at room temperature for 2 h. The volatiles were removed under reduced pressure. The residue was partitioned between dichloromethane (60 mL) and aqueous sodium bicarbonate solution (2.0 N, 30 mL). The organic layer was washed with brine (20 mL), dried over anhydrous sodium sulfate, and concentrated under reduced pressure to afford N-(3-(2-(4-(2-(2-(2-(2-aminoethoxy)ethoxy)ethoxy)ethoxy)phenylamino)-5-bromopyrimidin-4-ylamino)propyl)-N-methylcyclobutanecarboxamide (18 mg , 52% yield) as a yellow oil.

### Step 8: N-(3-(5-bromo-2-(4-(2-(2-(2-(2-(2-(2,6-dioxopiperidin-3-yl)-1,3-dioxoisoindolin-4-ylamino)ethoxy)ethoxy)ethoxy)ethoxy)phenylamino)pyrimidin-4-ylamino)propyl)-N-methylcyclobutanecarboxamide

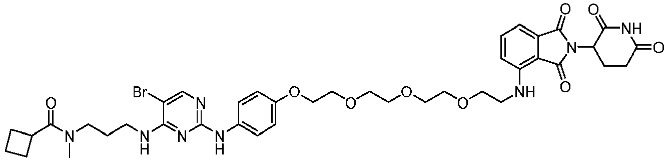

A mixture of N-(3-(2-(4-(2-(2-(2-(2-aminoethoxy)ethoxy)ethoxy)ethoxy) phenylamino)-5-bromopyrimidin-4-ylamino)propyl)-N-methylcyclobutane carboxamide (130 mg, 0.03mmol), 2-(2,6-dioxopiperidin-3-yl)-4-fluoro-2,3-dihydro-1H-isoindole-1,3-dione (8.2 mg, 0.03mmol) and N-ethyl-N-isopropylpropan-2-amine (7.6 mg, 0.06mmol) in dry N,N-dimethylformamide (1 mL) was stirred at 90 °C for 12 h. The reaction mixture was cooled to room temperature, partitioned between ethyl acetate (100 mL) and water (30 mL). The organic phase was washed with brine (30 mL), dried over anhydrous sodium sulfate, and concentrated under reduced pressure. The residue was purified by prep-TLC to afford N-(3-(5-bromo-2-(4-(2-(2-(2-(2-(2-(2,6-dioxopiperidin-3-yl)-1,3-dioxoisoindolin-4-ylamino)ethoxy)ethoxy)ethoxy)ethoxy) phenylamino)pyrimidin-4-ylamino)propyl)-N-methylcyclobutanecarboxamide (10.2 mg, 40% yield) as a yellow solid. LC-MS (ES⁺): m/z = 865.27/867.27 (1:1) [MH]⁺. t_{R} = 2.06 min. ¹H NMR (400 MHz, CD₃OD): δ 1.68-1.77 (m, 3H), 1.89-1.92 (m, 3H), 2.08-2.15 (m, 3H), 2.60-2.79 (m, 7H), 3.28-3.35 (m, 6H), 3.55-3.61 (m, 10H), 3.69-3.72 (m, 2H), 3.96-3.99 (m, 2H), 4.91-4.95 (m, 1H), 6.75-6.78 (m, 2H), 6.91-6.94 (m, 2H), 7.34-7.42 (m, 3H), 7.76 (d, J = 12.8 Hz, 1H).

### 8. 2-((S)-4-(4-chlorophenyl)-2,3,9-trimethyl-6H-thieno [3,2-f][1,2,4]triazolo[4,3-a][1,4]diazepin-6-yl)-N-(4-(2-(2-(2-(2-(2-(2,6-dioxopiperidin-3-yl)-1,3-dioxoisoindolin-4-ylamino)ethoxy)ethoxy)ethoxy)ethoxy)phenyl)acetamide

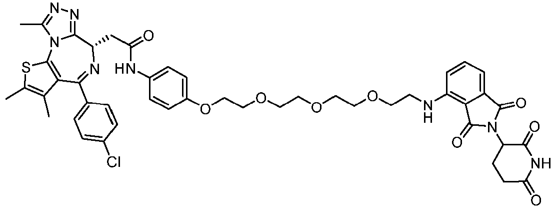

(Compound Structure #14 shown in Table 1)

### Step 1: (2-(2,6-dioxopiperidin-3-yl)- 4-(2-(2-(2-(2-( 4-nitrophenoxy)ethoxy)ethoxy)ethoxy)ethylamino)isoindoline-1,3-dione

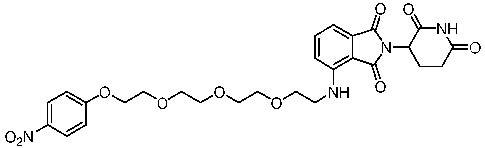

A mixture of 2-(2-(2-(2-(4-nitrophenoxy)ethoxy)ethoxy)ethoxy)ethanamine (128 mg, 0.41 mmol), 2-(2,6-dioxopiperidin-3-yl)-4-fluoro-2,3-dihydro-1H-isoindole-1,3-dione (112.5 mg, 0.41 mmol) and N-ethyl-N-isopropylpropan-2-amine (105 mg, 0.81 mmol) in dry N,N-dimethylformamide (2 mL) was stirred at 90 °C for 12 h. The mixture was cooled to room temperature, poured into water (20 mL) and extracted with ethyl acetate (35 mL×2). The combined organic phases were washed with water (30 mL) and brine (30 mL), dried over anhydrous sodium sulfate, and concentrated under reduced pressure. The crude residue was purified by pre-TLC to afford 2-(2,6-dioxopiperidin-3-yl)-4-(2-(2-(2-(2-(4-nitrophenoxy)ethoxy)ethoxy) ethoxy)ethylamino)isoindoline-1,3-dione (73 mg, 31% yield) as a yellow solid. LC-MS (ES⁺): m/z 571.3 [MH⁻], t_{R} = 2.46 min.

### Step 2: (4-(2-(2-(2-(2-(4-aminophenoxy)ethoxy)ethoxy)ethoxy)ethylamino)-2-(2,6-dioxopiperidin-3-yl)isoindoline-1,3-dione)

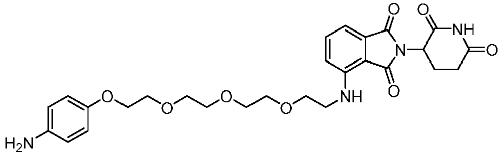

To a suspension of 2-(2,6-dioxopiperidin-3-yl)-4-(2-(2-(2-(2-(4-nitrophenoxy)ethoxy) ethoxy)ethoxy)ethylamino)isoindoline-1,3-dione (73 mg, 0.128 mmol) and iron powder (71.6 mg, 1.28 mmol) in ethanol (2 mL) was added a solution of ammonium chloride (68 mg, 1.26 mmol) in water (0.5 mL) at room temperature, the resulting mixture was stirred at 80 °C for 1 h. After the mixture was cooled to room temperature, the solid precipitate was filtered off and washed with ethyl acetate (10 mL x 2). The filtrate was partitioned between ethyl acetate (60 mL) and water (30 mL). The organic layer was washed with brine (30 mL), dried over anhydrous sodium sulfate, and concentrated under reduced pressure to afford 4-(2-(2-(2-(2-(4-aminophenoxy)ethoxy)ethoxy)ethoxy)ethylamino)-2-(2,6-dioxopiperidin-3-yl)isoindoline-1,3-dione (66.5 mg, crude) as a yellow oil. LC-MS (ES⁺): m/z 541.5 [MH⁺], t_{R} = 1.593 min.

### Step 3: 2-((S)-4-(4-chlorophenyl)-2,3,9-trimethyl-6H-thieno [3,2-f][1,2,4]triazolo[4,3-a][1,4]diazepin-6-yl)-N-(4-(2-(2-(2-(2-(2-(2,6-dioxopiperidin-3-yl)-1,3-dioxoisoindolin-4-ylamino)ethoxy)ethoxy)ethoxy)ethoxy)phenyl)acetamide

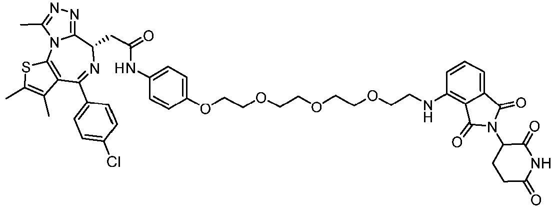

To a stirred solution of 4-(2-(2-(2-(2-(4-aminophenoxy)ethoxy)ethoxy)ethoxy)ethylamino)-2-(2,6-dioxopiperidin-3-yl)isoindoline-1,3-dione (58.4 mg, 0.11 mmol), (S)-2-(4-(4-chlorophenyl)-2,3,9-trimethyl-6H-thieno[3,2-f][1,2,4]triazolo[4,3-a][1,4]diazepin-6-yl)acetic acid (43.3 mg, 0.11 mmol) and N-ethyl-N-isopropylpropan-2-amine(41.8 mg, 0.32 mmol) in dry N,N-dimethylformamide (1 mL) was added (2-(7-aza-1H-benzotriazole-1-yl)- 1,1,3,3-tetramethyluroniumhexafluorophosphate) (82 mg, 0.21mmol) at 0 °C. The resulting mixture was allowed to warm up to room temperature and stirred at room temperature for 20 min. The mixture was poured into water (25 mL), extracted with ethyl acetate (35 ml×2). The combined organic phases were washed with water (20 mL) and brine (30 mL), dried over anhydrous sodium sulfate, and concentrated under reduced pressure. The crude residue was purified by prep-TLC to afford 2-((S)-4-(4-chlorophenyl)-2,3,9-trimethyl-6H-thieno[3,2-f][1,2,4]triazolo[4,3-a][1,4]diazepin-6-yl)-N-(4-(2-(2-(2-(2-(2-(2,6-dioxopiperidin-3-yl)-1,3-dioxoisoindolin-4-ylamino)ethoxy)ethoxy)ethoxy)ethoxy)phenyl)acetamide (52 mg, 52% yield) as a yellow solid. LC-MS (ES⁺): m/z 923.29/925.29 (3:1) [MH⁺], t_{R} = 2.689 min. ¹H NMR (400 MHz, CDCl₃): δ 1.67 (s, 3H), 2.05-2.12 (m, 1H), 2.40 (s, 3H), 2.65-2.85 (m, 6H), 3.41-3.54 (m, 4H), 3.65-3.74 (m, 10H), 3.81-3.85 (m, 2H), 4.06-4.11 (m, 2H), 4.63-4.69 (m, 1H), 4.85-4.93 (m, 1H), 6.38-6.55 (m, 1H), 6.83 (d, *J =* 8.8 Hz, 2H), 6.92 (d, *J =* 8.8 Hz, 1H), 7.09 (d, *J =* 7.2 Hz, 1H), 7.33 (d, *J =* 8.4 Hz, 2H), 7.39-7.51 (m, 5H), 8.59 (d, *J =* 5.2 Hz, 1H), 8.77 (d, *J =* 3.2 Hz, 1H).

### 9. (Z)-4-(4-((3-(2-(2-(2-(2-(2,6-dioxopiperidin-3-yl)-1,3- dioxoisoindolin-4-ylamino)ethoxy)ethoxy)ethyl)-2,4-dioxothiazolidin-5-ylidene)methyl)-2-methoxyphenoxy)-3-(trifluoromethyl)benzonitrile (reference)

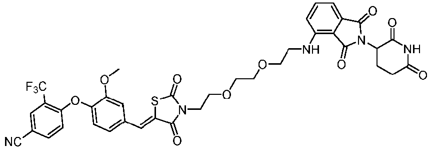

(Compound Structure #22 shown in Table 1)

### Step 1: (Z)-2-(2-(2-(5-(4-(4-cyano-2-(trifluoromethyl)phenoxy)-3-methoxybenzylidene)-2,4-dioxothiazolidin-3-yl)ethoxy)ethoxy)ethyl 4-methylbenzenesulfonate)

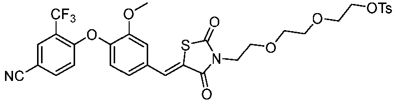

A mixture of (Z)-4-(4-((2,4-dioxothiazolidin-5-ylidene)methyl)-2-methoxyphenoxy)-3-(trifluoromethyl)benzonitrile (1.0 g, 2.3 mmol), potassium carbonate (1.0 g, 6.9 mmol) and 2,2'-(ethane-1,2-diylbis(oxy))bis(ethane-2,1-diyl) bis(4-methylbenzenesulfonate) (1.3 g, 2.7 mmol) in N,N-dimethylformamide (10 mL) was stirred at 80 °C for 16 h. The reaction mixture was cooled to room temperature, quenched with water (10 mL), and extracted with ethyl acetate (40 mL x 3). The combined organic phases were washed with water (50 mL) and brine (50 mL), dried over sodium sulfate, and evaporated under reduced pressure. The crude residue was purified by silica gel flash column chromatography (eluted with 10-30% ethyl acetate in hexane) to afford (Z)-2-(2-(2-(5-(4-(4-cyano-2-(trifluoromethyl)phenoxy)-3-methoxybenzylidene)-2,4-dioxothiazolidin-3-yl)ethoxy)ethoxy)ethyl 4-methylbenzenesulfonate (1.0 g, 61% yield) as a light yellow solid.

### Step 2: (Z)-4-(4-((3-(2-(2-(2-azidoethoxy)ethoxy)ethyl) -2,4-dioxothiazolidin-5-ylidene)methyl)-2-methoxyphenoxy)-3-(trifluoromethyl)benzonitrile

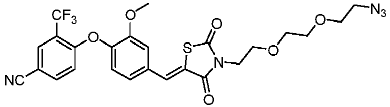

A mixture of (Z)-2-(2-(2-(5-(4-(4-cyano-2-(trifluoromethyl)phenoxy)-3-methoxybenzylidene) -2,4-dioxothiazolidin-3-yl)ethoxy)ethoxy)ethyl 4-methylbenzenesulfonate (1.0 g, 1.4 mmol) and sodium azide (185 mg, 2.8 mmol) in ethanol (20 mL) was refluxed for 16 h. The reaction mixture was cooled to room temperature and partitioned between ethyl acetate (100 mL) and water (20 mL). The organic layer was washed with brine (30 ml), dried over anhydrous sodium sulfate and concentrated under reduced pressure to afford (Z)-4-(4-((3-(2-(2-(2-azidoethoxy)ethoxy)ethyl)-2,4-dioxothiazolidin-5-ylidene)methyl)-2-methoxyphenoxy)-3-(trifluoromethyl)benzonitrile (130 mg, crude) as a light yellow oil, which was used in next step without further purification.

### Step 3: (Z)-4-(4-((3-(2-(2-(2-aminoethoxy)ethoxy)ethyl)-2,4- dioxothiazolidin-5-ylidene)methyl)-2-methoxyphenoxy)-3-(trifluoromethyl)benzonitrile

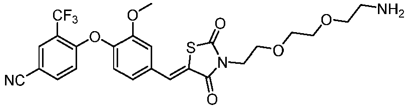

A mixture of the above (Z)-4-(4-((3-(2-(2-(2-azidoethoxy)ethoxy)ethyl)-2,4-dioxothiazolidin-5-ylidene)methyl)-2-methoxyphenoxy)-3-(trifluoromethyl)benzonitrile) (130 mg, crude), triphenylphosphine (100 mg, 0.34 mmol) in water (0.2 mL) and tetrahydrofuran (20 mL) was stirred at room temperature for 14 h. The mixture was concentrated under reduced pressure. The crude residue was purified by silica gel flash column chromatography (eluted with 3-5% methanol in dichloromethane) to give (Z)-4-(4-((3-(2-(2-(2-aminoethoxy) ethoxy)ethyl)-2,4-dioxothiazolidin-5-ylidene)methyl)-2-methoxyphenoxy)-3-(trifluoromethyl)benzonitrile (60 mg, 8% yield over two steps) as a yellow oil. LC-MS (ES⁺): m/z 552.1 [MH⁺], t_{R} = 2.15 min.

### Step 4: (Z)-4-(4-((3-(2-(2-(2-(2-(2,6-dioxopiperidin-3-yl)-1,3- dioxoisoindolin-4-ylamino)ethoxy)ethoxy)ethyl)-2,4-dioxothiazolidin-5-ylidene)methyl)-2-methoxyphenoxy)-3-(trifluoromethyl)benzonitrile

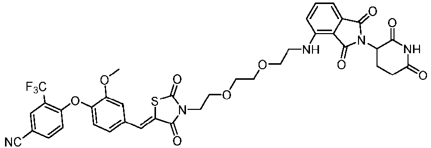

A mixture of (Z)-4-(4-((3-(2-(2-(2-aminoethoxy)ethoxy)ethyl)-2,4-dioxothiazolidin-5-ylidene) methyl)-2-methoxyphenoxy)-3-(trifluoromethyl)benzonitrile) (60 mg, 0.10 mmol), 2-(2,6-dioxopiperidin-3-yl)-4-fluoro-2,3-dihydro-1H-isoindole-1,3-dione (30 mg, 0.13 mmol) and N-ethyl-N-isopropylpropan-2-amine (50 mg, 0.39 mmol) in 1-methylpyrrolidin-2-one (1 mL) was stirred at 90 °C for 16 h. The reaction mixture was cooled to room temperature, quenched with water (5 mL), and extracted with ethyl acetate (20 mL x 3). The combined organic layers were washed with water (10 mL x 2) and brine (10 mL), dried over anhydrous sodium sulfate, and concentrated under reduced pressure. The crude residue was purified by prep-TLC to afford (Z)-4-(4-((3-(2-(2-(2-(2-(2,6-dioxopiperidin-3-yl)-1,3-dioxoisoindolin-4-ylamino)ethoxy)ethoxy)ethyl)-2,4-dioxothiazolidin-5-ylidene)methyl)-2-methoxyphenoxy)-3-(trifluoromethyl)benzonitrile (9.5 mg, 11.8% yield) as a yellow solid. LC-MS (ES⁺): m/z 808.19 [MH⁺], t_{R} = 3.022 min. ¹H NMR (400 MHz, CDCl₃): δ 2.12-2.16 (m, 1H), 2.73-2.91 (m, 3H), 3.42 (s, 2H), 3.67-3.80 (m, 11H), 3.99 (s, 2H), 4.91-4.95 (m, 1H), 6.51 (s, 1H), 6.76-6.86 (m, 2H), 7.02-7.19 (m, 4H), 7.43 (t*, J =* 7.6 Hz, 1H), 7.68 (d, *J =* 8.0 Hz, 1H), 7.85-8.12 (m, 3H).

### 10. 4-(3-(4-(3-(2-(2-(2-((2-(2,6-dioxopiperidin-3-yl)-1,3-dioxoisoindolin-4-yl)amino)ethoxy)ethoxy)ethoxy)propoxy)phenyl)-4,4-dimethyl-5-oxo-2-thioxoimidazolidin-1-yl)-2-(trifluoromethyl)benzonitrile

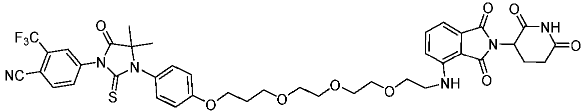

(Compound Structure #1 shown in Table 1)

### Step 1: 1,1,1,16-tetraphenyl-2,5,8,11,15-pentaoxahexadecane

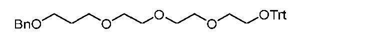

To a solution of 2-(2-(2-(trityloxy)ethoxy)ethoxy)ethanol (7 g, 17.7 mmol) in N,N-dimethylformamide (50 mL) was slowly added sodium hydride (60% in mineral oil, 707 mg, 17.7 mmol) at 0 °C. After the mixture was stirred at rt for 30 min, 3-(benzyloxy)propyl 4-methylbenzenesulfonate (5.8 g 18.0 mmol) was added in one portion at 0°C, the resulting mixture was allowed to stir at 70 °C overnight. After the mixture was cooled to rt, it was carefully quenched with water (40 mL), extracted with ethyl acetate (60 mL×3). The combined organic phases were washed with brine (80 mL), dried over anhydrous sodium sulfate, and concentrated under reduced pressure. The crude residue was purified by silica gel flash chromatography (eluted with 5-10% ethyl acetate in hexane) to afford 1,1,1,16-tetraphenyl-2,5,8,11,15-pentaoxahexadecane (4.8 g, 50% yield) as a colorless oil. ¹H NMR (400 MHz, CDCl₃): δ 1.85-1.92 (m, 2H), 3.23 (t, *J* = 5.2 Hz, 2H), 3.53-3.59 (m, 6H), 3.64-3.68 (m, 8H), 4.47 (s, 2H), 7.19-7.33 (m, 15H), 7.45-7.47 (m, 5H).

### Step 2: 1-phenyl-2,6,9,12-tetraoxatetradecan-14-ol

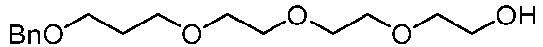

To a solution of 1,1,1,16-tetraphenyl-2,5,8,11,15-pentaoxahexadecane (4.8 g 8.8 mmol) in methylene dichloride (10 mL) and methanol (10 mL) was added aqueous hydrochloric acid (37%, 2.5 mL) at 0 °C. The reaction mixture was stirred at rt for 2 h. The reaction mixture was poured into water (30 mL), and extracted with dichloromethane (20 mL×3). The combined organic phases were washed with aqueous sodium bicarbonate (1N, 50 mL), water (30 mL), brine, dried over anhydrous Na₂SO₄, and concentrated under reduced pressure. The crude residue was purified by silica gel flash column chromatography (eluted with 20-40% ethyl acetate in hexane) to afford 1-phenyl-2,6,9,12-tetraoxatetradecan-14-ol (1.9 g, 73% yield) as a colorless oil.

### Step 3: 1-phenyl-2,6,9,12-tetraoxatetradecan-14-yl 4-methylbenzenesulfonate

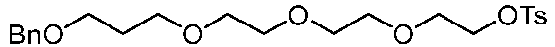

A mixture of 1-phenyl-2,7,10,13-tetraoxapentadecan-15-ol (1.9 g, 6.3 mmol), triethylamine (1.3 mL, 9.5 mmol), N,N-dimethylpyridin-4-amine (75 mg, 0.63 mmol) and 4-methylbenzene-1-sulfonyl chloride (1.45 g, 7.65 mmol) in dichloromethane (20 mL) was stirred at rt for 3 h. Water (20 mL) was added to quench the reaction, and the product was extracted with dichloromethane (40 mL x 3). The combined organic phases were washed with brine (50 mL), dried over sodium sulfate, and evaporated under reduced pressure. The crude residue was purified by silica gel flash column chromatography (eluted with 10-30% ethyl acetate in hexane) to afford 1-phenyl-2,6,9,12-tetraoxatetradecan-14-yl 4-methylbenzenesulfonate (2.2 g, 78% yield) as a colorless oil. ¹H NMR (400 MHz, CDCl₃): δ 1.87-1.92 (m, 2H), 2.43 (s, 3H), 3.54-3.60 (m, 12H), 3.67 (t, *J =* 5.2 Hz, 2H), 4.15 (t, *J* = 5.0 Hz, 2H), 4.48 (s, 2H), 7.27-7.33 (m, 7H), 7.79 (d, *J =* 8.4 Hz, 2H).

### Step 4: 14-azido-1-phenyl-2,6,9,12-tetraoxatetradecane

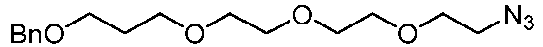

A mixture of 1-phenyl-2,6,9,12-tetraoxatetradecan-14-yl 4-methylbenzenesulfonate (2.2 g, 4.9 mmol) and sodium azide (420 mg, 6.3 mmol) in ethanol (10 mL) was refluxed for 5 h. The reaction mixture was cooled to rt, poured into water (10 mL), and extracted with dichloromethane (50 mL x 3). The combined organic layers were washed with brine (50 mL), dried over anhydrous sodium sulfate, and concentrated under reduced pressure to give 14-azido-1-phenyl-2,6,9,12-tetraoxatetradecane (1.4 g, crude) as a colorless oil, which was used in next step without further purification.

### Step 5: tert-butyl (1-phenyl-2,6,9,12-tetraoxatetradecan-14-yl)carbamate

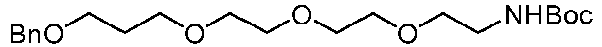

A mixture of the above 14-azido-1-phenyl-2,6,9,12-tetraoxatetradecane (1.4 g, crude) and triphenylphosphine (1.7 g, 6.5 mmol) in tetrahydrofuran (15 mL) and water (0.5 mL) was stirred at rt overnight under nitrogen atmosphere. To the reaction mixture were added triethylamine (0.9 mL, 6.5 mmol) and di-*tert*-butyl dicarbonate (1.1 g, 5.2 mmol) at 0 °C. The resulting mixture was allowed to warm up to rt and stir at rt for 2 h. The volatiles were evaporated under reduced pressure, and the residue was partitioned between dichloromethane (100 mL) and water (50 mL). The organic phase was washed with brine (30 mL), dried over anhydrous sodium sulfate, and concentrated under reduced pressure. The crude residue was purified by silica gel flash chromatography (eluted with 30-50% ethyl acetate in hexane) to afford *tert*-butyl (1-phenyl-2,6,9,12-tetraoxatetradecan-14-yl)carbamate (1.2 g, 50% yield over two steps) as a colorless oil.

### Step 6: tert-butyl 2-(2-(2-(3-hydroxypropoxy)ethoxy)ethoxy)ethylcarbamate

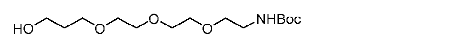

A mixture of I*tert*-butyl (1-phenyl-2,5,9,12-tetraoxatetradecan-14-yl) carbamate (1.2 g, 3 mmol) and palladium on carbon (10%, 200mg() in ethanol (50mL) was stirred at rt under hydrogen atmosphere (hydrogen ballon). Palladium on carbon was removed by filtration and washed with ethanol (20mL). The filtrate was concentrated under reduced pressure to afford *tert*-butyl 2-(2-(2-(3-hydroxypropoxy)ethoxy)ethylcarbamate (900 mg, crude) as a colorless oil, which was used in next step without further purification.

### Step 7: 2,2-dimethyl-4-oxo-3,8,11,14-tetraoxa-5-azaheptadecan-17-yl 4-methylbenzesulfonate

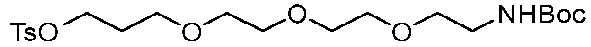

A mixture of the above *tert*-butyl 2-(2-(2-(3-hydroxypropoxy)ethoxy)ethoxy) ethylcarbamate (900 mg, crude), triethylamine (0.6 mL, 4.35 mmol), N,N-dimethylpyridin-4-amine (16 mg, 0.14 mmol) and 4-methylbenzene-1-sulfonyl chloride (660 mg, 3.5 mmol) in anhydrous dichloromethane (15 mL) was stirred at rt for 3 h. Water (20 mL) was added to quench the reaction and the product was extracted with dichloromethane (50 mL x 3). The combined organic phases were washed with brine (50 mL), dried over anhydrous sodium sulfate, and evaporated under reduced pressure. The crude residue was purified by silica gel flash column chromatography (eluted with 20-30% ethyl acetate in hexane) to afford 2,2-dimethyl-4-oxo-3,8,11,14-tetraoxa-5-azaheptadecan-17-yl 4-methylbenzenesulfonate (650 mg, 77% yield) as a light yellow oil. ¹H NMR (400 MHz, CDCl₃): δ 1.44 (s, 9H), 1.88-1.95 (m, 2H), 2.45 (s, 3H), 3.29-3.33 (m, 2H), 3.48-3.61 (m, 12H), 4.09-4.15 (m, 2H), 5.04 (brs, 1H), 7.34 (d, *J =* 8.0 Hz, 2H), 7.79 (d, *J =* 8.0 Hz, 2H).

### Step 8: tert-butyl (2-(2-(2-(3-(4-(3-(4-cyano-3-(trifluoromethyl)phenyl)-5,5-dimethyl-4-oxo-2-thioxoimidazolidin-1-yl)phenoxy)propoxy)ethoxy)ethoxy)ethyl)carbamate

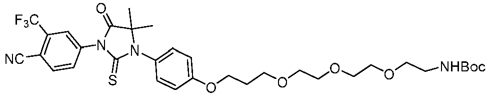

A mixture of 2,2-dimethyl-4-oxo-3,8,11,14-tetraoxa-5-azaheptadecan-17-yl 4-methylbenzenesulfonate (115 mg, 0.25 mmol), potassium carbonate (69 mg, 0.50 mmol) and 4-(3-(4-hydroxyphenyl)-4,4-dimethyl-5-oxo-2-thioxoimidazolidin-1-yl)-2-(trifluoromethyl)benzonitrile (100 mg, 0.25 mmol) in acetonitrile (5 mL) was stirred at 80 °C for 16 h. The reaction mixture was cooled to room temperature, quenched with water (30 mL), and extracted with ethyl acetate (30 mL x 3). The combined organic phases were washed with water (30 mL) and brine (30 mL), dried over magnesium sulfate, and evaporated under reduced pressure. The crude residue was purified by silica gel flash column chromatograph (eluted with 10-30% ethyl acetate in hexane) to afford tert-butyl 2-(2-(2-(3-(4-(3-(4-cyano-3-(trifluoromethyl)phenyl)-5,5-dimethyl-4-oxo-2-thioxoimidazolidin-1-yl)phenoxy)propoxy)ethoxy)ethoxy) ethylcarbamate (150 mg, 82% yield) as a yellow oil. LC-MS (ES⁺): m/z 695.40 [MH⁺], t_{R} = 2.79 min.

### Step 9: 4-(3-(4-(3-(2-(2-(2-aminoethoxy)ethoxy)ethoxy)propoxy)phenyl)-4,4-dimethyl-5-oxo-2-thioxoimidazolidin-1-yl)-2-(trifluoromethyl)benzonitrile

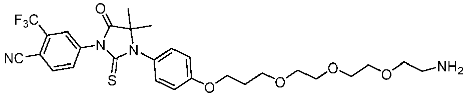

A mixture of *tert*-butyl 2-(2-(2-(3-(4-(3-(4-cyano-3-(trifluoromethyl)phenyl)-5,5-dimethyl-4-oxo-2-thioxoimidazolidin-1-yl)phenoxy)propoxy)ethoxy)ethoxy) ethylcarbamate (150 mg, 0.21 mmol) in anhydrous dichloromethane (2 mL) and 2,2,2-trifluoroacetic acid (1 mL) was stirred at rt for 1h. The volatiles were evaporated under reduced pressure, the residue was poured into aqueous sodium bicarbonate (1N, 20 mL), and extracted with dichloromethane (50 mL x 3). The combined organic phases were washed with brine (50 mL), dried over anhydrous sodium sulfate, and concentrated under reduced pressure to give 4-(3-(4-(3-(2-(2-(2-aminoethoxy)ethoxy)ethoxy)propoxy)phenyl)-4,4-dimethyl-5-oxo-2-thioxoimidazolidin-1-yl)-2-(trifluoromethyl)benzonitrile (115 mg, crude) as a brown oil, which was used in next step without further purification.

### Step 10: 4-(3-(4-(3-(2-(2-(2-((2-(2,6-dioxopiperidin-3-yl)-1,3-dioxoisoindolin-4-yl)amino)ethoxy)ethoxy)ethoxy)propoxy)phenyl)-4,4-dimethyl-5-oxo-2-thioxoimidazolidin-1-yl)-2-(trifluoromethyl)benzonitrile

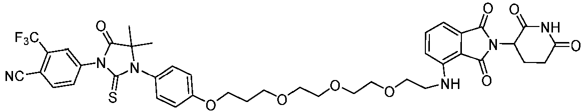

A solution of the above 4-(3-(4-(3-(2-(2-(2-aminoethoxy)ethoxy)ethoxy) propoxy)phenyl)-4,4-dimethyl-5-oxo-2-thioxoimidazolidin-1-yl)-2-(trifluoromethyl)benzonitrile (115 mg, crude), 2-(2,6-dioxopiperidin-3-yl)-4-fluoro-2,3-dihydro-1H-isoindole-1,3-dione (41 mg, 0.15 mmol) and N-ethyl-N-isopropylpropan-2-amine (58 mg, 0.44 mmol) in N,N-dimethylformamide (2 mL) was stirred at 90 °C for 16 h. The reaction mixture was cooled to rt, quenched with water (3 mL), and extracted with ethyl acetate (30 mL x 3). The combined organic layers were washed with water (30 mL x 2) and brine (20 mL), dried over anhydrous sodium sulfate, and concentrated under reduced pressure. The crude residue was purified by prep-TLC to afford 4-(3-(4-(3-(2-(2-(2-((2-(2,6-dioxopiperidin-3-yl)-1,3-dioxoisoindolin-4-yl)amino)ethoxy)ethoxy)ethoxy)propoxy)phenyl)-4,4-dimethyl-5-oxo-2-thioxoimidazolidin-1-yl)-2-(trifluoromethyl)benzonitrile (34.5 mg, 27% yield) as a yellow solid. LC-MS (ES⁺): m/z 851.25 [MH⁺], t_{R} = 2.652 min. ¹H NMR (400 MHz, CD₃OD): δ 1.57 (s, 6H), 2.07-2.11 (m, 3H), 2.70-2.90 (m, 3H), 3.46-3.72 (m, 14H), 4.10 (t, *J* = 6.2 Hz, 2H), 4.88-4.92 (m, 1H), 6.48-6.49 (m, 1H), 6.91-7.26 (m, 6H), 7.49 (t, *J =* 7.8 Hz, 1H), 7.83-7.85(m, 1H), 7.97-8.02 (m, 3H).

### 11. 4-{[5-(3-{[2-(2,6-dioxopiperidin-3-yl)-1,3-dioxo-2,3-dihydro-1H-isoindol-4-yl]amino}propoxy)pentyl]oxy}-N-[trans-3-(3-chloro-4-cyanophenoxy)-2,2,4,4-tetramethylcyclobutyl]benzamide

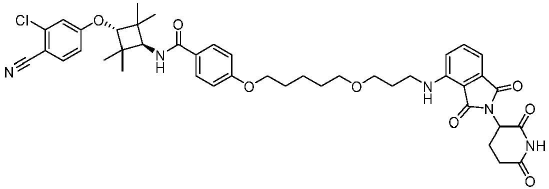

### Step 1: 3-[(5-hydroxypentyl)oxy]propanenitrile

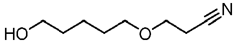

Pentane-1,5-diol (2.98 g, 28.6 mmol) was added to a suspension of sodium hydride (60% dispersion in mineral oil, 820 mg, 34.2 mmol) in THF (50 mL). After the mixture was stirred at rt for 20 min, it was cooled to 0 °C, and acrylonitrile (1.20 g, 22.8 mmol) was added dropwise. The resulting mixture was stirred at rt for 10 h. Part of the solvent was removed under vacuum and the residue was poured into water. The mixture was extracted with DCM (3x). The organic layer was filtered through a Biotage Universal Phase Separator and concentrated *in vacuo.* The crude material was purified by silica gel chromatography on a Teledyne Combiflash ISCO eluting with MeOH/DCM (0:100 to 3:97) to yield 3-[(5-hydroxypentyl)oxy]propanenitrile (635 mg, 18% yield). ¹H NMR (400 MHz, CDCl₃) δ 3.60-3.73 (m, 4H), 3.45-3.55 (m, 2H), 2.60 (dt, J = 4.1, 6.4 Hz, 2H), 2.06 (d, J = 3.9 Hz, 1H), 1.57-1.69 (m, 4H), 1.43-1.50 (m, 2H).

### Step 2: tert-butyl N-{3-[(5-hydroxypentyl)oxy]propyl}carbamate

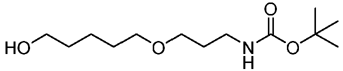

To a solution of 3-[(5-hydroxypentyl)oxy]propanenitrile (400 mg, 2.54 mmol) in MeOH (12 mL) and H₂O (2.0 mL) was added Nickel(II) chloride (393 mg, 3.04 mmol), followed by sodium borohydride (360 mg, 9.52 mmol) portionwise. The mixture was stirred at rt for 3 h, then quenched with MeOH (12 mL). The mixture was filtered through celite and washed with MeOH. The filtrate was concentrated *in vacuo.* To a solution of the above crude product in THF (5 mL) were added 6 M aq NaOH (0.5 mL) and di-tert-butyl dicarbonate (831 mg, 3.81 mmol), the resulting mixture was stirred at rt for 3 h, then concentrated *in vacuo.* The crude material was purified by silica gel chromatography on a Teledyne Combiflash ISCO eluting with MeOH/DCM (0:100 to 4:96) to yield tert-butyl N-{3-[(5-hydroxypentyl)oxy]propyl}carbamate (366 mg, 55% yield).

¹H NMR (400 MHz, CDCl₃) δ 4.91 (br. s., 1H), 3.66 (br. s., 2H), 3.49 (t, J = 5.9 Hz, 2H), 3.43 (t, J = 6.3 Hz, 2H), 3.24 (q, J = 5.9 Hz, 2H), 1.75 (quin, J = 6.2 Hz, 2H), 1.57-1.65 (m, 5H), 1.41-1.52 (m, 11H).

### Step 3: tert-butyl N-[3-({5-[(4-methylbenzenesulfonyl)oxy]pentyl}oxy)propyl]carbamate

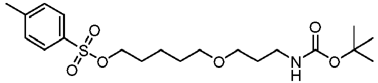

To a solution of *tert-butyl* (3-((5-hydroxypentyl)oxy)propyl)carbamate (300 mg, 3.88 mmol) in DCM (10 mL) were added DIPEA (599.3 µL, 3.44 mmol), tosyl chloride (262.3 mg, 1.38 mmol) and 4-dimethylaminopyridine (14.0 mg, 0.115 mmol). The resulting mixture was stirred at rt for 20 h. The reaction was quenched with a semi-saturated sodium bicarbonate, extracted with DCM (2x), filtered through a Biotage Universal Phase Separator, and concentrated in vacuo. The crude material was purified by silica gel chromatography on a Teledyne Combiflash ISCO eluting with EtOAc/Heptane (0:100 to 30:70) to yield *tert-butyl* N-[3-({5-[(4-methylbenzenesulfonyl)oxy]pentyl}oxy)propyl]carbamate (914 mg, 26% yield). ¹H NMR (400 MHz, CDCl₃) δ 7.78 (d, J = 8.2 Hz, 2H), 7.34 (d, J = 8.2 Hz, 2H), 4.02 (t, J = 6.5 Hz, 2H), 3.44 (t, J = 6.1 Hz, 2H), 3.35 (t, J = 6.3 Hz, 2H), 3.19 (q, J = 5.9 Hz, 2H), 2.44 (s, 3H), 1.64-1.74 (m, 5H), 1.49-1.54 (m, 2H), 1.42 (s, 9H), 1.33-1.40 (m, 2H). LC-MS (ES⁺): *m*/*z* 438.19 [MNa⁺], *t_{R} =* 2.65 min.

### Step 4: methyl 4-{[5-(3-{[(tert-butoxy)carbonyl]amino}propoxy)pentyl]oxy}benzoate

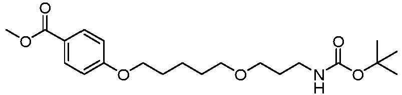

A mixture of *tert*-butyl N-[3-({5-[(4-methylbenzenesulfonyl)oxy]pentyl}oxy)propyl]carbamate (340 mg, 0.82 mmol), methyl 4-hydroxybenzoate (117 mg, 0.77 mmol), potassium carbonate (203 mg, 1.47 mmol) in MeCN (10 mL) were stirred at 80 °C for 24 h. The reaction mixture was diluted with EtOAc, washed with semi-saturated sodium bicarbonate solution (1x), water (2x), brine (1x) and then filtered through a Biotage Universal Phase Separator. The filtrate was concentrated in vacuo, and the residue was purified by silica gel chromatography on a Teledyne Combiflash ISCO eluting with EtOAc/Heptane (0: 100 to 50:50) to yield methyl 4-{[5-(3-{[(tert-butoxy)carbonyl]amino}propoxy)pentyl]oxy}benzoate (300 mg, 93% yield). LC-MS (ES⁺): *m*/*z* 418.21 [MNa⁺], *t_{R}* = 2.74 min.

### Step 5: 4-{[5-(3-{[(tert-butoxy)carbonyl]amino}propoxy)pentyl]oxy}benzoic acid

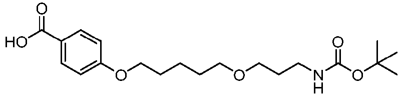

To a solution of 4-{[5-(3-{[(tert-butoxy)carbonyl]amino}propoxy)pentyl]oxy}benzoate (150 mg, 0.38 mmol) in 1:1:1 THF/Water/MeOH (6.0 mL, v/v/v) was added lithium hydroxide (81.6 mg, 3.41 mmol). The resulting mixture was stirred overnight at rt, then acidified to a pH 2-3 with 6N aqueous HCl. The mixture was concentrated *in vacuo* to remove most solvents, then diluted with EtOAc, washed with water (2 x), brine (2 x), filtered through a Biotage Universal Phase Separator, and concentrated in vacuo. The crude product was carried onto next step without further purification (123 mg). LC-MS (ES⁺): *m*/*z* 404.20 [MNa⁺], *t_{R} =* 2.40 min.

### Step 6: tert-butyl N-(3-{[5-(4-{[trans-3-(3-chloro-4-cyanophenoxy)-2,2,4,4-tetramethylcyclobutyl]carbamoyl}phenoxy)pentyl]oxy}propyl)carbamate

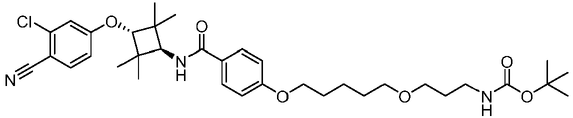

To a solution of 4-{[5-(3-{[(tert-butoxy)carbonyl]amino}propoxy)pentyl]oxy}benzoic acid (124 mg, 0.322 mmol), 2-chloro-4-(trans-3-amino-2,2,4,4-tetramethylcyclobutoxy)benzonitrile (89.8 mg, 0.322 mmol) in DMF (5 mL) were added DIPEA (112 µL, 0.65 mmol) and TBTU (155 mg, 0.48 mmol). The resulting mixture was stirred at rt for 1 h, then diluted with EtOAc, washed with water (3 x), brine (1 x), filtered through a Biotage Universal Phase Separator and concentrated *in vacuo.* The residue was purified by silica gel chromatography on a Teledyne Combiflash ISCO eluting with MeOH/DCM (0:100 to 5:95) to yield *tert-butyl* N-(3-{[5-(4-{[trans-3-(3-chloro-4-cyanophenoxy)-2,2,4,4-tetramethylcyclobutyl] carbamoyl}phenoxy)pentyl]oxy}propyl)carbamate (169 mg, 82% yield). LC-MS (ES⁺): m/z 643.32/645.31 (3:1) [MH⁺], *t_{R}* = 3.04 min.

### 12. 4-{[5-(3-aminopropoxy)pentyl]oxy}-N-[trans-3-(3-chloro-4-cyanophenoxy)-2,2,4,4-tetramethylcyclobutyl]benzamide

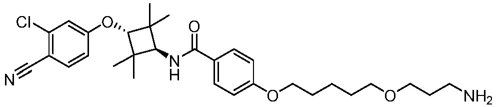

To a solution of *tert-butyl* N-(3-{[5-(4-{[trans-3-(3-chloro-4-cyanophenoxy)-2,2,4,4-tetramethylcyclobutyl]carbamoyl}phenoxy)pentyl]oxy}propyl)carbamate (124 mg, 0.192 mmol) in DCM (5 mL) was added trifluoroacetic acid (372 µL, 4.86 mmol) and heated at 45 °C for 1 h until completion. The reaction was then concentrated *in vacuo* to a solid and carried onto next step without further purification (104 mg, 99% yield). LC-MS (ES⁺): *m*/*z* 543.27/545.26 (3:1) [MH⁺], *t_{R}* = 2.26 min.

### 13. 4-{[5-(3-{[2-(2,6-dioxopiperidin-3-yl)-1,3-dioxo-2,3-dihydro-1H-isoindol-4-yl]amino}propoxy)pentyl]oxy}-N-[trans-3-(3-chloro-4-cyanophenoxy)-2,2,4,4-tetramethylcyclobutyl]benzamide

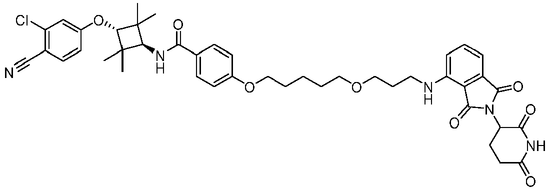

(Compound Structure #11 shown in Table 1)

To a solution of 4-{[5-(3-aminopropoxy)pentyl]oxy}-N-[trans-3-(3-chloro-4-cyanophenoxy)-2,2,4,4-tetramethylcyclobutyl]benzamide (30.0 mg, 0.0553 mmol) in 1,4-dioxane (2 mL) were added diisopropylethylamine (384 µL, 2.21 mmol), 2-(2,6-dioxopiperidin-3-yl)-4-fluoro-2,3-dihydro-1H-isoindole-1,3-dione (18.3 mg, 0.0664 mmol). The resulting mixture was refluxed for 16 h, then diluted with EtOAc, washed with semi-saturated brine solution (2 x), filtered through a Biotage Universal Phase Separator and concentrated *in vacuo.* The residue was purified by silica gel chromatography on a Teledyne Combiflash ISCO eluting with MeOH/DCM (0:100 to 7:93) to yield 4-{[5-(3-{[2-(2,6-dioxopiperidin-3-yl)-1,3-dioxo-2,3-dihydro-1H-isoindol-4-yl]amino}propoxy)pentyl]oxy}-N-[trans-3-(3-chloro-4-cyanophenoxy)-2,2,4,4-tetramethylcyclobutyl]benzamide (12 mg, 28% yield). LC-MS (ES⁺): *m*/*z* 799.31/801.31 (3:1) [MH⁺], *t_{R} =* 2.97 min. ¹H NMR (400 MHz, CDCl₃) δ 8.03 (s, 1H), 7.72 (d, J = 9.0 Hz, 2H), 7.58 (d, J = 8.6 Hz, 1H), 7.48 (dd, J = 7.2, 8.4 Hz, 1H), 7.07 (d, J = 7.0 Hz, 1H), 6.98 (d, J = 2.3 Hz, 1H), 6.89-6.96 (m, 3H), 6.82 (dd, J = 2.5, 8.8 Hz, 1H), 6.18 (d, J = 8.2 Hz, 1H), 4.89 (dd, J = 5.1, 12.1 Hz, 1H), 4.16 (d, J = 7.8 Hz, 1H), 4.06 (s, 1H), 4.02 (t, J = 6.7 Hz, 2H), 3.56 (t, J = 5.9 Hz, 2H), 3.50 (s, 2H), 3.46-3.48 (m, 1H), 3.41 (t, J = 6.5 Hz, 2H), 2.82-2.90 (m, 1H), 2.76-2.81 (m, 1H), 2.67-2.75 (m, 1H), 2.07-2.14 (m, 1H), 1.94 (quin, J = 6.1 Hz, 2H), 1.82-1.87 (m, 2H), 1.67-1.73 (m, 2H), 1.53-1.59 (m, 2H), 1.28 (s, 6H), 1.20-1.25 (m, 6H).

### C. Protein Degradation Bioassays:

The following bioassays were performed to evaluate the level of protein degradation observed in various cell types using representative compounds disclosed herein.

In each bioassay, cells were treated with varying amounts of compounds encompassed by the present disclosure, as shown in Table 1. The degradation of the following proteins were evaluated in this study: TANK-binding kinase 1 (TBK1), estrogen receptor α (ERα), bromodomain-containing protein 4 (BRD4), androgen receptor (AR), and c-Myc.

### 1. TBK1 Western Protocol

Panc02.13 cells were purchased from ATCC and cultured in RPMI-1640 (Gibco), supplemented with 15% FBS (ATCC) and 10Units/mL human recombinant insulin (Gibco). DMSO control and compound treatments (0.1µM, 0.3µM, and 1µM) were carried out in 12-well plates for 16h. TLR3 agonist Poly I:C (Invivogen; tlrl-pic) was added for the final 3h. Cells were harvested, and lysed in RIPA buffer (50mM Tris pH8, 150mM NaCl, 1% Tx-100, 0.1% SDS, 0.5% sodium deoxycholate) supplemented with protease and phosphatase inhibitors. Lysates were clarified at 16,000g for 10 minutes, and supernatants were separated by SDS-PAGE. Immunoblotting was performed using standard protocols. The antibodies used were TBK1 (Cell Signaling #3504), pIRF3 (abcam #ab76493), and GAPDH (Cell Signaling #5174). Bands were quantified using a Biorad ChemiDoc MP imaging system.

### 2. ERRα Western Protocol

NAMALWA cells (ATCC) were cultured in RPMI-1640 (Life Technologies) supplemented with 15% FBS (Life Technologies). DMSO controls and compound incubations (0.1µM, 0.3µM, and 1µM) were carried out in 24-well plates for 16h. Cells were harvested and lysed with cell lysis buffer (Cell Signaling Technologies) containing protease inhibitors (Thermo Scientific). Lysates were clarified at 16,000g for 10 minutes, and supernatants were separated by SDS-PAGE. Immunoblotting was performed using standard protocols. The antibodies used were ERRα (Cell Signaling #8644) and GAPDH (Cell Signaling #5174). Bands were quantified using a Bio-Rad ChemiDoc MP imaging system.

### 3. BRD4 Western Protocol

VCaP cells were purchased from ATCC and cultured in Dulbecco's Modified Eagle's Medium (ATCC), supplemented with 10% FBS (ATCC) and Penicillin/Streptomycin (Life Technologies). DMSO control and compound treatments (0.003µM, 0.01µM, 0.03 µM and 0.1µM) were performed in 12-well plates for 16h. Cells were harvested, and lysed in RIPA buffer (50mM Tris pH8, 150mM NaCl, 1% Tx-100, 0.1% SDS, 0.5% sodium deoxycholate) supplemented with protease and phosphatase inhibitors. Lysates were clarified at 16,000g for 10 minutes, and protein concentration was determined. Equal amount of protein (20µg) was subjected to SDS-PAGE analysis and followed by immunoblotting according to standard protocols. The antibodies used were BRD4 (Cell Signaling #13440), and Actin (Sigma #5441). Detection reagents were Clarity Western ECL substrate (Bio-rad #170-5060).

### 4. AR ELISA Protocol

VCaP cells were purchased from ATCC and cultured in Dulbecco's Modified Eagle's Medium (ATCC), supplemented with 10% FBS (ATCC) and Penicillin/Streptomycin (Life Technologies). DMSO control and compound treatments (0.0001 µM - 1µM) were performed in 96-well plates for 16h. Cells were harvested, and lysed with Cell Lysis Buffer (Catalog# 9803) (20mM Tris-HCL (pH 7.5), 150 mM NaCl, 1mM Na₂EDTA, 1 mM EGTA, 1% Triton, 2.5 mM sodium pyrophosphate, 1 mM B-glycerophosphate, 1 mM Na₃VO₄, 1 ug/ml leupeptin. Lysates were clarified at 16,000g for 10 minutes, and loaded into the PathScan AR ELISA (Cell Signaling Catalog#12850). The PathScan^{®} Total Androgen Receptor Sandwich ELISA Kit is a solid phase sandwich enzyme-linked immunosorbent assay (ELISA) that detects endogenous levels of total androgen receptor protein. An Androgen Receptor Rabbit mAb has been coated onto the microwells. After incubation with cell lysates, androgen receptor protein is captured by the coated antibody. Following extensive washing, an Androgen Receptor Mouse Detection mAb is added to detect the captured androgen receptor protein. Anti-mouse IgG, HRP-linked Antibody is then used to recognize the bound detection antibody. HRP substrate, TMB, is added to develop color. The magnitude of absorbance for the developed color is proportional to the quantity of total androgen receptor protein.

Antibodies in kit are custom formulations specific to kit.

### 5. c-Myc ELISA Assay Protocol

22RV-1 cells were purchased from ATCC and and cultured in RPMI +10% FBS media. Cells were harvested using trypsin (Gibco #25200-114), counted and seeded at 30,000 cells/well at a volume of 75 µL/well in RPMI +10% FBS media in 96-well plates. The cells were dosed with compounds diluted in 0.1% DMSO, incubated for 18h then washed and lysed in 50uL RIPA buffer (50mM Tris pH8, 150mM NaCl, 1% Tx-100, 0.1% SDS, 0.5% sodium deoxycholate) supplemented with protease and phosphatase inhibitors. The lysates were clarified at 4000rpm at 4 °C for 10 minutes then aliquots were added into a 96-well ELISA plate of Novex Human c-myc ELISA kit from Life Technologies Catalog #KHO2041. 50ul of c-Myc Detection antibody was added into every well, the plates incubated at room temperature for 3hrs, then washed with ELISA wash buffer. 100uL of the anti-rabbit IgG-HRP secondary antibody was added to each well and incubated at room temperature for 30 minutes. The plates were washed with ELISA wash buffer, 100 µL TMB added to each well, and then monitored every 5 minutes for a color change. 100 µL of stop solution is added and the plates read at 450nm.

### D. Results

Table 1 provides the results of experimental data obtained from a representative number of compounds encompassed by the present disclosure as well as compounds outside of the scope of the present invention which are given for reference and are marked accordingly with "reference". In particular, various cell types were treated with the Compounds listed in Table 1, which are identified by chemical structure, mass spectrometry characterization, and compound name.

Table 1 shows that (A) 10-30% degradation was acheived in cells treated with 1uM of Compounds 1, 6-9, 12, and 17; (B) 31-50% degradation was acheived in cells treated with 1uM of Compounds 2-5, 10, and 20; and (C) > 50% degreadation was achieved in cells treated with 1uM of Compounds 11, 13-16, 18-19, 21 and 22. Table 1 also shows that (D) Compounds 24 and 26-35 have an IC₅₀ <50nM, while (E) Compounds 23 and 25 have an IC₅₀ of >50nM.

**Table 1**

| # | Structure | Degradation Activity | | | | | MH⁺ | Chemical name |
|---|---|---|---|---|---|---|---|---|
| | | AR¹ | BRD4¹ | TBK1² | ERRa³ | cMyc⁴ | | |
| 1 | 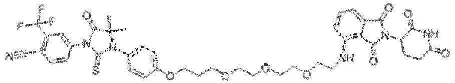 | A | | | | | 851.25 | 4-{3-[4-({1-[2-(2,6-dioxopiperidin-3-yl)-1,3-dioxo-2,3-dihydro-1H-isoindol-4-yl]-4,7,10-trioxa-1-azatridecan-13-yl}oxy)phenyl]-4,4-dimethyl-5-oxo-2-sulfanylideneimidazolidin-1-yl}-2-(trifluoromethyl)benzonitrile |
| 2 | 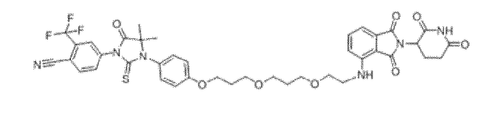 | B | | | | | 821.25 | 4-[3-(4-{3-[3-(2-{{2-(2,6-dioxopiperidin-3-yl)-1,3-dioxo-2,3-dihydro-1H-isoindol-4-yl]amino}ethoxy)propoxy]prop oxy}phenyl)-4,4-dimethyl-5-oxo-2-sulfanylideneimidazolidin-1-yl]-2-(trifluoromethyl)benzonitrile |
| 3 | 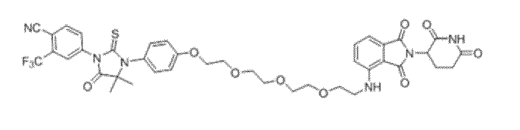 | B | | | | | 837.23 | 4-{3-[4-({1-[2-(2,6-dioxopiperidin-3-yl)-1,3-dioxo-2,3-dihydro-1H-isoindol-4-yl]-4,7,10-trioxa-1-azadodecan-12-yl}oxy)phenyl]-4,4-dimethyl-5-oxo-2-sulfanylideneimidazolidin-1-yl}-2-(trifluoromethyl)benzonitrile |
| 4 | 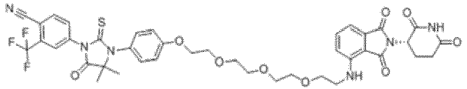 | B | | | | | 837.24 | 4-(3-{4-[(1-{2-[(3S)-2,6-dioxopiperidin-3-yl]-1, 3-di oxo-2,3-dihydro-1H-isoindol-4-yl}-4,7,10-trioxa-1-azadodecan-12-yl)oxy]phenyl)-4,4-dimethyl-5-oxo-2-sulfa nylideneimidazolidin-1-yl)-2-(trifluoromethyl)benzonitrile |

**Table 1 (continued)**

| | | | | | | | | |
|---|---|---|---|---|---|---|---|---|
| 5 | 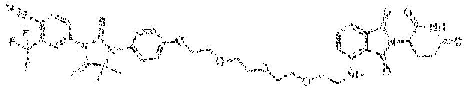 | B | | | | | 837.24 | 4-(3-{4-[(1-{2-[(3R)-2,6-dioxopiperidin-3-yl]-1,3-dioxo-2,3-dihydro-1H-isoindol-4-yl}-4,7,10-trioxa-1-azadodecan-12-yl)oxy]phenyl}-4,4-dimethyl-5-oxo-2-sulfanylideneimidazolidin-1-yl)-2-(trifluoromethyl)benzonitrile |
| 6 | 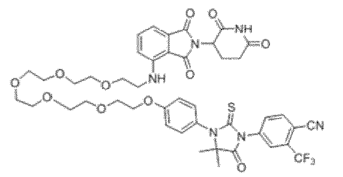 | A | | | | | 925.30 | 4-{3-[4-({1-[2-(2,6-dioxopiperidin-3-yl)-1,3-dioxo-2,3-dihydro-1H-isoindol-4-yl]-4,7,10,13,16-pentaoxa-1-azaoctadecan-18-yl}oxy)phenyl]-4,4-dimethyl-5-oxo-2-sulfanylideneimidazolidin-1-yl}-2-(trifluoromethyl)benzonitrile |
| 7 | 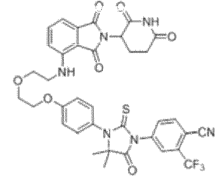 | A | | | | | 749.19 | 4-(3-{4-[2-(2-{[2-(2,6-dioxopiperidin-3-yl)-1,3-dioxo-2,3-dihydro-1H-isoindol-4-yl]amino}ethoxy)ethoxy]phenyl }-4,4-dimethyl-5-oxo-2-sulfanylideneimidazolidin-1-yl)-2-(trifluoromethyl)benzonitrile |
| 8 | 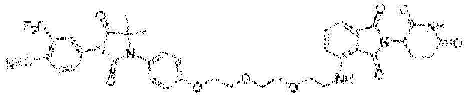 | A | | | | | 793.28 | 4-[3-(4-{2-[2-(2-{[2-(2,6-dioxopiperidin-3-yl)-1,3-dioxo-2,3-dihydro-1H-isoindol-4-yl]amino}ethoxy)ethoxy]ethoxy }phenyl)-4,4-dimethyl-5-oxo-2-sulfanylideneimidazolidin-1-yl]-2-(trifluoromethyl)benzonitrile |
| 9 | 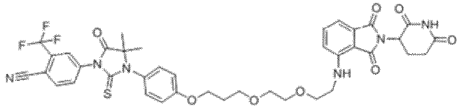 | A | | | | | 807.32 | 4-[3-(4-{3-[2-(2-{[2-(2,6-dioxopiperidin-3-yl)-1,3-dioxo-2,3-dihydro-1H-isoindol-4-yl]amino}ethoxy)ethoxy]propox y}phenyl)-4,4-dimethyl-5-oxo-2-sulfanylideneimidazolidin-1-yl]-2-(trifluoromethyl)benzonitrile |
| 10 | 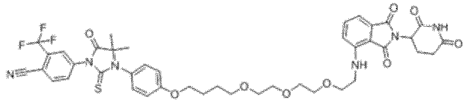 | B | | | | | 865.36 | 4-{3-[4-({1-[2-(2,6-dioxopiperidin-3-yl)-1,3-dioxo-2,3-dihydro-1H-isoindol-4-yl]-4,7,10-trioxa-1-azatetradecan-14-yl}oxy)phenyl]-4,4-dimethyl-5-oxo-2-sulfanylideneimidazolidin-1-yl}-2-(trifluoromethyl)benzonitrile |
| 11 | 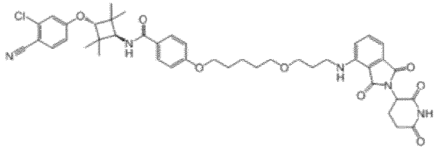 | C | | | | | 799.31 | 4-{[5-(3-{[2-{2,6-dioxopiperidin-3-yl)-1,3-dioxo-2,3-dihydro-1H-isoindol-4-yl]amino}propoxy)pentyl]oxy}-N-[trans-3-(3-chloro-4-cyanophenoxy)-2,2,4,4-tetramethylcyclobutyl]benzami de |
| 12 | 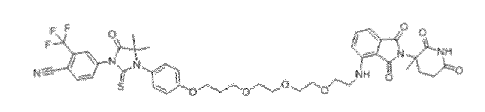 | A | | | | | 865.16 | 4-{4,4-dimethyl-3-[4-({1-[2-(3-methyl-2,6-dioxopiperidin-3-yl)-1,3-dioxo-2,3-dihydro-1H-isoindol-4-yl]-4,7,10-trioxa-1 - azatridecan-13-yl}oxy)phenyl]-5-oxo-2-sulfanylideneimidazolidin-1-yl}-2-(trifluoromethyl)benzonitrile |
| 13 | 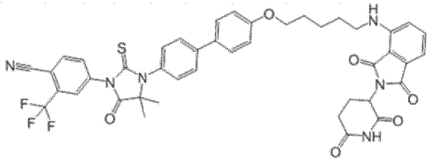 | C | | | | | 823.12 | 4-{3-(4-{4-[(5-{[2-(2,6-dioxopiperidin-3-yl)-1,3-dioxo-2,3-dihydro-1H-isoindol-4-yl]amino)pentyl)oxy]phenyl)ph enyl)-4,4-dimethyl-5-oxo-2-sulfanylideneimidazolidin-1-yl]-2-(trifluoromethyl)benzonitri le |
| 14 | 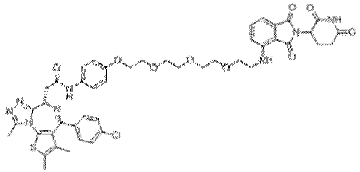 | | C | | | | 923.29 & 925.29 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[4-({1-[2-(2,6-dioxopiperidin-3-yl)-1,3-dioxo-2,3-dihydro-1H-isoindol-4-yl]-4,7,10-trioxa-1-azadodecan-12-yl}oxy)phenyl]acetamide |
| 15 | 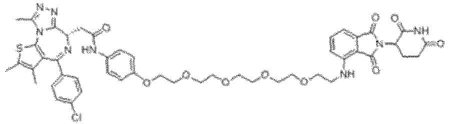 | | C | | | | 967.31 & 969.31 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[4-({1-(2-(2,6-dioxopiperidin-3-yl)-1,3-dioxo-2,3-dihydro-1H-isoindol-4-_{YIl}-4,7,10,13-tetraoxa-1-azapentadecan-15-yl}oxy)phenyl]acetamide |
| 16 | 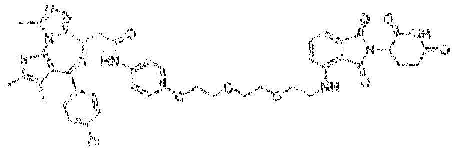 | | C | | | | 879.26 & 881.26 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-(4-{2-[2-(2-{[2-(2,6-dioxopiperidin-3-yl)-1,3-dioxo-2,3-dihydro-1H-isoindol-4-yl]amino}ethoxy)ethoxy]ethoxy }phenyl)acetamide |
| 17 | 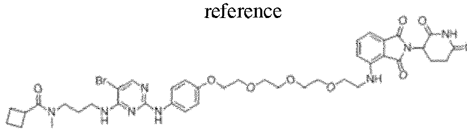 | | | A | | | 865.27 & 867.27 | N-{3-[(5-bromo-2-{[4-({1-[2-(2,6-dioxopiperidin-3-yl)-1,3-dioxo-2,3-dihydro-1H-isolndol-4-yl]-4,7,10-trioxa-1-azadodecan-12-yt}oxy)phenyl]amino}pyrimidin-4-yl)amino]propyl}-N-methylcyclobutanecarboxamid e |
| 18 | 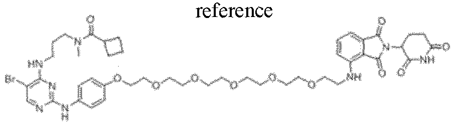 | | | C | | | 953.32 & 955.32 | N-{3-[(5-bromo-2-{[4-((1-{2-(2,6-dioxopiperidin-3-yl)-1,3-dioxo-2,3-dihydro-1H-isoindol-4-yl)-4,7,10,13,16-pentaoxa-1 - azaoctadecan-18-yl}oxy)phenyl]amino}pyrímldin-4-yl)amino]propyl}-N-methylcyclobutanecarboxamid e |
| 19 | 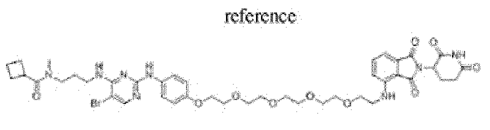 | | | C | | | 909.31 & 911.31 | N-{3-[(5-bromo-2-{[4-((1-[2-(2.6-dioxopiperidin-3-yl}-1,3-dioxo-2,3-dihydro-1H-isoindol-4-yl]-4,7,10,13-tetraoxa-1-azapentadecan-15-yl)oxy)phenyl]amino}pyrimidin-4-yl)amino]propyl}-N-methylycyclobutanecarboxamid e |
| 20 | 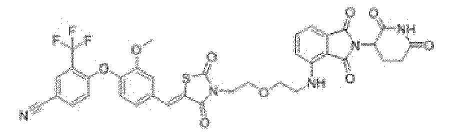 | | | | B | | 764.15 | 4-{4-{{(5Z)-3-{2-(2-[(2-(2,6-dioxopiperidin-3-yl)-1,3-dioxo 2,3-dihydro-1H-isoindol-4-yl]amino)ethoxy)ethyl]-2,4-dioxo-1,3-thiazolídin-5-ylidene]methyl}-2-methoxyphenoxy)-3-trifluoromethyl)benzonitrile |
| 21 | 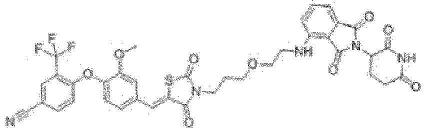 | | | | C | | 778.18 | 4-(4-{[(5Z)-3-[3-(2-{[2-(2,6-dioxopiperidin-3-yl)-1,3-dioxo-2,3-dhydro-1H-isoindol-4-yl]amino)ethoxy)propyl]-2,4-dioxo-1,3-thiazolidin-5-ylidene]methyl}-2-methoxyphenoxy)-3-truflurorormethyl(benzonitrile |
| 22 | 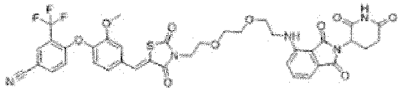 | | | | C | | 808.19 | 4-(4-{[(5Z)-3-[3-(2-{[2-(2,6-dioxopiperidin-3-yl)-1,3-dioxo-2,3-dihydro-1H-isoindol-4-yl]amino)elhoxy)ethoxy]ethyl)-2,4-dioxo-1,3-thiazolidin-5-ylidene]methy}2-methoxyphenoxy)-3-(trifluoromethyl)benzonitrile |
| 23 | 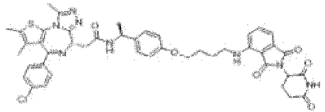 | | | | | E | 847.21 & 849.21 | 2-[{9S)-7-{4-chlorophenyl}-4,5,13-trimethyl-3-thia-1,8,11,12-tatraazatricyclo[8-3.0.0².⁶]tride ca-2(6),4,7,10,12-pentaen-9-yl]-N-{(1S)-1-[4-(4-{[2-(2,6-dioxopiperidin-3-yl)-1,3-dioxo-2,3-dihydro-1H-îsoindol-4-yl]amino)butoxy)phenyl]ethyl]a cetamide |
| 24 | 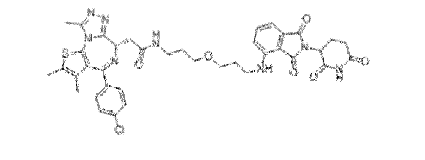 | | | | | D | 771.16& 773.16 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[3-(3- {[2-(2,6-dioxopiperidin-3-yl)-1,3-dioxo-2,3-dihydro-1H-isoindol-4-yl]amino}propoxy)propylyl]acetamide] |
| 25 | 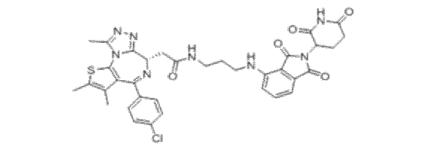 | | | | | E | 713.14 & 715.14 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-(3-{[2-(2,6-dioxopiperidin-3-yl)-1,3-dioxo-2,3-dihydro-1H-isoindol-4-yl]amino}propyl)acetamide |
| 26 | 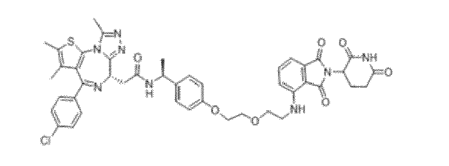 | | | | | D | 863.26 & 865.26 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[(1S)-1-{4-[2-(2-{[2-(2,6-dioxopiperidin-3-yl)-1,3-dioxo-2,3-dihydro-1H-isoindol-4-yl]amino}ethoxy)phenyl]ethyl] acetamide |
| 27 | 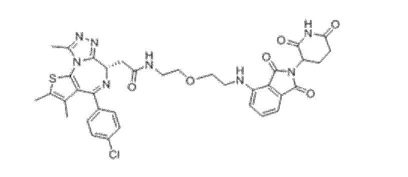 | | | | | D | 743.20& 745.20 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[2-(2-{[2-(2,6-dioxopiperidin-3-yl)-1,3-dioxo-2,3-dihydro-1H-isoindol-4-yl]amino}ethoxy)acetamide |
| 28 | 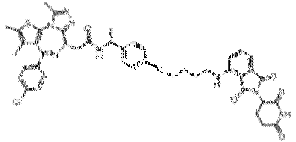 | | | | | D | 847.42 & 849.42 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[(1R)-1-[4-(4-{[2-(2,6-dioxopiperidin-3-yl)-1,3-dioxo-2,3-dihydro-1H-isoindol-4-yl]amino}butoxy)phenyl]ethyl]a cetamide |
| 29 | 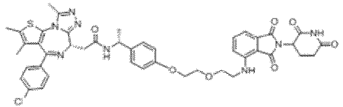 | | | | | D | 863.18 & 865.18 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[(1R)-1-{4-[2-(2-{[2-(2,6-dioxopiperidin-3-yl)-1,3-dloxo-2,3-dihydro-1H-isoindol-4-yl]amino}ethoxy)ethoxy]phenyl }ethyl]acetamide |
| 30 | 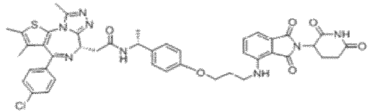 | | | | | D | 833.31 & 835.31 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[(1R)-1-[4-(3-{[2-(2,6-dioxopiperidin-3-yl)-1,3-dioxo-2,3-dihydro-1H-isoindol-4-yl]amino}propoxy)phenyl]ethyl] acetamide |
| 31 | 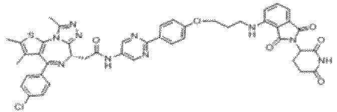 | | | | | D | 883.24 & 885.24 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-{2-[4-(3-{[2-(2,6-dioxopiperidin-3-yl)-1,3-dioxo-2,3-dihydro-1H-isoindol-4-yl]amino}propoxy)phenyl]pyrim idin-5-yl)acetamide |
| 32 | 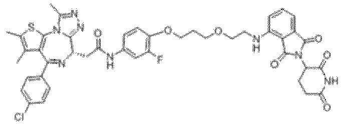 | | | | | D | 867.12 & 869.12 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-{4-[3-(2-{[2-(2,6-dioxopiperidin-3-yl)-1,3-dioxo-2,3-dihydro-1H-isoindol-4-yl]amino}ethoxy)propoxy]-3-fluorophenyl}acetamide |
| 33 | 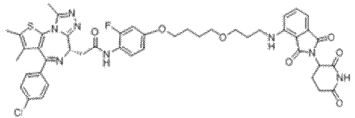 | | | | | D | 895.15 & 897.15 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-2-fluorophenyl}acetamide |
| 34 | 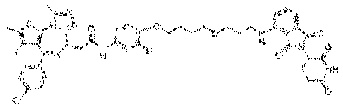 | | | | | D | 895.15 & 897.15 | 2-[(9S)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-3-fluorophenyl}acetamide |
| 35 | 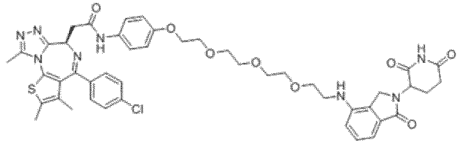 | | | | | D | 910.21 & 912.21 | 2-[(9R)-7-(4-chlorophenyl)-4,5,13-trimethyl-3-thia-1,8,11,12-tetraazatricyclo[8.3.0.0²,⁶]tride ca-2(6),4,7,10,12-pentaen-9-yl]-N-[4-({1-[2-(2,6-dioxopiperidin-3-yl)-1-oxo-2,3-dihydro-1H-isoindol-4-yl]-4,7,10-trioxa-1-azadodecan-12-yl}oxy)phenyl]acetamide |

| | | | | | | | | |
|---|---|---|---|---|---|---|---|---|
| Categories of degradation activity: A = 10-30% degradation at 1uM B = 31-50% degradation at 1uM C = >50% degradation at 1uM D = IC₅₀ <50nM E = IC₅₀ >50nM Cell used in the bioassy: ¹ VCaP cells ² Panc02.13 cells ³ Namalwa cells ⁴ 22RV-1 cells | | | | | | | | |

## Claims

1. A compound having a chemical structure comprising:
PTM-L-CLM
or a pharmaceutically acceptable salt, enantiomer or stereoisomer thereof, wherein
PTM is a small molecule protein target moiety that binds specifically to estrogen receptor, androgen receptor, or a BET bromodomain protein;
L is a chemical linker group covalently coupled to the PTM and CLM and is represented by: 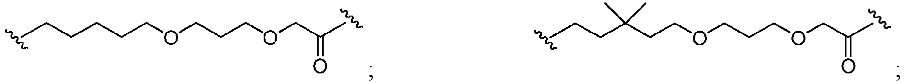 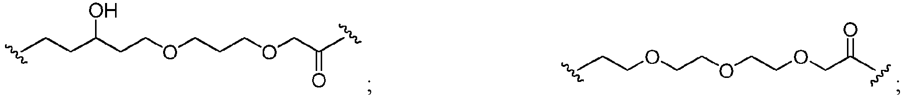 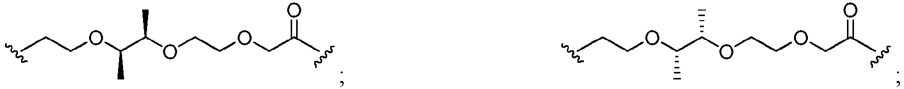 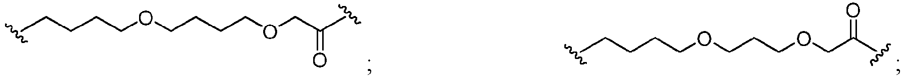 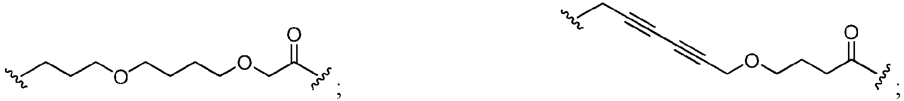 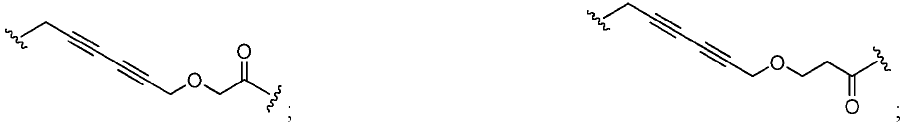 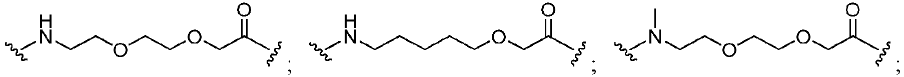 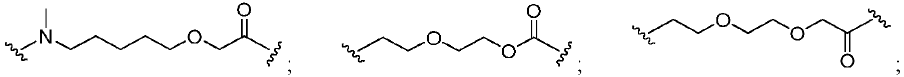 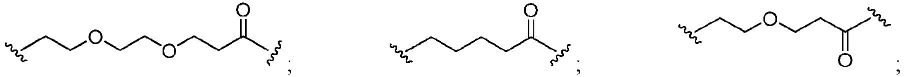 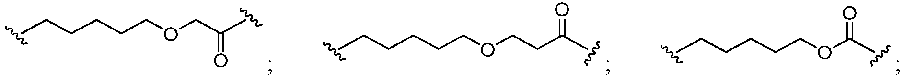 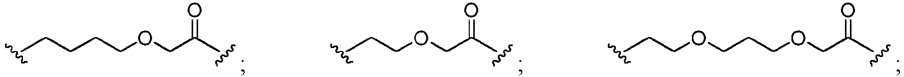 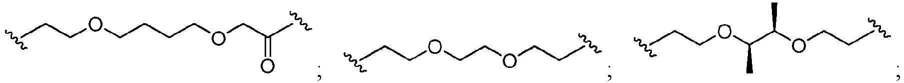 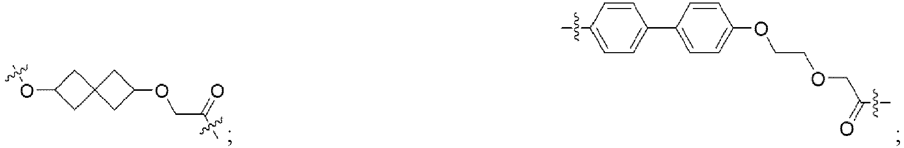 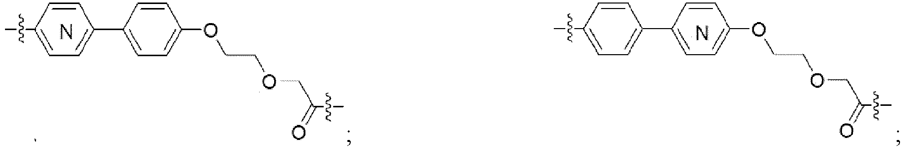 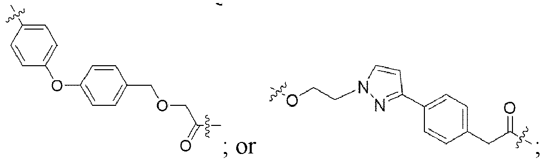 and
CLM is a cereblon E3 Ubiquitin Ligase binding moiety with the structure represented by: 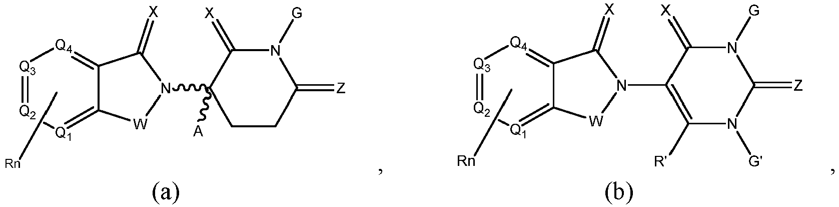 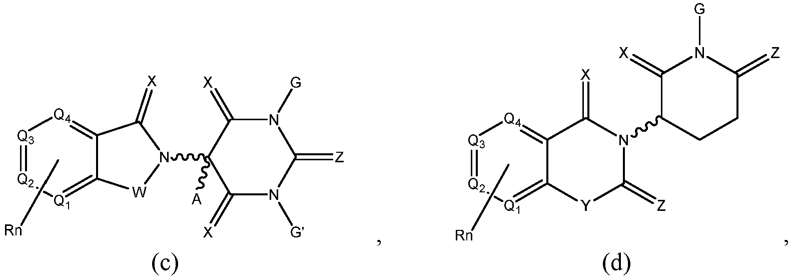 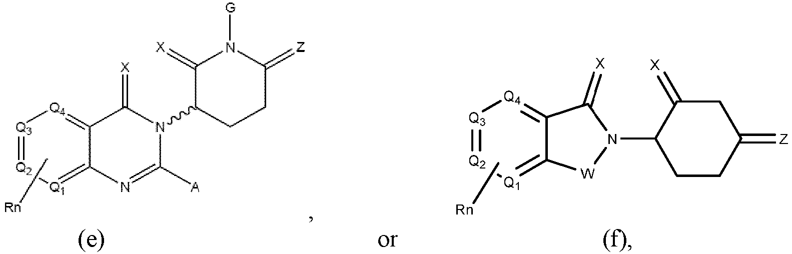 wherein
W is selected from the group consisting of CH₂, CHR, C=O, SO₂, NH, and N-alkyl;
each X is independently selected from the group consisting of O, S, and H₂;
Y is selected from the group consisting of NH, N-alkyl, N-aryl, N-heteroaryl, N-cycloalkyl, N-heterocyclyl, O, and S;
Z is selected from the group consisting of O, S, and H₂;
G and G' are independently selected from the group consisting of H, alkyl, OH, CH₂-heterocyclyl optionally substituted with R', and benzyl optionally substituted with R';
Q₁, Q₂, Q₃, and Q₄ represent (i) a nitrogen or (ii) a carbon substituted with H or R;
A is independently selected from the group H, alkyl, cycloalkyl, Cl and F;
R comprises -CONR'R", -OR', -NR'R", -SR', -SO₂R', -SO₂NR'R", -CR'R"-, -CR'NR'R"-, -aryl, -heteroaryl, -alkyl, -cycloalkyl, -heterocyclyl, -P(O)(OR')R", -P(O)R'R", - OP(O)(OR')R", -OP(O)R'R", -Cl, -F, -Br, -I, -CF₃, -CN, -NR'SO₂NR'R", -NR'CONR'R", - CONR'COR",
-NR'C(=N-CN)NR'R", -C(=N-CN)NR'R", -NR'C(=N-CN)R", -NR'C(=C-NO₂)NR'R", -SO₂NR'COR", -NO₂, -CO₂R', -C(C=N-OR')R", -CR'=CR'R", -CCR', -S(C=O)(C=N-R')R", -SF₅ and -OCF₃;
R' and R" are independently selected from the group consisting of a bond, H, alkyl, cycloalkyl, aryl, heteroaryl, heterocyclyl;
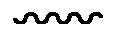 represents a bond that may be stereospecific ((R) or (S)) or non-stereospecific; and
wherein n is an integer from 1-4, and wherein
one R is modified to be covalently joined to the linker group (L).

2. The compound of claim 1, or a pharmaceutically acceptable salt, enantiomer or stereoisomer thereof, wherein the compound is selected from the group consisting of:
4-{3-[4-({1-[2-(2,6-dioxopiperidin-3-yl)-1,3-dioxo-2,3-dihydro-1H-isoindol-4-yl]-4,7,10-trioxa-1-azatridecan-13-yl}oxy)phenyl]-4,4-dimethyl-5-oxo-2-sulfanylideneimidazolidin-1-yl}-2-(trifluoromethyl)benzonitrile;
4-{3-[4-({1-[2-(2,6-dioxopiperidin-3-yl)-1,3-dioxo-2,3-dihydro-1H-isoindol-4-yl]-4,7,10-trioxa-1-azadodecan-12-yl}oxy)phenyl]-4,4-dimethyl-5-oxo-2-sulfanylideneimidazolidin-1-yl}-2-(trifluoromethyl)benzonitrile;
4-(3-{4-[(1-{2-[(3S)-2,6-dioxopiperidin-3-yl]-1,3-dioxo-2,3-dihydro-1H-isoindol-4-yl}-4,7,10-trioxa-1-azadodecan-12-yl)oxy]phenyl}-4,4-dimethyl-5-oxo-2-sulfanylideneimidazolidin-1-yl)-2-(trifluoromethyl)benzonitrile;
4-(3-{4-[[1-{2-[(3R)-2,6-dioxopiperidin-3-yl]-1,3-dioxo-2,3-dihydro-lH-isoindol-4-yl}-4,7,10-trioxa-1-azadodecan-12-yl)oxy]phenyl}-4,4-dimethyl-5-oxo-2-sulfanylideneimidazolidin-1-yl)-2-(trifluoromethyl)benzonitrile;
4-{3-[4-({1-[2-(2,6-dioxopiperidin-3-yl)-1,3-dioxo-2,3-dihydro-1H-isoindol-4-yl]-4,7,10,13,16-pentaoxa-1-azaoctadecan-18-yl}oxy)phenyl]-4,4-dimethyl-5-oxo-2-sulfanylideneimidazolidin-1-yl}-2-(trifluoromethyl)benzonitrile;
4-[3-(4-{2-[2-(2-{[2-(2,6-dioxopiperidin-3-yl)-1,3-dioxo-2,3-dihydro-1H-isoindol-4-yl]amino}ethoxy)ethoxy]ethoxy}phenyl)-4,4-dimethyl-5-oxo-2-sulfanylideneimidazolidin-1-yl]-2-(trifluoromethyl)benzonitrile;
4-[3-(4-{3-[2-(2-{[2-(2,6-dioxopiperidin-3-yl)-1,3-dioxo-2,3-dihydro-1H-isoindol-4-yl]amino}ethoxy)ethoxy]propoxy}phenyl)-4,4-dimethyl-5-oxo-2-sulfanylideneimidazolidin-1-yl]-2-(trifluoromethyl)benzonitrile;
4-{3-[4-({1-[2-(2,6-dioxopiperidin-3-yl)-1,3-dioxo-2,3-dihydro-1H-isoindol-4-yl]-4,7,10-trioxa-1-azatetradecan-14-yl}oxy)phenyl]-4,4-dimethyl-5-oxo-2-sulfanylideneimidazolidin-1-yl}-2-(trifluoromethyl)benzonitrile;
4-{4,4-dimethyl-3-[4-({1-[2-(3-methyl-2,6-dioxopiperidin-3-yl)-1,3-dioxo-2,3-dihydro-1H-isoindol-4-yl]-4,7,10-trioxa-1-azatridecan-13-yl}oxy)phenyl]-5-oxo-2-sulfanylideneimidazolidin-1-yl}-2-(trifluoromethyl)benzonitrile;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[4-({1-[2-(2,6-dioxopiperidin-3-yl)-1,3-dioxo-2,3-dihydro-1H-isoindol-4-yl]-4,7,10-trioxa-1-azadodecan-12-yl}oxy)phenyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[4-({1-[2-(2,6-dioxopiperidin-3-yl)-1,3-dioxo-2,3-dihydro-1H-isoindol-4-yl]-4,7,10,13-tetraoxa-1-azapentadecan-15-yl}oxy)phenylacetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-(4-{2-[2-(2-{[2-(2,6-dioxopiperidin-3-yl)-1,3-dioxo-2,3-dihydro-1H-isoindol-4-yl]amino}ethoxy)ethoxy]ethoxy}phenyl)acetamide;
2-[(9R)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[4-({1-[2-(2,6-dioxopiperidin-3-yl)-1-oxo-2,3-dihydro-1H-isoindol-4-yl]-4,7,10-trioxa-1-azadodecan-12-yl}oxy)phenyl]acetamide; and
4-(4-{[(5Z)-3-{2-[2-(2-{[2-(2,6-dioxopiperidin-3-yl)-1,3-dioxo-2,3-dihydro-1H-isoindol-4-yl]amino}ethoxy)ethoxy]ethyl}-2,4-dioxo-1,3-thiazolidin-5-ylidene]methyl}-2-methoxyphenoxy)-3-(trifluoromethyl)benzonitrile.

3. A compound, or a pharmaceutically acceptable salt, enantiomer or stereoisomer thereof, selected from the group consisting of:
4-[3-(4-{3-[3-(2-{[2-(2,6-dioxopiperidin-3-yl)-1,3-dioxo-2,3-dihydro-1H-isoindol-4-yl]amino}ethoxy)propoxy]propoxy}phenyl)-4,4-dimethyl-5-oxo-2-sulfanylideneimidazolidin-1-yl]-2-(trifluoromethyl)benzonitrile;
4-(3-{4-[2-(2-{[2-(2,6-dioxopiperidin-3-yl)-1,3-dioxo-2,3-dihydro-1H-isoindol-4-yl]amino}ethoxy)ethoxy]phenyl}-4,4-dimethyl-5-oxo-2-sulfanylideneimidazolidin-1-yl)-2-(trifluoromethyl)benzonitrile;
4-{[5-(3-{[2-(2,6-dioxopiperidin-3-yl)-1,3-dioxo-2,3-dihydro-1H-isoindol-4-yl]amino}propoxy)pentyl]oxy}-N-[trans-3-(3-chloro-4-cyanophenoxy)-2,2,4,4-tetramethylcyclobutyl]benzamide;
4-[3-(4-{4-[(5-{[2-(2,6-dioxopiperidin-3-yl)-1,3-dioxo-2,3-dihydro-1H-isoindol-4-yl]amino}pentyl)oxy]phenyl}phenyl)-4,4-dimethyl-5-oxo-2-sulfanylideneimidazolidin-1-yl]-2-(trifluoromethyl)benzonitrile;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[(1S)-1-[4-(4-{[2-(2,6-dioxopiperidin-3-yl)-1,3-dioxo-2,3-dihydro-1H-isoindol-4-yl]amino}butoxy)phenyl]ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[3-(3-{[2-(2,6-dioxopiperidin-3-yl)-1,3-dioxo-2,3-dihydro-1H-isoindol-4-yl]amino}propoxy)propyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-(3-{[2-(2,6-dioxopiperidin-3-yl)-1,3-dioxo-2,3-dihydro-1H-isoindol-4-yl]amino }propyl)acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[(1S)-1-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[2-(2-{[2-(2,6-dioxopiperidin-3-yl)-1,3-dioxo-2,3-dihydro-1H-isoindol-4-yl]amino}ethoxy)ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-[4-(4-{[2-(2,6-dioxopiperidin-3-yl)-1,3-dioxo-2,3-dihydro-1H-isoindol-4-yl]amino}butoxy)phenyl]ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-[4-(3-{[2-(2,6-dioxopiperidin-3-yl)-1,3-dioxo-2,3-dihydro-1H-isoindol-4-yl]amino}propoxy)phenyl]ethyl]acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-{2-[4-(3-{[2-(2,6-dioxopiperidin-3-yl)-1,3-dioxo-2,3-dihydro-1H-isoindol-4-yl]amino}propoxy)phenyl]pyrimidin-5-yl}acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-{4-[3-(2-{[2-(2,6-dioxopiperidin-3-yl)-1,3-dioxo-2,3-dihydro-1H-isoindol-4-yl]amino}ethoxy)propoxy]-3-fluorophenyl}acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-2-fluorophenyl}acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-3-fluorophenyl}acetamide;
4-(4-{[(5Z)-3-[2-(2-{[2-(2,6-dioxopiperidin-3-yl)-1,3-dioxo-2,3-dihydro-1H-isoindol-4-yl]amino}ethoxy)ethyl]-2,4-dioxo-1,3-thiazolidin-5-ylidene]methyl}-2-methoxyphenoxy)-3-(trifluoromethyl)benzonitrile; and
4-(4-{[(5Z)-3-[3-(2-{[2-(2,6-dioxopiperidin-3-yl)-1,3-dioxo-2,3-dihydro-1H-isoindol-4-yl]amino}ethoxy)propyl]-2,4-dioxo-1,3-thiazolidin-5-ylidene]methyl}-2-methoxyphenoxy)-3-(trifluoromethyl)benzonitrile.

4. The compound of claim 1, or a pharmaceutically acceptable salt, enantiomer or stereoisomer thereof, wherein the PTM binds to estrogen receptor or androgen receptor.

5. The compound of claim 1, or a pharmaceutically acceptable salt, enantiomer or stereoisomer thereof, wherein the PTM binds to estrogen receptor.

6. The compound of claim 1, or a pharmaceutically acceptable salt, enantiomer or stereoisomer thereof, wherein the PTM binds to androgen receptor.

7. The compound of claim 1, or a pharmaceutically acceptable salt, enantiomer or stereoisomer thereof, wherein the PTM binds to BET bromodomain protein.

8. A pharmaceutical composition comprising the compound of any one of claims 1 to 3, or a pharmaceutically acceptable salt, enantiomer or stereoisomer thereof, and a pharmaceutically acceptable carrier, additive, and/or excipient.

9. The pharmaceutical composition of claim 8, further comprising an antiviral agent, an anti-HIV agent, or an anticancer agent.

10. The pharmaceutical composition of claim 8, further comprising an anti-inflammation agent, an immunological agent, a cardiovascular agent, or a neurological agent.

11. The pharmaceutical composition of claim 9 wherein the anticancer agent is selected from the group consisting of a FLT-3 inhibitor, a VEGFR inhibitor, an EGFR TK inhibitor, an aurora kinase inhibitor, a PIK-1 modulator, a Bcl-2 inhibitor, an HDAC inhbitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, an mTORC1/2 inhibitor, a JAK/STAT inhibitor, a checkpoint-1 or 2 inhibitor, a focal adhesion kinase inhibitor, a Map kinase kinase (mek) inhibitor, a VEGF trap antibody, and mixtures thereof.

12. A compound of any one of claims 1 to 3, or a pharmaceutically acceptable salt, enantiomer or stereoisomer thereof, for use in a method of treating a disease state or condition that is modulated through protein degradation in a patient, the method comprising administering an effective amount of the compound or pharmaceutically acceptable salt, enantiomer or stereoisomer thereof.

13. A compound of any one of claims 1 to 3, or a pharmaceutically acceptable salt, enantiomer or stereoisomer thereof, for use in a method of treating cancer, the method comprising administering an effective amount of the compound or pharmaceutically acceptable salt, enantiomer or stereoisomer thereof to a patient in need thereof, wherein the cancer is large B-cell lymphoma, prostate cancer, ovarian cancer, breast cancer, multiple myeloma, uterine cancer, colon cancer, or leukemia.

14. The compound or pharmaceutically acceptable salt, enantiomer or stereoisomer thereof for use of claim 13, wherein the cancer is prostate cancer or breast cancer.

## Patentansprüche

1. Verbindung mit einer chemischen Struktur, umfassend:
PTM-L-CLM
oder ein pharmazeutisch annehmbares Salz, Enantiomer oder Stereoisomer davon, wobei
PTM eine niedermolekulare Protein-Zieleinheit ist, die spezifisch an einen Östrogenrezeptor, Androgenrezeptor oder ein BET-Bromodomänen-Protein bindet;
L eine chemische Linkergruppe ist, die kovalent an das PTM und CLM gekoppelt ist und dargestellt wird durch: 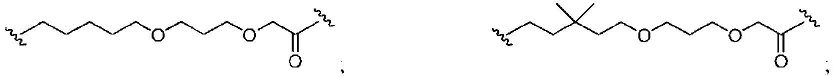 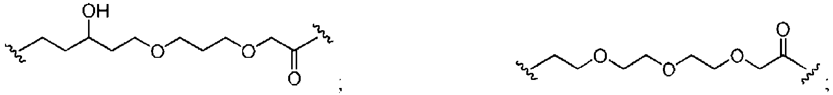 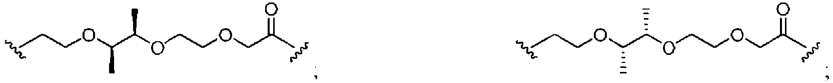 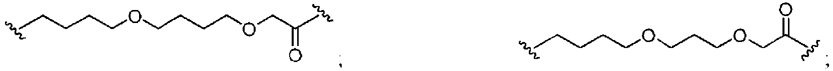 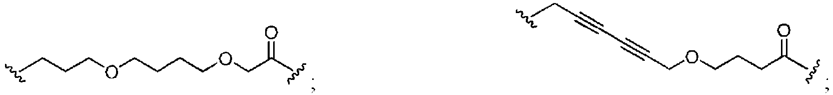 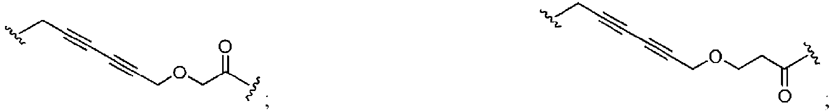 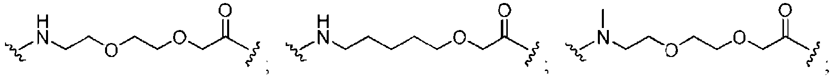 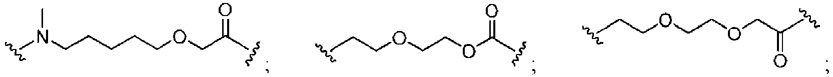 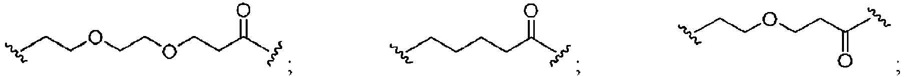 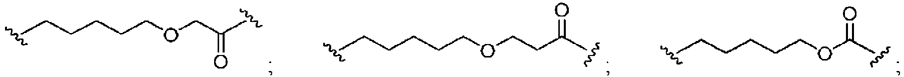 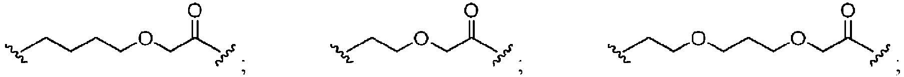 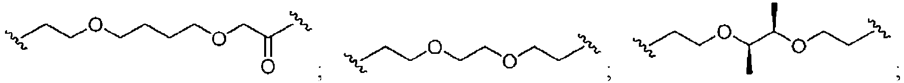 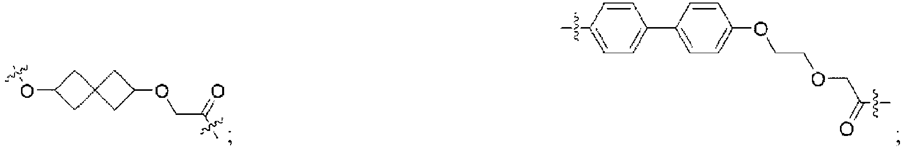 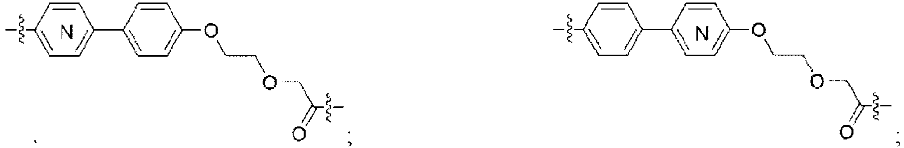 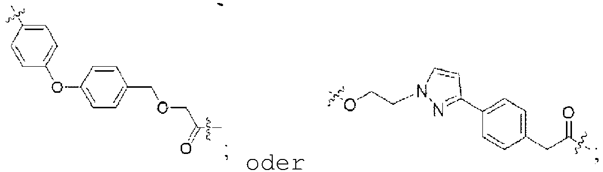 und
CLM eine Cereblon-E3-Ubiquitin-Ligase-Bindungseinheit
mit der durch Folgendes dargestellten Struktur ist: 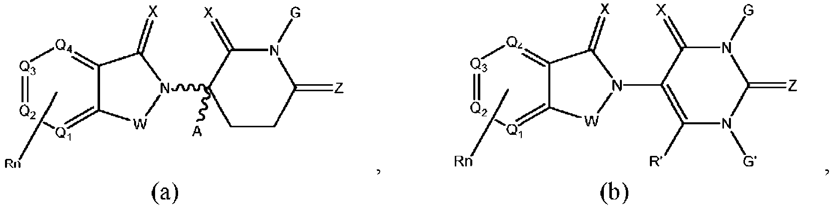 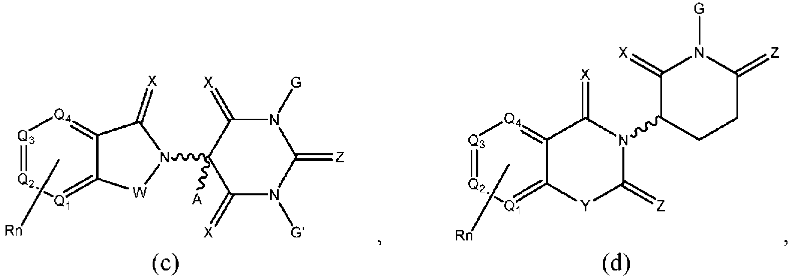 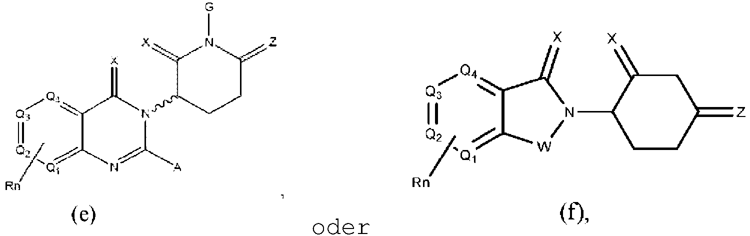 wobei
W ausgewählt ist aus der Gruppe bestehend aus CH₂, CHR, C=O, SO₂, NH und N-Alkyl;
jedes X unabhängig voneinander ausgewählt ist aus der Gruppe bestehend aus O, S und H₂;
Y ausgewählt ist aus der Gruppe bestehend aus NH, N-Alkyl, N-Aryl, N-Heteroaryl, N-Cycloalkyl,
N-Heterocyclyl, O und S;
Z ausgewählt ist aus der Gruppe bestehend aus O, S und H₂;
G und G' unabhängig voneinander ausgewählt sind aus der Gruppe bestehend aus H, Alkyl, OH, CH₂-Heterocyclyl, optional substituiert mit R', und Benzyl, optional substituiert mit R';
Q₁, Q₂, Q₃ und Q₄ (i) einen Stickstoff oder (ii) einen mit H oder R substituierten Kohlenstoff darstellen;
A unabhängig ausgewählt ist aus der Gruppe H, Alkyl, Cycloalkyl, Cl und F;
R -CONR'R", -OR', -NR'R", -SR', -SO₂R', -SO₂NR'R", -CR'R"-, -CR'NR'R"-, -Aryl, -Heteroaryl, -Alkyl, -Cycloalkyl, -Heterocyclyl, -P(O)(OR')R", -P(O)R'R", -
OP(O) (OR')R", -OP(O)R'R", -Cl, -F, -Br, -I, -CF₃, -CN, - NR'SO₂NR'R", -NR'CONR'R", - CONR'COR",
-NR'C(=N-CN)NR'R", -C(=N-CN)NR'R", -NR'C(=N-CN)R", - NR'C(=C-NO₂)NR'R", -SO₂NR'COR", -NO₂, -CO₂R', -C(C=N-OR')R", -CR'=CR'R", -CCR', -S(C=O)(C=N-R')R", -SF₅ und - OCF₃ umfasst;
R' und R" unabhängig voneinander ausgewählt sind aus der Gruppe bestehend aus einer Bindung, H, Alkyl, Cycloalkyl, Aryl, Heteroaryl, Heterocyclyl;
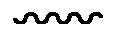 eine Bindung darstellt, die stereospezifisch ((R) oder (S)) oder nicht stereospezifisch sein kann; und
wobei n eine ganze Zahl von 1-4 ist, und wobei ein R modifiziert ist, um kovalent mit der Linkergruppe (L) verknüpft zu sein.

2. Verbindung nach Anspruch 1 oder pharmazeutisch annehmbares Salz, Enantiomer oder Stereoisomer davon, wobei die Verbindung ausgewählt ist aus der Gruppe bestehend aus:
4-{3-[4-({1-[2-(2,6-Dioxopiperidin-3-yl)-1,3-dioxo-2,3-dihydro-1H-isoindol-4-yl]-4,7,10-trioxa-1-azatridecan-13-yl}oxy)phenyl]-4,4-dimethyl-5-oxo-2-sulfanylidenimidazolidin-1-yl}-2-(trifluormethyl)benzonitril;
4-{3-[4-({1-[2-(2,6-Dioxopiperidin-3-yl)-1,3-dioxo-2,3-dihydro-1H-isoindol-4-yl]-4,7,10-trioxa-1-azadodecan-12-yl}oxy)phenyl]-4,4-dimethyl-5-oxo-2-sulfanylidenimidazolidin-1-yl}-2-(trifluormethyl)benzonitril;
4-(3-{4-[(1-{2-[(3S)-2,6-Dioxopiperidin-3-yl]-1,3-dioxo-2,3-dihydro-1H-isoindol-4-yl}-4,7,10-trioxa-1-azadodecan-12-yl)oxy]phenyl}-4,4-dimethyl-5-oxo-2-sulfanylidenimidazolidin-1-yl)-2-(trifluormethyl)benzonitril;
4-(3-{4-[(1-{2-[(3R)-2,6-Dioxopiperidin-3-yl]-1,3-dioxo-2,3-dihydro-1H-isoindol-4-yl}-4,7,10-trioxa-1-azadodecan-12-yl)oxy]phenyl}-4,4-dimethyl-5-oxo-2-sulfanylidenimidazolidin-1-yl)-2-(trifluormethyl)benzonitril;
4-{3-[4-({1-[2-(2,6-Dioxopiperidin-3-yl)-1,3-dioxo-2,3-dihydro-1H-isoindol-4-yl]-4,7,10,13,16-pentaoxa-1-azaoctadecan-18-yl}oxy)phenyl]-4,4-dimethyl-5-oxo-2-sulfanylidenimidazolidin-1-yl}-2-(trifluormethyl)benzonitril;
4-[3-(4-{2-[2-(2-{[2-(2,6-Dioxopiperidin-3-yl)-1,3-dioxo-2,3-dihydro-1H-isoindol-4-yl]amino}ethoxy)ethoxy]ethoxy}phenyl)-4,4-dimethyl-5-oxo-2-sulfanylidenimidazolidin-1-yl]-2-(trifluormethyl)benzonitril;
4-[3-(4-{3-[2-(2-{ [2-(2,6-Dioxopiperidin-3-yl)-1,3-dioxo-2,3-dihydro-1H-isoindol-4-yl]amino}ethoxy)ethoxy]propoxy}phenyl)-4,4-dimethyl-5-oxo-2-sulfanylidenimidazolidin-1-yl]-2-(trifluormethyl)benzonitril;
4-{3-[4({1-[2-(2,6-Dioxopiperidin-3-yl)-1,3-dioxo-2,3-dihydro-1H-isoindol-4-yl]-4,7,10-trioxa-1-azatetradecan-14-yl}oxy)phenyl]-4,4-dimethyl-5-oxo-2-sulfanylidenimidazolidin-1-yl}-2-(trifluormethyl)benzonitril;
4-{4,4-Dimethyl-3-[4-({1-[2-(3-methyl-2,6-dioxopiperidin-3-yl)-1,3-dioxo-2,3-dihydro-1H-isoindol-4-yl]-4,7,10-trioxa-1-azatridecan-13-yl}oxy)phenyl]-5-oxo-2-sulfanylidenimidazolidin-1-yl}-2-(trifluormethyl)benzonitril;
2-[(9S)-7-(4-Chlorphenyl)-4,5,13-trimethyl-3-thia-1,8,11,12-tetraazatricyclo[8.3.0.0²,⁶]trideca-2(6) ,4,7,10,12-pentaen-9-yl]-N-[4-({1-[2-(2,6-dioxopiperidin-3-yl)-1,3-dioxo-2,3-dihydro-1H-isoindol-4-yl]-4,7,10-trioxa-1-azadodecan-12-yl}oxy)phenyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[4-({1-[2-(2,6-dioxopiperidin-3-yl)-1,3-dioxo-2,3-dihydro-1H-isoindol-4-yl]-4,7,10,13-tetraoxa-1-azapentadecan-15-yl}oxy)phenyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-(4-{2-[2-(2-{[2-(2,6-dioxopiperidin-3-yl)-1,3-dioxo-2,3-dihydro-1H-isoindol-4-yl]amino}ethoxy)ethoxy]ethoxy}phenyl)acetamid;
2-[(9R)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[4-({1-[2-(2,6-dioxopiperidin-3-yl)-1-oxo-2,3-dihydro-1H-isoindol-4-yl]-4,7,10-trioxa-1-azadodecan-12-yl}oxy)phenyl]acetamid; und
4-(4-{[(5Z)-3-{2-[2-(2-{[2-(2,6-Dioxopiperidin-3-yl)-1,3-dioxo-2,3-dihydro-1H-isoindol-4-yl]amino}ethoxy)ethoxy]ethyl}-2,4-dioxo-1,3-thiazolidin-5-yliden]methyl}-2-methoxyphenoxy)-3-(trifluormethyl)benzonitril.

3. Verbindung oder pharmazeutisch annehmbares Salz, Enantiomer oder Stereoisomer davon, ausgewählt aus der Gruppe bestehend aus:
4-[3-(4-{3-[3-(2-{[2-(2,6-Dioxopiperidin-3-yl)-1,3-dioxo-2,3-dihydro-1H-isoindol-4-yl]amino}ethoxy)propoxy]propoxy}phenyl)-4,4-dimethyl-5-oxo-2-sulfanylidenimidazolidin-1-yl]-2-(trifluormethyl)benzonitril;
4-(3-{4-[2-(2-{[2-(2,6-Dioxopiperidin-3-yl)-1,3-dioxo-2,3-dihydro-1H-isoindol-4-yl]amino}ethoxy)ethoxy]phenyl}-4,4-dimethyl-5-oxo-2-sulfanylidenimidazolidin-1-yl)-2-(trifluormethyl)benzonitril;
4-{[5-(3-{[2-(2,6-Dioxopiperidin-3-yl)-1,3-dioxo-2,3-dihydro-1H-isoindol-4-yl]amino}propoxy)pentyl]oxy}-N-[trans-3-(3-chlor-4-cyanophenoxy)-2,2,4,4-tetramethylcyclobutyl]benzamid;
4-[3-(4-{4-[(5-{[2-(2,6-Dioxopiperidin-3-yl)-13-dioxo-2,3-dihydro-1H-isoindol-4-yl]amino}pentyl)oxy]phenyl}phenyl)-4,4-dimethyl-5-oxo-2-sulfanylidenimidazolidin-1-yl]-2-(trifluormethyl)benzonitril;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[(1S)-1-[4-(4-{[2-(2,6-dioxopiperidin-3-yl)-1,3-dioxo-2,3-dihydro-1H-isoindol-4-yl]amino}butoxy)phenyl]ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[3-(3-{[2-(2,6-dioxopiperidin-3-yl)-1,3-dioxo-2,3-dihydro-1H-isoindol-4-yl]amino}propoxy)propyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-(3-{[2-(2,6-dioxopiperidin-3-yl)-1,3-dioxo-2,3-dihydro-1H-isoindol-4-yl]amino}propyl)acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[(1S)-1-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[2-(2-{[2-(2,6-dioxopiperidin-3-yl)-1,3-dioxo-2,3-dihydro-1H-isoindol-4-yl]amino}ethoxy)ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-[4-(4-{[2-(2,6-dioxopiperidin-3-yl)-1,3-dioxo-2,3-dihydro-1H-isoindol-4-yl]amino}butoxy)phenyl]ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-{4-[2-(2-{[2-(2,6-dioxopiperidin-3-yl)-1,3-dioxo-2,3-dihydro-1H-isoindol-4-yl]amino}ethoxy)ethoxy]phenyl}ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-[(1R)-1-[4-(3-{[2-(2,6-dioxopiperidin-3-yl)-1,3-dioxo-2,3-dihydro-1H-isoindol-4-yl]amino}propoxy)phenyl]ethyl]acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-{2-[4-(3-{[2-(2,6-dioxopiperidin-3-yl)-1,3-dioxo-2,3-dihydro-1H-isoindol-4-yl]amino}propoxy)phenyl]pyrimidin-5-yl}acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-{4-[3-(2-{[2-(2,6-dioxopiperidin-3-yl)-1,3-dioxo-2,3-dihydro-1H-isoindol-4-yl]amino}ethoxy)propoxy]-3-fluorphenyl}acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-2-fluorphenyl}acetamid;
2-[(9S)-7-(4-Chlorphenyl)-4,5,13-trimethyl-3-thia-1,8,11,12-tetraazatricyclo[8.3.0.0²,⁶]trideca-2(6),4,7,10,12-pentaen-9-yl]-N-{4-[4-(3-{[2-(2,6-dioxopiperidin-3-yl)-1,3-dioxo-2,3-dihydro-1H-isoindol-4-yl]amino}propoxy)butoxy]-3-fluorphenyl}acetamid;
4-(4-{[(5Z)-3-[2-(2-{[2-(2,6-Dioxopiperidin-3-yl)-1,3-dioxo-2,3-dihydro-1H-isoindol-4-yl]amino}ethoxy)ethyl]-2,4-dioxo-1,3-thiazolidin-5-yliden]methyl}-2-methoxyphen (trifluormethyl)benzonitril; und
4-(4-{[(5Z)-3-[3-(2-{[2-(2,6-Dioxopiperidin-3-yl)-1,3-dioxo-2,3-dihydro-1H-isoindol-4-yl]amino}ethoxy)propyl]-2,4-dioxo-1,3-thiazolidin-5-yliden]methyl}-2-methoxyphenoxy)-3-(trifluormethyl)benzonitril.

4. Verbindung nach Anspruch 1 oder pharmazeutisch annehmbares Salz, Enantiomer oder Stereoisomer davon, wobei das PTM an den Östrogenrezeptor oder Androgenrezeptor bindet.

5. Verbindung nach Anspruch 1 oder pharmazeutisch annehmbares Salz, Enantiomer oder Stereoisomer davon, wobei das PTM an den Östrogenrezeptor bindet.

6. Verbindung nach Anspruch 1 oder pharmazeutisch annehmbares Salz, Enantiomer oder Stereoisomer davon, wobei das PTM an den Androgenrezeptor bindet.

7. Verbindung nach Anspruch 1 oder pharmazeutisch annehmbares Salz, Enantiomer oder Stereoisomer davon, wobei das PTM an das BET-Bromodomänen-Protein bindet.

8. Pharmazeutische Zusammensetzung, umfassend die Verbindung nach irgendeinem der Ansprüche 1 bis 3, oder pharmazeutisch annehmbares Salz, Enantiomer oder Stereoisomer davon und einen pharmazeutisch annehmbaren Träger, Zusatzstoff und/oder Exzipienten.

9. Pharmazeutische Zusammensetzung nach Anspruch 8, ferner umfassend ein Antivirusmittel, ein Anti-HIV-Mittel oder ein Antikrebsmittel.

10. Pharmazeutische Zusammensetzung nach Anspruch 8, ferner umfassend ein entzündungshemmendes Mittel, ein immunologisches Mittel, ein kardiovaskuläres Mittel oder ein neurologisches Mittel.

11. Pharmazeutische Zusammensetzung nach Anspruch 9, wobei das Antikrebsmittel ausgewählt ist aus der Gruppe bestehend aus einem FLT-3-Inhibitor, einem VEGFR-Inhibitor, einem EGFR-TK-Inhibitor, einem Aurora-Kinase-Inhibitor, einem PIK-1-Modulator, einem Bcl-2-Inhibitor, einem HDAC-Inhibitor, einem c-MET-Inhibitor, einem PARP-Inhibitor, einem Cdk-Inhibitor, ein EGFR-TK-Inhibitor, einem IGFR-TK-Inhibitor, einem Anti-HGF-Antikörper, einem PI3-Kinase-Inhibitor, einem AKT-Inhibitor, einem mTORC1/2-Inhibitor, einem JAK/STAT-Inhibitor, einem Checkpoint-1- oder 2-Inhibitor, einem Focal-Adhesion-Kinase-Inhibitor, einem Map-Kinase-Kinase (mek)-Inhibitor, einem VEGF-Trap-Antikörper und Mischungen davon.

12. Verbindung nach irgendeinem der Ansprüche 1 bis 3 oder pharmazeutisch annehmbares Salz, Enantiomer oder Stereoisomer davon zur Verwendung bei einem Verfahren zur Behandlung eines Krankheitszustands oder einer Störung, die durch Proteinabbau bei einem Patienten moduliert wird, wobei das Verfahren das Verabreichen einer wirksamen Menge der Verbindung oder des pharmazeutisch annehmbaren Salzes, Enantiomers oder Stereoisomers davon umfasst.

13. Verbindung nach irgendeinem der Ansprüche 1 bis 3 oder pharmazeutisch annehmbares Salz, Enantiomer oder Stereoisomer davon zur Verwendung bei einem Verfahren zur Behandlung von Krebs, wobei das Verfahren das Verabreichen einer wirksamen Menge der Verbindung oder des pharmazeutisch annehmbaren Salzes, Enantiomers oder Stereoisomers davon an einen Patienten, der dessen bedarf, umfasst, wobei der Krebs ein großzelliges B-Zellen-Lymphom, Prostatakrebs, Eierstockkrebs, Brustkrebs, multiples Myelom, Gebärmutterkrebs, Dickdarmkrebs oder Leukämie ist.

14. Verbindung oder pharmazeutisch annehmbares Salz, Enantiomer oder Stereoisomer davon zur Verwendung nach Anspruch 13, wobei der Krebs Prostatakrebs oder Brustkrebs ist.

## Revendications

1. Composé ayant une structure chimique comprenant :
PTM-L-CLM
ou un sel, un énantiomère ou un stéréoisomère pharmaceutiquement acceptable de celui-ci, dans lequel
PTM est un petit groupement cible de protéines moléculaires qui se lie spécifiquement au récepteur d'œstrogène, au récepteur d'androgène ou à une protéine à bromodomaine BET ;
L est un groupe de liaison chimique couplé de manière covalente au PTM et au CLM et est représenté par : 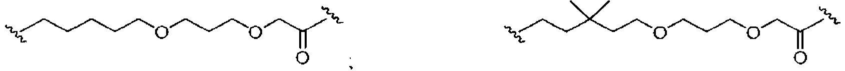 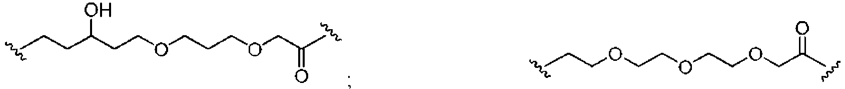 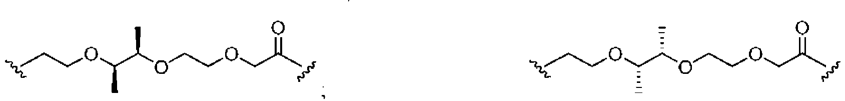 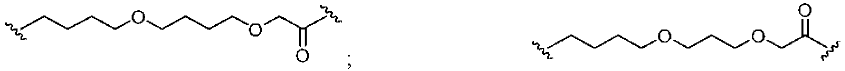 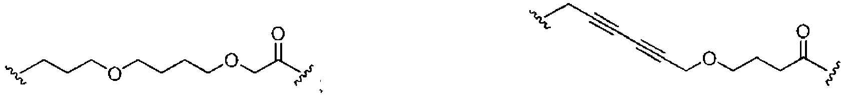 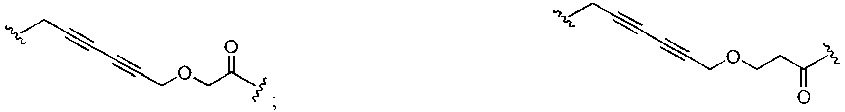 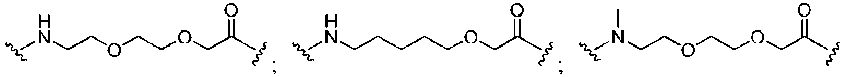 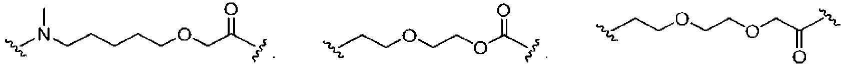 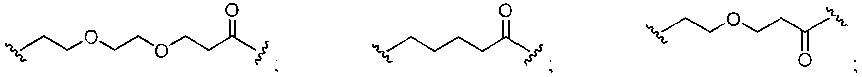 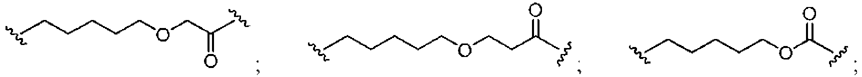 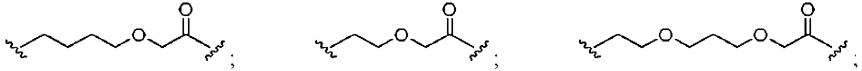 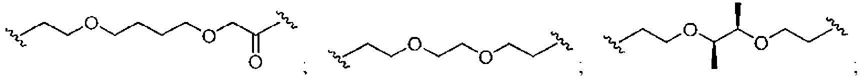 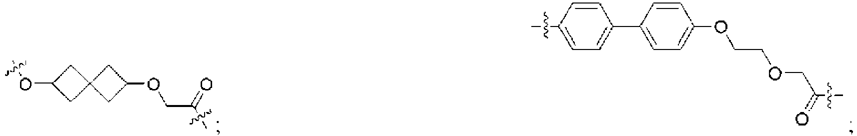 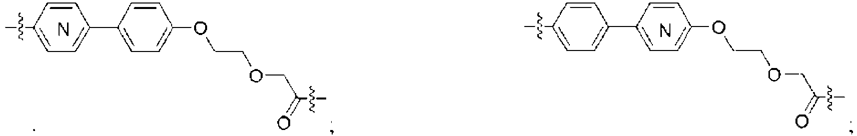 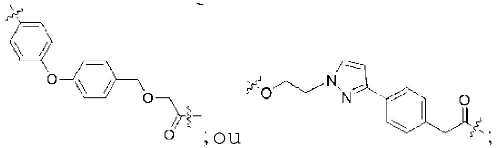 et
CLM est un groupement de liaison de l'ubiquitine ligase E3 Cereblon dont la structure est représentée par : 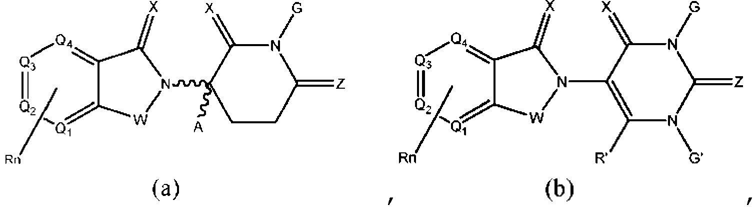 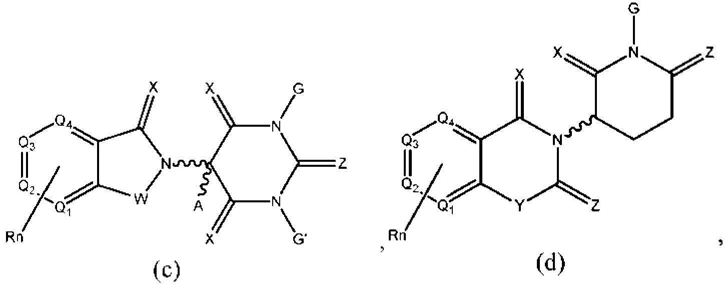 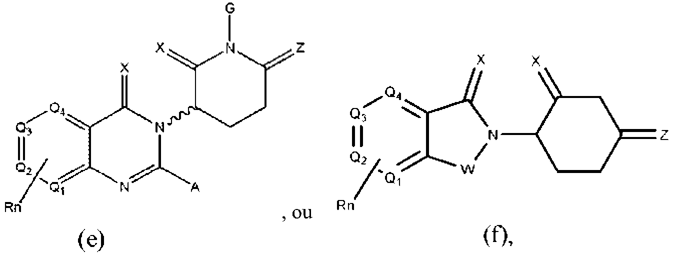 dans lequel
W est sélectionné dans le groupe constitué de CH₂, CHR, C=O, SO₂, NH et N-alkyle ;
chaque X est indépendamment sélectionné dans le groupe constitué de O, S et H₂ ;
Y est sélectionné dans le groupe constitué de NH, N-alkyle, N-aryle, N-hétéroaryle, N-cycloalkyle, N-hétérocyclyle, O et S ;
Z est sélectionné dans le groupe constitué de O, S et H₂ ;
G et G' sont indépendamment sélectionnés dans le groupe constitué de H, d'alkyle, OH, de CH₂- hétérocyclyle éventuellement substitué par R', et de benzyle éventuellement substitué par R' ;
Q₁, Q₂, Q₃ et Q₄ représentent (i) un azote ou (ii) un carbone substitué par H ou R ;
A est indépendamment sélectionné dans le groupe H, d'alkyle, de cycloalkyle, Cl et F ;
R comprend -CONR'R", -OR', -NR'R", -SR', -SO₂R', - SO₂NR'R", -CR'R"-, -CR'NR'R"-, -aryle, -hétéroaryle, - alkyle, -cycloalkyle, -hétérocyclyle, -P(O)(OR')R", - P(O)R'R", - OP(O)(OR')R", -OP(O)R'R", -Cl, -F, -Br, -I, -CF₃, -CN, -NR'SO₂NR'R", -NR'CONR'R", - CONR'COR", -NR'C(=N-CN)NR'R", -C(=N-CN)NR'R", -NR'C(=N-CN)R", - NR'C(=C-NO₂)NR'R", -SO₂NR' COR", -NO₂, -CO₂R', -C (C=N-OR')R", -CR'=CR'R", -CCR' , -S (C=O)(C=N-R') R", -SF₅ et - OCF₃ ;
R' et R" sont indépendamment sélectionnés dans le groupe constitué d'une liaison, d'H, d'alkyle, de cycloalkyle, d'aryle, d'hétéroaryle, de hétérocyclyle ;
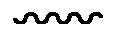 représente une liaison qui peut être stéréospécifique ((R) ou (S)) ou non stéréo spécifique ;
et dans lequel n est un nombre entier compris entre 1 et 4, et dans lequel
un R est modifié pour être lié de manière covalente au groupe de liaison (L).

2. Composé selon la revendication 1, ou sel, énantiomère ou stéréoisomère pharmaceutiquement acceptable de celui-ci, sélectionné dans le groupe constitué de :
4-{3-[4-({1-[2-(2,6-dioxopipéridine-3-yl)-1,3-dioxo-2,3-dihydro-1H-isoindole-4-yl]-4,7,10-trioxa-1-azatridécane-13-yl}oxy)phényle]-4,4-diméthyle-5-oxo-2-sulfanylidèneimidazolidine-1-yl}-2-(trifluorométhyle)benzonitrile ;
4-{3-[4-({1-[2-(2,6-dioxopipéridine-3-yl)-1,3-dioxo-2,3-dihydro-1H-isoindole-4-yl]-4,7,10-trioxa-1-azadodécane-12-yl}oxy)phényle]-4,4-diméthyle-5-oxo-2-sulfanylidèneimidazolidine-1-yl}-2-(trifluorométhyle)benzonitrile ;
4-(3-{4-[(1-{2-[(3S)-2,6-dioxopipéridine-3-yl]-1,3-dioxo-2,3-dihydro-1H-isoindole-4-yl}-4,7,10-trioxa-1-azadodécane-12-yl)oxy]phényle}-4,4-diméthyle-5-oxo-2-sulfanylidèneimidazolidine-1-yl)-1-(trifluorométhyle)benzonitrile ;
4-(3-{4-[(1-{2-[(3R)-2,6-dioxopipéridine-3-yl]-1,3-dioxo-2,3-dihydro-1H-isoindole-4-yl}-4,7,10-trioxa-1-azadodécane-12-yl)oxy]phényle}-4,4-diméthyle-5-oxo-2-sulfanylidèneimidazolidine-1-yl)-2-(trifluorométhyle)benzonitrile ;
4-{3-[4-({1-[2-(2,6-dioxopipéridine-3-yl)-1,3-dioxo-2,3-dihydro-1H-isoindole-4-yl]-4,7,10,13,16-pentaoxa-1-azaoctadécane-18-yl}oxy)phényle]-4,4-diméthyle-5-oxo-2-sulfanylidèneimidazolidine-1-yl}-2-(trifluorométhyle)benzonitrile ;
4-[3-(4-{2-[2-(2-{[2-(2,6-dioxopipéridine-3-yl)-1,3-dioxo-2,3-dihydro-1H-isoindole-4-yl]amino}éthoxy)éthoxy]éthoxy}phényle)-4,4-diméthyle-5-oxo-2-sulfanylidèneimidazolidine-1-yl]-2-(trifluorométhyle)benzonitrile ;
4-[3-(4-{3-[2-(2-{[2-(2,6-dioxopipéridine-3-yl)-1,3-dioxo-2,3-dihydro-1H-isoindole-4-yl]amino}éthoxy)éthoxy]propoxy}phényle)-4,4-diméthyle-5-oxo-2-sulfanylidèneimidazolidine-1-yl]-2-(trifluorométhyle)benzonitrile ;
4-{3-[4-({1-[2-(2,6-dioxopipéridine-3-yl)-1,3-dioxo-2,3-dihydro-1H-isoindole-4-yl]-4,7,10-trioxa-1-azatétradécane-14-yl}oxy)phényle]-4,4-diméthyle-5-oxo-2-sulfanylidèneimidazolidine-1-yl}-2-(trifluorométhyle)benzonitrile ;
4-{4,4-diméthyle-3-[4-({1-[2-(3-méthyle-2,6-dioxopipéridine-3-yl)-1,3-dioxo-2,3-dihydro-1H-isoindole-4-yl]-4,7,10-trioxa-1-azatridécane-13-yl}oxy)phényle]-5-oxo-2-sulfanylidèneimidazolidine-1yl}-2-(trifluorométhyle)benzonitrile ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[4-({1-[2-(2,6-dioxopipéridine-3-yl)-1,3-dioxo-2,3-dihydro-1H-isoindole-4-yl]-4,7,10-trioxa-1-azadodécane-12-yl}oxy)phényle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-y1]-N-[4-({1-[2-(2,6-dioxopipéridine-3-yl)-1,3-dioxo-2,3-dihydro-1H-isoindole-4-yl]-4,7,10,13-tétraoxa-1-azapentadécane-15-yl}oxy)phényle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-(4-{2-[2-(2-{[2-(2,6-dioxopipéridine-3-yl)-1,3-dioxo-2,3-dihydro-1H-isoindole-4-yl]amino}éthoxy)éthoxy]éthoxy}phényle)acétamide ;
2-[(9R)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[4-({1-[2-(2,6-dioxopipéridine-3-yl)-1-oxo-2,3-dihydro-1H-isoindole-4-yl]-4,7,10-trioxa-1-azadodécane-12-yl}oxy)phényle]acétamide ; et
4-(4-{[(5Z)-3-{2-[2-(2-{[2-(2,6-dioxopipéridine-3-yl)-1,3-dioxo-2,3-dihydro-1H-isoindole-4-yl]amino}éthoxy)éthoxy]éthyle}-2,4-dioxo-1,3-thiazolidine-5-ylidène]méthyle}-2-méthoxyphénoxy)-3-(trifluorométhyle)benzonitrile.

3. Composé, ou sel, énantiomère ou stéréoisomère pharmaceutiquement acceptable de celui-ci, sélectionné dans le groupe constitué de :
4-[3-(4-{3-[3-(2-{[2-(2,6-dioxopipéridine-3-yl)-1,3-dioxo-2,3-dihydro-1H-isoindole-4-yl]amino}éthoxy)propoxy]propoxy}phényle)-4,4-diméthyle-5-oxo-2-sulfanylidèneimidazolidine-1-yl]-2-(trifluorométhyle)benzonitrile ;
4-(3-{4-[2-(2-{[2-(2,6-dioxopipéridine-3-yl)-1,3-dioxo-2,3-dihydro-1H-isoindole-4-yl]amino}éthoxy)éthoxy]phényle}-4,4-diméthyle-5-oxo-2-sulfanylidèneimidazolidine-1-yl)-2-(trifluorométhyle)benzonitrile ;
4-{[5-(3-{[2-(2,6-dioxopipéridine-3-yl)-1,3-dioxo-2,3-dihydro-1H-isoindole-4-yl]amino}propoxy)pentyle]oxy}-N-[trans-3-(3-chloro-4-cyanophénoxy)-2,2,4,4-tétraméthylcyclobutyl]benzamide ;
4-[3-(4-{4-[(5-{[2-(2,6-dioxopipéridine-3-yl)-1,3-dioxo-2,3-dihydro-1H-isoindole-4-yl]amino}pentyle)oxy]phényle}phényle)-4,4-diméthyle-5-oxo-2-sulfanylidèneimidazolidine-1-yl]-2-(trifluorométhyle)benzonitrile ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[(1S)-1-[4-(4-{[2-(2,6-dioxopipéridin-3-yl)-1,3-dioxo-2,3-dihydro-1H-isoindole-4-yl]amino}butoxy)phényle]éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[3-(3-{[2-(2,6-dioxopipéridine-3-yl)-1,3-dioxo-2,3-dihydro-1H-isoindole-4-yl]amino}propoxy)propyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-(3-{[2-(2,6-dioxopipéridine-3-yl)-1,3-dioxo-2,3-dihydro-1H-isoindole-4-yl]amino}propyle)acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[(1S)-1-{4-[2-(2-{[2-(2,6-dioxopipéridine-3-yl)-1,3-dioxo-2,3-dihydro-1H-isoindole-4-yl]amino}éthoxy)éthoxy]phényle}éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[2-(2-{[2-(2,6-dioxopipéridine-3-yl)-1,3-dioxo-2,3-dihydro-1H-isoindole-4-yl]amino}éthoxy)éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[(1R)-1-[4-(4-{[2-(2,6-dioxopipéridine-3-yl)-1,3-dioxo-2,3-dihydro-1H-isoindole-4-yl]amino}butoxy)phényle]éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[(1R)-1-{4-[2-(2-{[2-(2,6-dioxopipéridine-3-yl)-1,3-dioxo-2,3-dihydro-1H-isoindole-4-yl]amino}éthoxy)éthoxy]phényle}éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-[(1R)-1-[4-(3-{[2-(2,6-dioxopipéridine-3-yl)-1,3-dioxo-2,3-dihydro-1H-isoindole-4-yl]amino}propoxy)phényle]éthyle]acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-{2-[4-(3-{[2-(2,6-dioxopipéridine-3-yl)-1,3-dioxo-2,3-dihydro-1H-isoindole-4-yl]amino}propoxy)phényle]pyrimidine-5-yl}acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-{4-[3-(2-{[2-(2,6-dioxopipéridine-3-yl)-1,3-dioxo-2,3-dihydro-1H-isoindole-4-yl]amino}éthoxy)propoxy]-3-fluorophényle}acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-{4-[4-(3-{[2-(2,6-dioxopipéridine-3-yl)-1,3-dioxo-2,3-dihydro-1H-isoindole-4-yl]amino}propoxy)butoxy]-2-fluorophényle}acétamide ;
2-[(9S)-7-(4-chlorophényle)-4,5,13-triméthyle-3-thia-1,8,11,12-tétraazatricyclo[8.3.0.0²,⁶]tridéca-2(6),4,7,10,12-pentaen-9-yl]-N-{4-[4-(3-{[2-(2,6-dioxopipéridine-3-yl)-1,3-dioxo-2,3-dihydro-1H-isoindole-4-yl]amino}propoxy)butoxy]-3-fluorophényle}acétamide ;
4-(4-{[(5Z)-3-[2-(2-{[2-(2,6-dioxopipéridine-3-yl)-1,3-dioxo-2,3-dihydro-1H-isoindole-4-yl]amino}éthoxy)éthyle]-2,4-dioxo-1,3-thiazolidine-5-ylidène]méthyle}-2-méthoxyphénoxy)-3-(trifluorométhyle)benzonitrile ; et
4-(4-{[(5Z)-3-[3-(2-{[2-(2,6-dioxopipéridine-3-yl)-1,3-dioxo-2,3-dihydro-1H-isoindole-4-yl]amino}éthoxy)propyle]-2,4-di oxo-1,3-thiazoli dine-5-ylidène]méthyle}-2-méthoxyphénoxy)-3-(trifluorométhyle)benzonitrile.

4. Composé selon la revendication 1, ou sel, énantiomère ou stéréoisomère pharmaceutiquement acceptable de celui-ci, dans lequel le PTM se lie au récepteur d'œstrogène ou au récepteur d'androgène.

5. Composé selon la revendication 1, ou sel, énantiomère ou stéréoisomère pharmaceutiquement acceptable de celui-ci, dans lequel le PTM se lie au récepteur d'œstrogène.

6. Composé selon la revendication 1, ou sel, énantiomère ou stéréoisomère pharmaceutiquement acceptable de celui-ci, dans lequel le PTM se lie au récepteur d'androgène.

7. Composé selon la revendication 1, ou sel, énantiomère ou stéréoisomère pharmaceutiquement acceptable de celui-ci, dans lequel le PTM se lie à la protéine à bromodomaine BET.

8. Composition pharmaceutique comprenant le composé selon l'une quelconque des revendications 1 à 3, ou un sel, un énantiomère ou un stéréoisomère pharmaceutiquement acceptable de celui-ci, et un support, un additif et/ou un excipient pharmaceutiquement acceptable.

9. Composition pharmaceutique selon la revendication 8, comprenant en outre un agent antiviral, un agent anti-VIH ou un agent anticancéreux.

10. Composition pharmaceutique selon la revendication 8, comprenant en outre un agent anti-inflammatoire, un agent immunologique, un agent cardiovasculaire ou un agent neurologique.

11. Composition pharmaceutique selon la revendication 9 dans laquelle l'agent anticancéreux est sélectionné dans le groupe constitué d'un inhibiteur de FLT-3, d'un inhibiteur de VEGFR, d'un inhibiteur d'EGFR TK, d'un inhibiteur d'aurora kinase, d'un modulateur de PIK-1, d'un inhibiteur de Bcl-2, d'un inhibiteur de HD AC, d'un inhibiteur de c-MET, d'un inhibiteur de PARP, d'un inhibiteur de Cdk, d'un inhibiteur d'EGFR TK, d'un inhibiteur d'IGFR-TK, d'un anticorps anti-HGF, d'un inhibiteur de PI3 kinase, d'un inhibiteur d'AKT, d'un inhibiteur de mTORC1/2, d'un inhibiteur de JAK/STAT, d'un inhibiteur de point de contrôle-1 ou 2, d'un inhibiteur de kinase d'adhésion focale, d'un inhibiteur de Map kinase kinase (mek), d'un anticorps piège à VEGF, et des mélanges de ceux-ci.

12. Composé selon l'une quelconque des revendications 1 à 3, ou sel, énantiomère ou stéréoisomère pharmaceutiquement acceptable de celui-ci, destiné à être utilisé dans un procédé de traitement d'une pathologie ou d'une condition qui est modulée par la dégradation des protéines chez un patient, le procédé comprenant l'administration d'une quantité efficace du composé ou du sel, de l'énantiomère ou du stéréoisomère pharmaceutiquement acceptable de celui-ci.

13. Composé selon l'une quelconque des revendications 1 à 3, ou sel, énantiomère ou stéréoisomère pharmaceutiquement acceptable de celui-ci, destiné à être utilisé dans un procédé de traitement du cancer, le procédé comprenant l'administration d'une quantité efficace du composé ou sel, énantiomère ou stéréoisomère pharmaceutiquement acceptable de celui-ci à un patient en ayant besoin, dans lequel le cancer est un lymphome à grandes cellules B, un cancer de la prostate, un cancer de l'ovaire, un cancer du sein, un myélome multiple, un cancer de l'utérus, un cancer du côlon ou une leucémie.

14. Composé ou sel, énantiomère ou stéréoisomère pharmaceutiquement acceptable de celui-ci destiné à être utilisé selon la revendication 13, dans lequel le cancer est un cancer de la prostate ou un cancer du sein.
